# EUROPEAN PATENT APPLICATION

(11) **EP 3 395 834 A1**
(43) Date of publication of application: **31.10.2018**
(21) Application number: 16877783.7
(22) Date of filing: 23.12.2016
(51) Int. Cl.: C07K 16/30, C12N 15/13, C12N 15/63, A61K 39/395, A61K 45/00, A61K 31/4025, A61P 35/00, G01N 33/574

(54) **TPBG ANTIBODY AND PREPARATION METHOD THEREFOR, CONJUGATE AND USE THEREOF**

(30) Priority: 24.12.2015 CN 201510990545
(71) Applicant: XDCExplorer (Shanghai) Co., Ltd., Shanghai 201210 (CN)
(72) Inventor: SUN, Xiaolan, Shanghai 201210 (CN); ZHANG, Ying, Shanghai 201210 (CN); ZHANG, Yu, Shanghai 201210 (CN); HU, Feifei, Shanghai 201210 (CN); GONG, Shiyong, Shanghai 201210 (CN); LIU, Lile, Shanghai 201210 (CN)
(74) Representative: Gulde & Partner
(86) International application number: PCT/CN2016/111699
(87) International publication number: WO 2017/107973

(57) **Abstract**

The present invention provides a TPBG antibody, a preparation method thereof, conjugates and use thereof. The TPBG antibody comprises one or more of the heavy chain CDR1, the heavy chain CDR2 and the heavy chain CDR3 of the heavy chain variable region of TPBG antibody, and/or one or more of the light chain CDR1, the light chain CDR2 and the light chain CDR3 of the light chain variable region of TPBG antibody, and the amino acid sequences of which are described in the present invention. The TPBG antibody is humanized antibody with high affinity, and the conjugates prepared by coupling the TPBG antibody with the small-molecule drug toxin MMAF can have a cytotoxicity effects on TPBG-positive cells, therefore the TPBG antibody can be used in the preparation of drugs for treating tumors and the like.

<110>  Shanghai Pharmaexplorer Co.,Ltd.

<120>  A TPBG Antibody and Method of Preparation, Conjugates and Use Thereof

<130>  P184114345EP

<150>  CN201510990545.6

<151>  2015-12-24

<160>  100

<170>  PatentIn version 3.5

<210>  1

<211>  120

<212>  PRT

<213>  Mus musculus

<400>  1

Asp Val Gln Leu Val Glu Ser Gly Gly Gly Leu Val Gln Pro Gly Gly

1               5                   10                  15

Ser Arg Lys Leu Ser Cys Ala Ala Ser Gly Phe Thr Phe Ser Asn Phe

20                  25                  30

Gly Met His Trp Val Arg Gln Ala Pro Glu Lys Gly Leu Glu Trp Val
        35                  40                  45

Ala Tyr Ile Ser Ser Gly Ser Thr Ser Phe Tyr Tyr Ala Asp Thr Val
    50                  55                  60

Lys Gly Arg Phe Thr Ile Ser Arg Asp Asn Pro Lys Asn Thr Leu Phe
65                  70                  75                  80

Leu Gln Met Thr Ser Leu Arg Ser Glu Asp Thr Ala Met Tyr Tyr Cys
                85                  90                  95

Ser Arg Thr Asn Ser Leu Leu Arg Gly Phe Phe Asp Ala Trp Gly Ala
            100                 105                 110

Gly Thr Thr Val Thr Val Ser Ser
        115                 120

<210>  2

<211>  5

<212>  PRT

<213>  Mus musculus

<400>  2

Asn Phe Gly Met His

1               5

<210>  3

<211>  16

<212>  PRT

<213>  Mus musculus

<400>  3

Ile Ser Ser Gly Ser Thr Ser Phe Tyr Tyr Ala Asp Thr Val Lys Gly

1               5                   10                  15

<210>  4

<211>  10

<212>  PRT

<213>  Mus musculus

<400>  4

Thr Asn Ser Leu Leu Arg Gly Phe Phe Asp
1               5                   10

<210>  5
<211>  111
<212>  PRT
<213>  Mus musculus

<400>  5

Asp Ile Val Leu Thr Gln Ser Pro Ala Ser Leu Ala Val Ser Leu Gly
1               5                   10                  15

Gln Arg Ala Thr Ile Ser Cys Arg Ala Ser Gln Ser Val Ser Ser Ser
            20                  25                  30

Ser Tyr Thr Tyr Leu His Trp Tyr Gln Gln Lys Pro Gly Gln Pro Pro
        35                  40                  45

Lys Leu Leu Ile Lys Ser Ala Ser Asn Leu Glu Ser Gly Val Pro Ala
    50                  55                  60

Arg Phe Ser Gly Thr Gly Ser Gly Thr Asp Phe Thr Leu Asn Ile His
65                  70                  75                  80

Pro Val Glu Glu Glu Asp Thr Ala Thr Tyr Tyr Cys Gln His Ser Trp
                85                  90                  95

Glu Ile Pro Leu Thr Phe Gly Ala Gly Thr Lys Leu Glu Leu Lys
            100                 105                 110

<210>  6
<211>  21
<212>  PRT
<213>  Mus musculus

<400>  6

Arg Ala Thr Ile Ser Cys Arg Ala Ser Gln Ser Val Ser Ser Ser Ser
1               5                   10                  15

Tyr Thr Tyr Leu His
            20

<210>  7
<211>  7
<212>  PRT
<213>  Mus musculus

<400>  7

Ser Ala Ser Asn Leu Glu Ser
1               5

<210>  8
<211>  9
<212>  PRT
<213>  Mus musculus

<400>  8

Gln His Ser Trp Glu Ile Pro Leu Thr
1               5

<210>  9

<211>  121

<212>  PRT

<213>  Mus musculus

<400>  9

Glu Val Gln Leu Val Glu Ser Gly Gly Asp Leu Val Gln Pro Gly Gly
1               5                   10                  15

Ser Leu Lys Leu Phe Cys Ala Ala Ser Gly Phe Thr Phe Ser Thr Tyr
            20                  25                  30

Gly Leu Ser Trp Val Arg Gln Thr Pro Asp Lys Arg Leu Glu Leu Val
        35                  40                  45

Ala Thr Ile Thr Asn Asn Asp Gly Ser Ala Tyr Tyr Pro Asp Ser Val
    50                  55                  60

Lys Gly Arg Phe Thr Ile Ser Arg Asp Lys Ala Lys Asn Thr Leu Phe

65                  70                  75                  80

Leu Gln Met Thr Ser Leu Lys Ser Glu Asp Thr Ala Met Tyr Tyr Cys

                85                  90                  95

Ala Arg Glu Thr Gly Phe Gly Ser Ser Tyr Leu Phe Asp Tyr Trp Gly

            100                 105                 110

Gln Gly Thr Leu Val Thr Val Ser Ala

        115                 120

<210>  10

<211>  5

<212>  PRT

<213>  Mus musculus

<400>  10

Thr Tyr Gly Leu Ser

1               5

<210>  11

<211>  17

<212>  PRT

<213>  Mus musculus

<400>  11

Thr Ile Thr Asn Asn Asp Gly Ser Ala Tyr Tyr Pro Asp Ser Val Lys
1               5                   10                  15

Gly

<210>  12

<211>  12

<212>  PRT

<213>  Mus musculus

<400>  12

Glu Thr Gly Phe Gly Ser Ser Tyr Leu Phe Asp Tyr
1               5                   10

<210>  13

<211>  106

<212>  PRT

<213>  Mus musculus

<400>  13

Asn Ile Val Met Thr Gln Thr Pro Lys Phe Leu Leu Val Ser Ala Gly
1               5                   10                  15

Asp Arg Val Thr Ile Thr Cys Lys Ala Ser Gln Ser Val Ser Asn Asp
            20                  25                  30

Val Ala Trp Tyr Gln Gln Lys Pro Gly Gln Ser Pro Lys Leu Leu Ile
        35                  40                  45

Tyr Phe Ala Thr Asn Arg Tyr Thr Gly Val Pro Asn Arg Phe Thr Gly
    50                  55                  60

Ser Gly Tyr Gly Thr Glu Phe Thr Phe Thr Ile Ser Ser Val Gln Ala
65                  70                  75                  80

Glu Asp Leu Ala Val Tyr Phe Cys Gln Gln Asp Tyr Ser Ser Pro Thr
                85                  90                  95

Phe Gly Gly Gly Thr Lys Leu Glu Ile Lys
            100                 105

<210>  14
<211>  11
<212>  PRT
<213>  Mus musculus

<400>  14

Lys Ala Ser Gln Ser Val Ser Asn Asp Val Ala
1               5                   10

<210>  15
<211>  7
<212>  PRT
<213>  Mus musculus

<400>  15

Phe Ala Thr Asn Arg Tyr Thr
1               5

<210>  16
<211>  8
<212>  PRT
<213>  Mus musculus

<400>  16

Gln Gln Asp Tyr Ser Ser Pro Thr
1               5

<210>  17
<211>  120
<212>  PRT
<213>  Mus musculus

<400>  17

Gln Val Gln Leu Gln Gln Ser Asp Ala Glu Leu Val Arg Pro Gly Ala
1               5                   10                  15

Ser Val Lys Met Ser Cys Lys Val Ser Gly Tyr Thr Phe Thr Asp His
            20                  25                  30

Thr Ile His Trp Met Lys Gln Arg Pro Glu Gln Gly Leu Glu Trp Ile
        35                  40                  45

Gly Tyr Ile Tyr Pro Arg Asp Gly Ser Gly Thr Tyr Asn Glu Lys Phe
    50                  55                  60

Lys Gly Lys Ala Thr Leu Thr Ala Asp Lys Ser Ser Ser Thr Ala Tyr
65                  70                  75                  80

Met Gln Leu Asn Ser Leu Thr Ser Glu Asp Ser Ala Val Tyr Phe Cys
                85                  90                  95

Ala Arg Asp Gly Ser Ser His Tyr Trp Tyr Phe Asp Val Trp Gly Thr
            100                 105                 110

Gly Thr Thr Val Thr Val Ser Ser

115                 120

<210>  18

<211>  5

<212>  PRT

<213>  Mus musculus

<400>  18

Asp His Thr Ile His
1               5

<210>  19

<211>  16

<212>  PRT

<213>  Mus musculus

<400>  19

Ile Tyr Pro Arg Asp Gly Ser Gly Thr Tyr Asn Glu Lys Phe Lys Gly
1               5                   10                  15

<210>  20

<211>  11

<212>  PRT

<213>  Mus musculus

<400>  20

Asp Gly Ser Ser His Tyr Trp Tyr Phe Asp Val
1               5                   10

<210>  21

<211>  107

<212>  PRT

<213>  Mus musculus

<400>  21

Asp Ile Gln Met Thr Gln Ser Pro Ala Ser Leu Ser Ala Ser Val Gly
1               5                   10                  15

Glu Thr Val Thr Ile Thr Cys Arg Ala Ser Glu Ile Ile Tyr Asn Tyr
            20                  25                  30

Leu Ala Trp Tyr Gln Gln Lys Gln Gly Lys Ser Pro Gln Leu Leu Val

35                  40                  45

Tyr Asn Ala Lys Thr Leu Ala Glu Gly Val Pro Ser Arg Phe Ser Gly

    50                  55                  60

Ser Tyr Ser Gly Thr Gln Phe Ser Leu Asn Ile Asn Ser Leu Gln Ser

65                  70                  75                  80

Glu Asp Phe Gly Ile Tyr Tyr Cys Gln His His His Asp Thr Pro Leu

                85                  90                  95

Thr Phe Gly Ala Gly Thr Lys Leu Asp Leu Lys

            100                 105

<210>  22

<211>  11

<212>  PRT

<213>  Mus musculus

<400>  22

Arg Ala Ser Glu Ile Ile Tyr Asn Tyr Leu Ala
1               5                   10

<210>  23
<211>  7
<212>  PRT
<213>  Mus musculus

<400>  23

Asn Ala Lys Thr Leu Ala Glu
1               5

<210>  24
<211>  9
<212>  PRT
<213>  Mus musculus

<400>  24

Gln His His His Asp Thr Pro Leu Thr
1               5

<210>  25

<211>  116

<212>  PRT

<213>  Mus musculus

<400>  25

Gln Val Gln Leu Gln Gln Ser Gly Ala Glu Leu Met Lys Pro Gly Ala
1               5                   10                  15

Ser Met Arg Leu Ser Cys Lys Ala Thr Gly Tyr Thr Phe Thr Gly Tyr
            20                  25                  30

Trp Leu Glu Trp Ile Lys Arg Arg Pro Gly His Gly Leu Glu Trp Ile
        35                  40                  45

Gly Glu Ile Leu Pro Gly Arg Gly Ser Thr Asn Tyr Asn Glu Lys Phe
    50                  55                  60

Lys Gly Lys Ala Thr Phe Thr Ala Glu Thr Ser Ser Asn Thr Ala Tyr
65                  70                  75                  80

Met Gln Leu Thr Ser Leu Thr Thr Glu Asp Ser Ala Ile Tyr Tyr Cys
                85                  90                  95

Ala Arg Gly Gly Arg Asp Phe Asp Asn Trp Gly Gln Gly Thr Thr Leu
            100                 105                 110

Thr Val Ser Ser
        115

<210>  26
<211>  5
<212>  PRT
<213>  Mus musculus

<400>  26

Gly Tyr Trp Leu Glu
1               5

<210>  27
<211>  16

<212>  PRT

<213>  Mus musculus

<400>  27

Ile Leu Pro Gly Arg Gly Ser Thr Asn Tyr Asn Glu Lys Phe Lys Gly
1               5                   10                  15

<210>  28

<211>  7

<212>  PRT

<213>  Mus musculus

<400>  28

Gly Gly Arg Asp Phe Asp Asn
1               5

<210>  29

<211>  107

<212>  PRT

<213>  Mus musculus

<400>  29

Asp Ile Gln Met Thr Gln Ser Thr Ser Ser Leu Ser Ala Ser Leu Gly
1               5                   10                  15

Asp Arg Val Thr Ile Ser Cys Ser Ala Ser Gln Gly Ile Asn Asn His
            20                  25                  30

Leu Asn Trp Tyr Gln Gln Lys Pro Asp Gly Thr Val Lys Leu Leu Ile
        35                  40                  45

Tyr Tyr Thr Ser Ser Leu His Ser Gly Val Pro Ser Arg Phe Ser Gly
    50                  55                  60

Ser Gly Ser Gly Thr Asp Tyr Ser Leu Thr Ile Ser Asn Leu Glu Pro
65                  70                  75                  80

Glu Asp Ile Ala Ile Tyr Tyr Cys Gln Gln Phe Ser Lys Leu Pro Tyr
                85                  90                  95

Thr Phe Gly Gly Gly Thr Lys Val Glu Met Lys

100                    105

<210>  30

<211>  11

<212>  PRT

<213>  Mus musculus

<400>  30

Ser Ala Ser Gln Gly Ile Asn Asn His Leu Asn
1               5                   10

<210>  31

<211>  7

<212>  PRT

<213>  Mus musculus

<400>  31

Tyr Thr Ser Ser Leu His Ser
1               5

<210>  32

<211>  9

<212>  PRT

<213>  Mus musculus

<400>  32

Gln Gln Phe Ser Lys Leu Pro Tyr Thr

1               5

<210>  33

<211>  116

<212>  PRT

<213>  Mus musculus

<400>  33

Gln Val Gln Leu Gln Gln Ser Gly Ala Glu Leu Met Lys Pro Gly Ala

1               5                   10                  15

Ser Val Lys Leu Ser Cys Lys Ala Thr Gly Tyr Thr Phe Thr Gly Tyr

            20                  25                  30

Trp Ile Glu Trp Ile Lys Gln Arg Pro Gly His Gly Leu Glu Trp Ile

35                  40                  45

Gly Glu Ile Leu Pro Gly Arg Gly Ser Thr Asp Tyr Asn Glu Lys Phe

    50                  55                  60

Lys Gly Lys Ala Thr Phe Thr Ala Asp Thr Ser Ser Asn Thr Ala Tyr

65                  70                  75                  80

Met Gln Leu Ser Ser Leu Thr Thr Glu Asp Ser Ala Ile Tyr Tyr Cys

                85                  90                  95

Thr Arg Gly Arg Leu Asp Phe Thr Tyr Trp Gly Gln Gly Thr Leu Val

            100                 105                 110

Thr Val Ser Ala

        115

<210>  34

<211>  5

<212>  PRT

<213>  Mus musculus

<400>  34

Gly Tyr Trp Ile Glu
1               5

<210>  35
<211>  16
<212>  PRT
<213>  Mus musculus

<400>  35

Ile Leu Pro Gly Arg Gly Ser Thr Asp Tyr Asn Glu Lys Phe Lys Gly
1               5                   10                  15

<210>  36
<211>  7
<212>  PRT
<213>  Mus musculus

<400>  36

Gly Arg Leu Asp Phe Thr Tyr
1               5

<210>  37
<211>  107
<212>  PRT
<213>  Mus musculus

<400>  37

Asp Ile Gln Met Thr Gln Thr Thr Ser Ser Leu Ser Ala Ser Leu Gly
1               5                   10                  15

Asp Arg Val Thr Ile Ser Cys Ser Ala Ser Gln Gly Ile Arg Asn Tyr
            20                  25                  30

Leu Asn Trp Tyr Gln Gln Lys Pro Asp Gly Thr Val Lys Leu Leu Ile
        35                  40                  45

Tyr Tyr Thr Ser Ser Leu His Ser Gly Val Pro Ser Arg Phe Ser Gly
    50                  55                  60

Ser Gly Ser Gly Thr Asp Tyr Ser Leu Thr Ile Ser Asn Leu Glu Pro
65                  70                  75                  80

Glu Asp Ile Ala Thr Tyr Tyr Cys Gln Gln Tyr Ser Lys Val Pro Trp
                85                  90                  95

Thr Phe Gly Gly Gly Thr Lys Leu Glu Ile Lys
            100                 105

<210>  38
<211>  11
<212>  PRT
<213>  Mus musculus

<400>  38

Ser Ala Ser Gln Gly Ile Arg Asn Tyr Leu Asn
1               5                   10

<210>  39
<211>  7

<212>  PRT

<213>  Mus musculus

<400>  39

Tyr Thr Ser Ser Leu His Ser

1               5

<210>  40

<211>  9

<212>  PRT

<213>  Mus musculus

<400>  40

Gln Gln Tyr Ser Lys Val Pro Trp Thr

1               5

<210>  41

<211>  120

<212>  PRT

<213>  Mus musculus

<400>  41

Asp Val Gln Leu Val Glu Ser Gly Gly Gly Leu Val Arg Pro Gly Gly
1               5                   10                  15

Ser Leu Lys Leu Ser Cys Ala Ala Ser Gly Phe Thr Phe Ser Asn Tyr
            20                  25                  30

Val Met Ser Trp Ala Arg Gln Ser Pro Glu Lys Arg Leu Glu Trp Val
        35                  40                  45

Ala Glu Ile Ser Thr Gly Gly Ser His Thr Tyr Tyr Ser Asp Thr Val
    50                  55                  60

Thr Gly Arg Phe Thr Ile Ser Arg Asp Asn Ala Lys Lys Ala Leu Tyr
65                  70                  75                  80

Leu Glu Met Ser Ser Leu Arg Ser Glu Asp Thr Ala Ile Tyr Tyr Cys
                85                  90                  95

Ile Met Phe Tyr Tyr Gly Ser Ser Tyr Ser Met Asp Tyr Trp Gly Gln

            100                 105                 110

Gly Thr Ser Val Thr Val Ser Ser

        115                 120

<210> 42

<211> 5

<212> PRT

<213> Mus musculus

<400> 42

Asn Tyr Val Met Ser

1               5

<210> 43

<211> 16

<212> PRT

<213> Mus musculus

<400> 43

Ile Ser Thr Gly Gly Ser His Thr Tyr Tyr Ser Asp Thr Val Thr Gly

1               5                   10                  15

<210>  44

<211>  11

<212>  PRT

<213>  Mus musculus

<400>  44

Phe Tyr Tyr Gly Ser Ser Tyr Ser Met Asp Tyr

1               5                   10

<210>  45

<211>  107

<212>  PRT

<213>  Mus musculus

<400>  45

Asp Ile Gln Met Thr Gln Ser Thr Ser Ser Leu Ser Ala Ser Leu Gly

1               5                   10                  15

Asp Arg Ile Thr Ile Ser Cys Arg Ala Ser Gln Asp Ile Arg Asn Tyr

20                  25                  30

Leu Asn Trp Tyr Gln Gln Lys Pro Asp Gly Thr Val Lys Leu Leu Ile

        35                  40                  45

Tyr His Thr Ser Arg Leu His Ser Gly Val Pro Ser Arg Phe Ser Gly

    50                  55                  60

Ser Gly Ser Gly Thr Asp Tyr Ser Leu Thr Ile Ser Asn Leu Glu Gln

65                  70                  75                  80

Glu Asp Ile Ala Thr Tyr Phe Cys Gln Gln Gly Asp Thr Leu Pro Leu

                85                  90                  95

Thr Phe Gly Ala Gly Thr Arg Leu Glu Leu Lys

            100                 105

<210>  46

<211>  11

<212>  PRT

<213>  Mus musculus

<400>  46

Arg Ala Ser Gln Asp Ile Arg Asn Tyr Leu Asn
1               5                   10

<210>  47
<211>  7
<212>  PRT
<213>  Mus musculus

<400>  47

His Thr Ser Arg Leu His Ser
1               5

<210>  48
<211>  9
<212>  PRT
<213>  Mus musculus

<400>  48

Gln Gln Gly Asp Thr Leu Pro Leu Thr

1               5

<210>  49

<211>  119

<212>  PRT

<213>  Mus musculus

<400>  49

Asp Val Gln Leu Gln Glu Ser Gly Pro Gly Met Val Lys Pro Ser Gln

1               5                   10                  15

Ser Leu Ser Leu Thr Cys Thr Val Thr Gly Tyr Ser Ile Thr Ser Gly

            20                  25                  30

Tyr Asp Trp His Trp Ile Arg His Phe Pro Gly Asn Lys Leu Glu Trp

        35                  40                  45

Met Gly Tyr Ile Ser Tyr Ser Gly Asn Thr Asn Tyr Asn Pro Ser Leu

    50                  55                  60

Lys Ser Arg Ile Ser Ile Thr His Asn Thr Ser Lys Asn His Phe Phe
65                  70                  75                  80

Leu Arg Leu Asn Ser Val Thr Thr Glu Asp Thr Ala Thr Tyr Tyr Cys
                85                  90                  95

Ala Arg Gly Tyr Gly Asn Trp Asp Ala Met Asp Tyr Trp Gly Gln Gly
            100                 105                 110

Thr Ser Val Thr Val Ser Ser
        115

<210>  50
<211>  6
<212>  PRT
<213>  Mus musculus

<400>  50

Ser Gly Tyr Asp Trp His
1               5

<210>  51

<211>  15

<212>  PRT

<213>  Mus musculus

<400>  51

Ile Ser Tyr Ser Gly Asn Thr Asn Tyr Asn Pro Ser Leu Lys Ser

1               5                   10                  15

<210>  52

<211>  10

<212>  PRT

<213>  Mus musculus

<400>  52

Gly Tyr Gly Asn Trp Asp Ala Met Asp Tyr

1               5                   10

<210>  53

<211>  107

<212>  PRT

<213>  Mus musculus

<400>  53

Asp Ile Val Met Thr Gln Ser Gln Lys Phe Met Ser Thr Ser Val Gly
1               5                   10                  15

Asp Arg Val Ser Val Thr Cys Lys Ala Ser Gln Ile Val Asp Thr Asn
            20                  25                  30

Val Ala Trp Tyr Gln Gln Lys Pro Gly Gln Ser Pro Lys Ala Leu Ile
        35                  40                  45

Ser Ser Ala Ser Tyr Arg Tyr Ser Gly Val Pro Asp Arg Phe Thr Gly
    50                  55                  60

Ser Gly Ser Gly Thr Asp Phe Thr Leu Thr Ile Ser Asn Val Gln Ser
65                  70                  75                  80

Glu Asp Leu Ala Glu Tyr Phe Cys Gln Gln Tyr Asn Ser Tyr Pro Leu

85                  90                  95

Thr Phe Gly Ala Gly Thr Lys Leu Glu Leu Thr

            100                 105

<210>  54

<211>  11

<212>  PRT

<213>  Mus musculus

<400>  54

Lys Ala Ser Gln Ile Val Asp Thr Asn Val Ala

1               5                   10

<210>  55

<211>  7

<212>  PRT

<213>  Mus musculus

<400>  55

Ser Ala Ser Tyr Arg Tyr Ser

1               5

<210>  56

<211>  9

<212>  PRT

<213>  Mus musculus

<400>  56

Gln Gln Tyr Asn Ser Tyr Pro Leu Thr

1               5

<210>  57

<211>  117

<212>  PRT

<213>  Mus musculus

<400>  57

Gln Ile Gln Leu Val Gln Ser Gly Pro Glu Leu Lys Lys Pro Gly Glu

1               5                   10                  15

Thr Val Lys Ile Ser Cys Lys Pro Ser Gly Tyr Thr Phe Thr Ala Tyr

            20                  25                  30

Gly Leu Ser Trp Val Lys Gln Ala Pro Gly Gln Gly Phe Gln Trp Met
        35                  40                  45

Gly Trp Ile Asn Thr Tyr Ser Gly Val Pro Thr Tyr Thr Asp Asp Phe
    50                  55                  60

Lys Gly Arg Phe Ala Phe Ser Leu Glu Thr Ser Ala Gly Thr Ala Ser
65                  70                  75                  80

Leu Gln Ile Thr Asn Leu Lys Asn Glu Asp Thr Ala Thr Tyr Phe Cys
                85                  90                  95

Ala Arg Ser Ser Ile Pro Pro Phe Glu Tyr Trp Gly Pro Gly Thr Thr
            100                 105                 110

Leu Thr Val Ser Ser
        115

<210> 58

<211> 5

<212> PRT

<213> Mus musculus

<400> 58

Ala Tyr Gly Leu Ser
1               5

<210> 59

<211> 16

<212> PRT

<213> Mus musculus

<400> 59

Ile Asn Thr Tyr Ser Gly Val Pro Thr Tyr Thr Asp Asp Phe Lys Gly
1               5                   10                  15

<210> 60

<211> 8

<212> PRT

<213>  Mus musculus

<400>  60

Ser Ser Ile Pro Pro Phe Glu Tyr
1               5

<210>  61
<211>  107
<212>  PRT
<213>  Mus musculus

<400>  61

Ser Phe Val Met Thr Gln Ser Pro Lys Phe Leu Leu Val Ser Val Gly
1               5                   10                  15

Asp Arg Val Thr Ile Thr Cys Lys Ala Ser Gln Ser Val Ser Asn Asp
            20                  25                  30

Val Ala Trp Tyr Gln Gln Lys Pro Gly Gln Pro Pro Arg Val Leu Ile
        35                  40                  45

Tyr Phe Ala Ser Asn Arg Tyr Thr Gly Val Pro Asp Arg Phe Thr Gly
    50                  55                  60

Ser Gly Tyr Gly Thr Asp Phe Thr Phe Thr Ile Ser Thr Val Gln Ala
65                  70                  75                  80

Glu Asp Leu Ala Leu Tyr Phe Cys Gln Gln Asp Tyr Ser Ser Pro Trp
                85                  90                  95

Thr Phe Gly Gly Gly Thr Lys Leu Glu Ile Lys
            100                 105

<210>  62
<211>  11
<212>  PRT
<213>  Mus musculus

<400>  62

Lys Ala Ser Gln Ser Val Ser Asn Asp Val Ala
1               5                   10

<210>  63

<211>  7

<212>  PRT

<213>  Mus musculus

<400>  63

Phe Ala Ser Asn Arg Tyr Thr

1               5

<210>  64

<211>  9

<212>  PRT

<213>  Mus musculus

<400>  64

Gln Gln Asp Tyr Ser Ser Pro Trp Thr

1               5

<210>  65

<211>  119

<212>  PRT

<213>  Mus musculus

<400>  65

Glu Val Gln Leu Gln Gln Ser Gly Pro Val Leu Val Lys Pro Gly Ala
1               5                   10                  15

Ser Val Lys Met Ser Cys Glu Ala Ser Gly Tyr Ser Phe Ile Asp Tyr
            20                  25                  30

Tyr Met Ser Trp Val Lys Gln Ser Arg Gly Arg Ser Leu Glu Trp Ile
        35                  40                  45

Gly Val Phe Asn Pro Tyr Ser Gly Glu Ile Ile Tyr Asn Gln Lys Phe
    50                  55                  60

Lys Gly Lys Ala Thr Leu Thr Val Asp Lys Ser Ser Ser Thr Ala Tyr
65                  70                  75                  80

Met Glu Leu Ser Ser Leu Thr Ser Glu Asp Ser Gly Val Tyr Ser Cys

85                  90                  95

Ala Arg Leu Arg Ile Ser Tyr Tyr Ala Ile Asp Tyr Trp Gly Gln Gly

            100                 105                 110

Thr Ser Val Ala Val Ser Ser

        115

<210>  66

<211>  5

<212>  PRT

<213>  Mus musculus

<400>  66

Asp Tyr Tyr Met Ser

1               5

<210>  67

<211>  16

<212>  PRT

<213>  Mus musculus

<400>  67

Phe Asn Pro Tyr Ser Gly Glu Ile Ile Tyr Asn Gln Lys Phe Lys Gly
1               5                   10                  15

<210>  68
<211>  10
<212>  PRT
<213>  Mus musculus

<400>  68

Leu Arg Ile Ser Tyr Tyr Ala Ile Asp Tyr
1               5                   10

<210>  69
<211>  108
<212>  PRT
<213>  Mus musculus

<400>  69

Glu Asn Val Leu Thr Gln Ser Pro Ala Ile Met Ser Ala Ser Pro Gly

1               5                   10                  15

Glu Lys Val Thr Met Thr Cys Arg Ala Thr Ser Ser Val Ser Ser Gly
            20                  25                  30

His Phe His Trp Tyr Gln Gln Lys Ser Gly Ala Ser Pro Lys Val Trp
        35                  40                  45

Ile Tyr Ser Thr Ser Asn Leu Ala Ser Gly Val Pro Ala Arg Phe Ser
    50                  55                  60

Gly Ser Gly Ser Gly Thr Ser Phe Ser Leu Thr Ile Ser Ser Val Glu
65                  70                  75                  80

Ala Glu Asp Val Ala Thr Tyr Phe Cys Gln Gln Tyr Ser Gly Tyr Pro
                85                  90                  95

Leu Thr Phe Gly Gly Gly Thr Lys Leu Glu Ile Lys
            100                 105

<210>  70

<211>  12

<212>  PRT

<213>  Mus musculus

<400>  70

Arg Ala Thr Ser Ser Val Ser Ser Gly His Phe His
1               5                   10

<210>  71

<211>  7

<212>  PRT

<213>  Mus musculus

<400>  71

Ser Thr Ser Asn Leu Ala Ser
1               5

<210>  72

<211>  9

<212>  PRT

<213>  Mus musculus

<400>  72

Gln Gln Tyr Ser Gly Tyr Pro Leu Thr
1               5

<210>  73
<211>  117
<212>  PRT
<213>  Mus musculus

<400>  73

Glu Val Gln Leu Val Glu Ser Gly Gly Gly Leu Val Lys Pro Gly Gly
1               5                   10                  15

Ser Leu Lys Leu Ser Cys Ala Ala Ser Gly Phe Thr Phe Ser Asp Tyr
            20                  25                  30

Gly Met His Trp Val Arg Gln Ala Pro Glu Lys Gly Leu Glu Trp Val
        35                  40                  45

Gly Tyr Ile Ser Ser Gly Thr Tyr Val Val Tyr Tyr Ala Asp Thr Val
    50                  55                  60

Lys Gly Arg Phe Thr Ile Ser Arg Asp Asn Ala Lys Asn Thr Leu Phe
65                  70                  75                  80

Leu Gln Met Thr Ser Leu Arg Ser Glu Asp Thr Ala Ile Tyr Tyr Cys
                85                  90                  95

Ser Thr Tyr Gly Ser Asn Pro Phe Asp Tyr Trp Gly Gln Gly Thr Thr
            100                 105                 110

Leu Thr Val Ser Ser
        115

<210>  74
<211>  5
<212>  PRT
<213>  Mus musculus

<400>  74

Asp Tyr Gly Met His
1               5

<210>  75
<211>  16
<212>  PRT
<213>  Mus musculus

<400>  75

Ile Ser Ser Gly Thr Tyr Val Val Tyr Tyr Ala Asp Thr Val Lys Gly
1               5                   10                  15

<210>  76
<211>  8
<212>  PRT
<213>  Mus musculus

<400>  76

Tyr Gly Ser Asn Pro Phe Asp Tyr
1               5

<210>  77

<211>  108

<212>  PRT

<213>  Mus musculus

<400>  77

Gln Leu Val Leu Thr Gln Ser Pro Ala Ile Met Ser Ala Ser Pro Gly
1               5                   10                  15

Glu Lys Val Thr Ile Thr Cys Ser Ala Thr Ser Ser Val Ser Ser Met
            20                  25                  30

His Trp Phe Gln Gln Lys Pro Val Thr Ser Pro Lys Leu Trp Ile Tyr
        35                  40                  45

Ser Thr Ser Asn Leu Ala Ser Gly Val Pro Thr Arg Phe Ser Gly Ser
    50                  55                  60

Gly Ser Gly Thr Ser Tyr Ser Leu Thr Ile Ser Arg Met Glu Ala Glu

65                  70                  75                  80

Asp Ala Ala Thr Tyr Tyr Cys Gln Gln Arg Ser Gly Tyr Pro Pro Ile

                85                  90                  95

Phe Thr Phe Gly Ser Gly Thr Lys Leu Glu Ile Lys

            100                 105

<210>  78

<211>  10

<212>  PRT

<213>  Mus musculus

<400>  78

Ser Ala Thr Ser Ser Val Ser Ser Met His

1               5                   10

<210>  79

<211>  7

<212>  PRT

<213>  Mus musculus

<400>  79

Ser Thr Ser Asn Leu Ala Ser
1               5

<210>  80
<211>  11
<212>  PRT
<213>  Mus musculus

<400>  80

Gln Gln Arg Ser Gly Tyr Pro Pro Ile Phe Thr
1               5                   10

<210>  81
<211>  360
<212>  DNA
<213>  Mus musculus

<400>  81
gatgtgcagc tggtggagtc tgggggaggc ttagtgcagc ctggagggtc ccggaaactc        60

tcctgtgcag cctctggatt cactttcagt aactttggaa tgcactgggt tcgtcaggct 120

ccagagaagg ggctggagtg ggtcgcatac attagtagtg gtagtacttc cttctactat 180

gcagacaccg tgaagggccg attcaccatc tccagagaca atcccaagaa caccctgttc 240

ctgcaaatga ccagtctaag gtctgaggac acggccatgt attactgttc aagaacgaat 300

tcattactac gaggattctt cgatgcctgg ggcgcaggga ccacggtcac cgtctcctca 360

<210> 82

<211> 333

<212> DNA

<213> Mus musculus

<400> 82

gacattgtcc tgacacagtc tcctgcttcc ttagctgtat ctctggggca gagggccacc 60

atctcatgca gggccagcca aagtgtcagt tcatctagtt atacttattt gcactggtac 120

caacagaaac caggacagcc acccaaactc ctcatcaagt ctgcatccaa cctagaatct 180

ggggtccctg ccaggttcag tggcactggg tctgggacag acttcaccct caacatacat 240

cctgtggagg aggaggatac tgcaacatat tactgtcagc acagttggga gattccactc 300

acgttcggtg ctgggaccaa gttggagctg aaa 333

<210> 83
<211> 363
<212> DNA
<213> Mus musculus

<400> 83
gaggtacaac tggtggagtc tgggggagac ttagtgcagc ctggagggtc cctgaaactc 60

ttctgtgcag cctctggatt cactttcagc acctatggcc tgtcttgggt tcgccagact 120

ccagacaaga ggctggagtt ggtcgcaacc attactaata atgatggtag tgcctattat 180

ccagacagtg tgaagggccg attcaccatc tccagagaca aggccaagaa caccctgttc 240

ttgcaaatga ccagtctgaa gtctgaggac acagccatgt attattgtgc aagagagacg 300

ggtttcggta gcagctacct gtttgattat tggggccaag ggactctggt cactgtctct 360

gca 363

<210> 84

<211>  318

<212>  DNA

<213>  Mus musculus

<400>  84

aatattgtga tgacccagac tcccaaattc ctgcttgtat cagcaggaga cagggttacc        60

ataacctgca aggccagtca gagtgtgagt aatgatgtgg cttggtacca acagaagcca       120

gggcagtctc ctaaactgct gatatacttt gcaaccaatc gttatactgg agtccctaat       180

cgcttcactg gcagtggata tgggacggaa ttcactttca ccatcagctc tgtgcaggct       240

gaagacctgg cggtttattt ctgtcagcag gattatagtt ctccgacgtt cggtggaggc       300

accaagctgg aaatcaaa                                                     318

<210>  85

<211>  360

<212>  DNA

<213>  Mus musculus

<400>  85

caggtccagc tgcaacagtc tgacgctgag ttggtgagac ctggagcctc agtgaagatg        60

tcctgcaagg tttctggcta taccttcact gaccatacta ttcactggat gaagcagagg 120

cctgaacagg gcctggaatg gattggatat atttatccta gagatggtag tggtacgtac 180

aatgagaagt tcaagggcaa ggccacattg actgcagaca aatcctccag cacagcctac 240

atgcagctca acagcctgac atctgaggac tctgcagtct atttctgtgc aagagacggt 300

agtagtcact actggtactt cgatgtctgg ggcacaggga ccacggtcac cgtctcctca 360

<210> 86

<211> 321

<212> DNA

<213> Mus musculus

<400> 86

gacatccaga tgactcagtc tccagcctcc ctatctgcat ctgtgggaga aactgtcacc 60

atcacatgtc gagcaagtga gattatttac aattatttag catggtatca gcagaaacag 120

ggaaaatctc ctcagctcct ggtctataat gcaaaaacct tagcagaagg tgtgccatca 180

aggttcagtg gcagttactc aggcacacag ttttctctga atatcaacag cctgcagtct 240

gaagattttg ggatttatta ctgtcaacat catcatgata ctccgctcac gttcggtgct 300

gggaccaagc tggatctgaa a 321

<210> 87
<211> 348
<212> DNA
<213> Mus musculus

<400> 87
caggttcagc tgcagcagtc tggagctgag ctgatgaagc ctggggcctc aatgagactt 60

tcctgcaagg ctactggcta cacattcact ggctactggc tagagtggat aaagcggagg 120

cctggacatg gccttgagtg gattggagag attttacctg gaagaggtag tactaattac 180

aatgagaaat tcaagggcaa ggccacattc actgcagaga catcttccaa caccgcctac 240

atgcaactca ccagcctgac aactgaggac tctgccatct attactgtgc aaggggggga 300

cgcgactttg acaactgggg ccaaggcacc actctcacag tctcctca 348

<210> 88
<211> 321
<212> DNA

<213>  Mus musculus

<400>  88

gatatccaga tgacacagag tacatcctcc ctgtctgcct ctctgggaga cagagtcacc        60

atcagttgca gtgcaagtca gggcattaac aatcatttaa attggtatca gcagaaacca       120

gatggaactg ttaaactcct gatctattat acatcaagtt tacactcagg agtcccatca       180

aggttcagtg gcagtgggtc tgggacagat tattctctca ccatcagcaa cctggaacct       240

gaagatattg ccatttacta ttgtcagcag tttagtaagc ttccgtacac gttcggaggg       300

gggaccaaag tggaaatgaa a                                                 321

<210>  89

<211>  348

<212>  DNA

<213>  Mus musculus

<400>  89

caggttcagc tgcagcagtc tggagctgaa ctgatgaagc ctggggcctc agtgaagctt        60

tcctgcaagg ctactggcta cacattcact ggctattgga tagagtggat aaagcagagg       120

cctggacatg gccttgagtg gattggagag attttacctg gaagaggtag tactgactac 180

aatgagaagt tcaagggcaa ggccacattc actgcagata catcctccaa tacagcctac 240

atgcagctca gcagcctgac aactgaagac tctgccatct attattgtac aagagggaga 300

ctggacttta cttactgggg ccaagggact ctggtcactg tctctgca 348

<210> 90

<211> 321

<212> DNA

<213> Mus musculus

<400> 90

gatatccaga tgacacagac tacatcctcc ctgtctgcct ctctgggaga cagagtcacc 60

atcagttgca gtgcaagtca gggcattagg aattatttaa actggtatca gcagaaacca 120

gatggaactg ttaaactcct gatctattac acatcaagtt tacactcagg agtcccatca 180

aggttcagtg gcagtgggtc tgggacagat tattctctca ccatcagcaa cctggaacct 240

gaagatattg ccacttacta ttgtcagcag tatagtaagg ttccgtggac gttcggtgga 300

ggcaccaagc tggaaatcaa a 321

<210>  91

<211>  360

<212>  DNA

<213>  Mus musculus

<400>  91

gatgtacaac tggtggaatc tgggggaggc ttagtgaggc ctggagggtc cctgaaactc        60

tcctgtgcag cctccggatt cactttcagt aactatgtca tgtcttgggc tcgccagtct       120

ccagagaaga ggctggagtg ggtcgcagaa attagtactg gtgggagtca cacctactat       180

tcagacactg tgacgggccg atttaccatc tccagagaca atgccaagaa agccctatac       240

ctggaaatga gcagtctgag gtctgaggac acggccatat attattgtat tatgttttac       300

tacggtagta gctattctat ggactactgg ggtcaaggaa cctcagtcac cgtctcctca       360

<210>  92

<211>  321

<212>  DNA

<213>  Mus musculus

<400>  92

gatatccaga tgacacagag tacatcctcc ctgtctgcct ctctgggaga cagaatcacc        60

atcagttgca gggcaagtca ggacattagg aattatttaa actggtatca gcagaaacca       120

gatgggactg ttaaactcct gatctaccac acatcaagat tacactcagg agtcccatca       180

aggttcagtg gcagtgggtc tggcacagat tattctctca ccattagtaa cctggaacaa       240

gaagatattg ccacttattt ttgccaacag ggtgatacgc ttccgctcac gttcggtgcc       300

gggaccaggc tggagctgaa a                                                 321

<210>  93

<211>  357

<212>  DNA

<213>  Mus musculus

<400>  93

gatgtgcagc ttcaggagtc aggacctggc atggtgaaac cttctcagtc actttccctc        60

acctgcactg tcactggcta ctccatcacc agtggttacg actggcactg gatccgacat       120

tttccaggaa acaaactgga gtggatgggc tacataagct acagtggtaa cactaactac       180

aacccatccc tcaaaagtcg aatctccatc actcataaca catctaagaa ccatttcttc 240

ctgaggttga attctgtgac tactgaggac acagccacat attactgtgc aagaggctat 300

ggtaactggg atgctatgga ctactggggt caaggaacct cagtcaccgt ctcctca 357

<210> 94

<211> 321

<212> DNA

<213> Mus musculus

<400> 94

gacattgtga tgacccagtc tcaaaaattc atgtccacat cagtaggaga cagggtcagc 60

gtcacctgca aggccagtca gattgtggac actaatgtag cctggtatca acagaaacca 120

gggcaatctc ctaaagcact gatttcctcg gcatcctacc gctacagtgg agtccctgat 180

cgcttcacag gcagtggatc tgggacagat ttcactctca ccatcagcaa tgtacagtct 240

gaagacttgg cagaatattt ctgtcagcaa tataacagct atcctctcac gttcggtgct 300

gggaccaagc tggagctgac a 321

<210>  95

<211>  351

<212>  DNA

<213>  Mus musculus

<400>  95

cagatccagt tggtacagtc tggacctgag ttgaagaagc ctggagagac agtcaagatc        60

tcctgcaagc cttctggata taccttcaca gcctatggat tgagctgggt gaaacaggct       120

ccaggacagg gtttccagtg gatgggctgg ataaacacct actctggagt gcccacatat       180

actgatgatt tcaagggacg atttgccttc tctttggaaa cctctgccgg cactgcctct       240

ttgcagatca ccaacctcaa aaatgaggac acggctactt atttctgtgc aagatcttca       300

attcccccct ttgagtactg gggcccaggc accactctca cagtctcctc a                351

<210>  96

<211>  321

<212>  DNA

<213>  Mus musculus

<400>  96

agttttgtga tgacccagag tcccaaattc ctgcttgtat cagtaggaga cagggttacc        60

ataacctgca aggccagtca gagtgtgagt aatgatgtag cttggtacca acagaagcca 120

gggcagcctc ctagagtact gatatacttt gcatccaatc gctacactgg agtccctgat 180

cgcttcactg gcagtggata tgggacggat ttcactttca ccatcagcac tgtgcaggct 240

gaagacctgg cactttattt ctgtcagcag gattatagct ctccgtggac gttcggtgga 300

ggcaccaagc tggagatcaa a 321

<210> 97

<211> 357

<212> DNA

<213> Mus musculus

<400> 97

gaggtccagc tgcaacagtc tggacctgtg ctggtgaaac ctggggcttc ggtgaagatg 60

tcctgtgagg cttctggata ctctttcatt gactactaca tgagttgggt gaagcagagc 120

cgtggaagga gccttgagtg gattggagtt tttaatcctt acagtggtga gataatttac 180

aaccagaagt tcaagggcaa ggccacattg actgttgaca agtcctccag cacagcctac 240

atggagctca gtagcctgac atctgaggac tctggagtct attcctgtgc aagattacgc 300

atctcttatt atgctatcga ttactggggt caagggacct cagtcgccgt ctcctca 357

<210> 98

<211> 324

<212> DNA

<213> Mus musculus

<400> 98

gaaaatgtgc tcacccagtc tccagcaatc atgtctgcat ctccagggga aaaggtcacc 60

atgacctgca gggccacctc aagtgttagt tccggtcact tccactggta ccagcagaag 120

tcaggtgcct cccccaaagt ctggatttat agcacatcca acttggcttc tggagtccct 180

gctcgcttca gtggcagtgg gtctgggacc tctttctctc tcacaatcag cagtgtggag 240

gctgaagatg tagccactta tttctgccag caatacagtg gttacccact cacgttcgga 300

ggggggacca agctggaaat aaaa 324

<210> 99

<211> 351

<212>  DNA

<213>  Mus musculus

<400>  99

gaggtgcaac tggtggagtc tgggggaggc ttagtgaagc ctggagggtc cctgaaactc      60

tcctgtgcag cctctggatt cactttcagt gactatggaa tgcactgggt tcgtcaggct      120

ccagagaagg gactggagtg ggttggatac attagtagtg gcacttatgt cgtctactat      180

gcagacacag tgaagggccg attcaccatc tccagagaca atgccaagaa caccctgttc      240

ctgcaaatga ccagtctgag gtctgaggac acggccatat attactgttc aacgtacggt      300

agtaaccctt ttgactactg gggccaaggc accactctca cagtctcctc a              351

<210>  100

<211>  324

<212>  DNA

<213>  Mus musculus

<400>  100

caacttgttc tcacccagtc tccagcaatc atgtctgcat ccccagggga gaaggtcacc      60

ataacctgca gtgccacctc aagtgtaagt tccatgcact ggttccagca gaagccagtc      120

acttctccca aactctggat ttatagcacc tccaatttgg cttctggagt ccctactcgc 180

ttcagtggca gtggatctgg gacctcttac tctctcacaa tcagccgaat ggaggctgaa 240

gatgctgcca cttattactg ccagcaaagg agtggttacc cacccatatt cacgttcggc 300

tcggggacaa agttggaaat aaaa 324

## Description

The present application claims priority from Chinese patent application NO: CN201510990545.6 filed on December 24, 2015. The entire content of which is hereby incorporated by reference.

### Field of the Invention

The present invention relates to the field of antibody, specifically relates to a TPBG antibody and method of preparation, conjugates and use thereof.

### Background

A cell surface molecule, trophoblast glycoprotein (TPBG, also known as 5T4), was found to be a protein specifically expressed by embryonic trophoblast when comparing embryonic stem cell trophoblast with cancer cells. The human TPBG comprises 420 amino acid residues, which has a molecular weight of approximately 72kDa. The diverse structure of N-terminal oligosaccharides, which can prevent proteolysis, interact with other molecules in the process of signal transduction on plasma membrane. The TPBG protein includes a total of seven leucine-rich repeats (LRR), which can participate in protein-protein interaction.

Trophoblast is a special layer of embryonic stem cells between the placenta and the fetus, and TPBG is widely expressed in various types of trophoblast cells during the embryonic development. Whereas TPBG has a limited expression by several types of epithelial cells in normal adult tissues, it can be detected in various types of cancer cells, e.g. uterine cancer, colon cancer, gastric cancer, ovarian cancer, oral cancer, prostate cancer, lung cancer or renal cancer. For colon cancer, gastric cancer or ovarian cancer, there is evidence that the expression level of TPBG is related to the poor cure rate of cancer. And the expression of TPBG is as high as 95% in tissues of non-small cell lung cancer, renal cancer or pancreatic cancer.

Numerous studies show that overexpression of TPBG could promote cell migration as well as escape immune surveillance. Overexpression of TPBG in mouse fibroblast could induce the exhibition of spindle-like cell morphology and reduce cell adhesion. In normal mouse epithelial cells, it was also discovered that TPBG could inhibit the expression of epithelial cell cadherin (E-cadherin) and promote cell migration. However, the intracellular moiety of TPBG has the function of inhibiting the formation of cytoskeleton. Meanwhile, TPBG is associated with epithelial-mesenchymal transition (EMT), and as an early marker of embryonic stem cell development, it can increase the proteinase activities in cellular interstitial substance and interfere with the arrangement of actin cytoskeleton, thereby down-regulating the expression level of E-cadherin. Other studies discovered that the colocalization of TPBG and CXCR4 on plasma membrane can induce the binding of its ligand chemokine CXCL12 and promote the spread of inflammation and tumor. In TPBG-negative cells, the binding of CXCL12 to another receptor CXCR7 inhibits chemotaxis, facilitating cell growth and survival. Wnt/b-catenin signaling pathway plays a crucial role in development and cell regeneration. TPBG inhibits Wnt signaling pathway through inhibition of the endocytosis of LRP6 and Wnt receptors, thus inhibiting cell adhesion and the formation of cytoskeleton and facilitating metastasis and proliferation of tumor. There is also evidence that TPBG participates in the non-canonical Wnt pathway in the cells of breast cancer and gastric cancer, similarly facilitating the metastasis and infiltration of cancer cells.

Antibody-drug conjugates are conjugates formed by coupling an antibody with a highly potent small-molecule drug through a linker, which enables the highly toxic small-molecule drug to specifically recognize the target proteins on cancer cells, and thus to specifically kill cancer cells. In the past century, antibody-based immunotherapy and chemical drug-based chemotherapy have been the two major therapeutic strategies for cancer treatment in clinical practice. Antibodies target antigens overexpressed by tumor cells, so that various therapeutic monoclonal antibodies have achieved great success in the clinic. In clinical practice, therapeutic antibodies have excellent targeting, however, the cytotoxicity of therapeutic antibodies are limited. Small-molecule drugs have an efficient cytotoxicity effects on cancer cells, but they also inflict harm on non-cancer cells. Due to the limitations of antibody drug and small-molecule drug in clinic, new demands have been put forward for drug research and development. The new generation of antibody-drug conjugates have achieved highly efficient targeting cytotoxicity effects on cancer cells by delivering highly cytotoxic chemical drugs utilizing the binding specificity of antibody to its target cells. With the emergence of new chemical linker technology, antibody-drug conjugates began to enter clinical research in the late 1980s, and now two ADC drugs have received FDA marketing approval.

The development of ADC drugs involves several aspects, including the screening of drug targets, the preparation of recombinant antibodies, the development of linker techniques and the screening of highly cytotoxic chemical compounds. As a cancer cell-specific protein, TPBG is a candidate target for ADC drugs.

### Detailed Description of the Invention

The technical problem to be solved by present invention is to overcome the deficiency of lacking TPBG antibody and provide a high affinity and high specificity of TPBG antibody, preparation method thereof and use thereof. The TPBG antibody shows high affinity with human, mouse and cynomolgus derived TPBG protein. The present invention further provides a conjugate of an active pharmaceutical ingredient (API), which comprises the TPBG antibody and the conjugated small-molecule chemical compound having anti-tumor effects. The conjugate can enter the cells, develop cytotoxicity effects on TPBG-positive cells, which is able to be used in the preparation of drugs for treating tumors and the like.

As described herein, the lead antibody of TPBG antibody was obtained by adapting conventional hybridoma preparation technique (Kohler and Milstein, Nature, 1975, 25:495) through a series of adjustments and improvements, using human TPBG protein or recombinant cell strain overexpressing human TPBG protein as immunogen. The TPBG antibody having high affinity with human TPBG protein etc. were obtained by preliminarily production, purification and identification of the lead antibody. The TPBG antibody was conjugated with a small-molecule compound, e.g. MMAF, to form a conjugate, which is able to enter cells and shows remarkable cytotoxicity effects on TPBG-positive cells. The amino acid sequences of the heavy chain variable region and the light chain variable region of the TPBG antibody were then obtained by molecular biology methods, such as sequencing.

The present invention provides an isolated protein comprising one or more of heavy chain CDR1, heavy chain CDR2 and heavy chain CDR3 of TPBG antibody, and/or, one or more of light chain CDR1, light chain CDR2 and light chain CDR3 of TPBG antibody, wherein the amino acid sequence of the heavy chain CDR1 is set forth as SEQ ID NO:2, SEQ ID NO:10, SEQ ID NO:18, SEQ ID NO:26, SEQ ID NO:34, SEQ ID NO:42, SEQ ID NO:50, SEQ ID NO:58, SEQ ID NO:66 or SEQ ID NO:74; the amino acid sequence of the heavy chain CDR2 is set forth as SEQ ID NO:3, SEQ ID NO:11, SEQ ID NO:19, SEQ ID NO:27, SEQ ID NO:35, SEQ ID NO:43, SEQ ID NO:51, SEQ ID NO:59, SEQ ID NO:67 or SEQ ID NO:75; the amino acid sequence of the heavy chain CDR3 is set forth as SEQ ID NO:4, SEQ ID NO:12, SEQ ID NO:20, SEQ ID NO:28, SEQ ID NO:36, SEQ ID NO:44, SEQ ID NO:52, SEQ ID NO:60, SEQ ID NO:68 or SEQ ID NO:76; the amino acid sequence of the light chain CDR1 is set forth as SEQ ID NO:6, SEQ ID NO:14, SEQ ID NO:22, SEQ ID NO:30, SEQ ID NO:38, SEQ ID NO:46, SEQ ID NO:54, SEQ ID NO:62, SEQ ID NO:70 or SEQ ID NO:78; the amino acid sequence of the light chain CDR2 is set forth as SEQ ID NO:7, SEQ ID NO:15, SEQ ID NO:23, SEQ ID NO:31, SEQ ID NO:39, SEQ ID NO:47, SEQ ID NO:55, SEQ ID NO:63, SEQ ID NO:71 or SEQ ID NO:79; the amino acid sequence of the light chain CDR3 is set forth as SEQ ID NO:8, SEQ ID NO:16, SEQ ID NO:24, SEQ ID NO:32, SEQ ID NO:40, SEQ ID NO:48, SEQ ID NO:56, SEQ ID NO:64, SEQ ID NO:72 or SEQ ID NO:80; or the amino acid sequence of the heavy chain CDR1 has at least 80% sequence identity to the sequence set forth as SEQ ID NO:2, SEQ ID NO:10, SEQ ID NO:18, SEQ ID NO:26, SEQ ID NO:34, SEQ ID NO:42, SEQ ID NO:50, SEQ ID NO:58, SEQ ID NO:66 or SEQ ID NO:74; the amino acid sequence of the heavy chain CDR2 has at least 80% sequence identity to the sequence set forth as SEQ ID NO:3, SEQ ID NO:11, SEQ ID NO:19, SEQ ID NO:27, SEQ ID NO:35, SEQ ID NO:43, SEQ ID NO:51, SEQ ID NO:59, SEQ ID NO:67 or SEQ ID NO:75; the amino acid sequence of the heavy chain CDR3 has at least 80% sequence identity to the sequence set forth as SEQ ID NO:4, SEQ ID NO:12, SEQ ID NO:20, SEQ ID NO:28, SEQ ID NO:36, SEQ ID NO:44, SEQ ID NO:52, SEQ ID NO:60, SEQ ID NO:68 or SEQ ID NO:76; the amino acid sequence of the light chain CDR1 has at least 80% sequence identity to the sequence set forth as SEQ ID NO:6, SEQ ID NO:14, SEQ ID NO:22, SEQ ID NO:30, SEQ ID NO:38, SEQ ID NO:46, SEQ ID NO:54, SEQ ID NO:62, SEQ ID NO:70 or SEQ ID NO:78; the amino acid sequence of the light chain CDR2 has at least 80% sequence identity to the sequence set forth as SEQ ID NO:7, SEQ ID NO:15, SEQ ID NO:23, SEQ ID NO:31, SEQ ID NO:39, SEQ ID NO:47, SEQ ID NO:55, SEQ ID NO:63, SEQ ID NO:71 or SEQ ID NO:79; the amino acid sequence of the light chain CDR3 has at least 80% sequence identity to the sequence set forth as SEQ ID NO:8, SEQ ID NO:16, SEQ ID NO:24, SEQ ID NO:32, SEQ ID NO:40, SEQ ID NO:48, SEQ ID NO:56, SEQ ID NO:64, SEQ ID NO:72 or SEQ ID NO:80.

Preferably, the amino acid sequence of the heavy chain CDR1 is set forth as SEQ ID NO:2, the amino acid sequence of the heavy chain CDR2 is set forth as SEQ ID NO:3, and the amino acid sequence of the heavy chain CDR3 is set forth as SEQ ID NO:4; the amino acid sequence of the heavy chain CDR1 is set forth as SEQ ID NO:10, the amino acid sequence of the heavy chain CDR2 is set forth as SEQ ID NO:11, and the amino acid sequence of the heavy chain CDR3 is set forth as SEQ ID NO:12; the amino acid sequence of the heavy chain CDR1 is set forth as SEQ ID NO:18, the amino acid sequence of the heavy chain CDR2 is set forth as SEQ ID NO:19, and the amino acid sequence of the heavy chain CDR3 is set forth as SEQ ID NO:20; the amino acid sequence of the heavy chain CDR1 is set forth as SEQ ID NO:26, the amino acid sequence of the heavy chain CDR2 is set forth as SEQ ID NO:27, and the amino acid sequence of the heavy chain CDR3 is set forth as SEQ ID NO:28; the amino acid sequence of the heavy chain CDR1 is set forth as SEQ ID NO:34, the amino acid sequence of the heavy chain CDR2 is set forth as SEQ ID NO:35, and the amino acid sequence of the heavy chain CDR3 is set forth as SEQ ID NO:36; the amino acid sequence of the heavy chain CDR1 is set forth as SEQ ID NO:42, the amino acid sequence of the heavy chain CDR2 is set forth as SEQ ID NO:43, and the amino acid sequence of the heavy chain CDR3 is set forth as SEQ ID NO:44; the amino acid sequence of the heavy chain CDR1 is set forth as SEQ ID NO:50, the amino acid sequence of the heavy chain CDR2 is set forth as SEQ ID NO:51, and the amino acid sequence of the heavy chain CDR3 is set forth as SEQ ID NO:52; the amino acid sequence of the heavy chain CDR1 is set forth as SEQ ID NO:58, the amino acid sequence of the heavy chain CDR2 is set forth as SEQ ID NO:59, and the amino acid sequence of the heavy chain CDR3 is set forth as SEQ ID NO:60; the amino acid sequence of the heavy chain CDR1 is set forth as SEQ ID NO:66, the amino acid sequence of the heavy chain CDR2 is set forth as SEQ ID NO:67, and the amino acid sequence of the heavy chain CDR3 is set forth as SEQ ID NO:68; the amino acid sequence of the heavy chain CDR1 is set forth as SEQ ID NO:74, the amino acid sequence of the heavy chain CDR2 is set forth as SEQ ID NO:75, and the amino acid sequence of the heavy chain CDR3 is set forth as SEQ ID NO:76; the amino acid sequence of the light chain CDR1 is set forth as SEQ ID NO: 6, the amino acid sequence of the light chain CDR2 is set forth as SEQ ID NO:7, and the amino acid sequence of the light chain CDR3 is set forth as SEQ ID NO:8; the amino acid sequence of the light chain CDR1 is set forth as SEQ ID NO: 14, the amino acid sequence of the light chain CDR2 is set forth as SEQ ID NO:15, and the amino acid sequence of the light chain CDR3 is set forth as SEQ ID NO:16; or the amino acid sequence of the light chain CDR1 is set forth as SEQ ID NO: 22, the amino acid sequence of the light chain CDR2 is set forth as SEQ ID NO:23, and the amino acid sequence of the light chain CDR3 is set forth as SEQ ID NO:24; the amino acid sequence of the light chain CDR1 is set forth as SEQ ID NO: 30, the amino acid sequence of the light chain CDR2 is set forth as SEQ ID NO:31, and the amino acid sequence of the light chain CDR3 is set forth as SEQ ID NO:32; the amino acid sequence of the light chain CDR1 is set forth as SEQ ID NO: 38, the amino acid sequence of the light chain CDR2 is set forth as SEQ ID NO:39, and the amino acid sequence of the light chain CDR3 is set forth as SEQ ID NO:40; the amino acid sequence of the light chain CDR1 is set forth as SEQ ID NO: 46, the amino acid sequence of the light chain CDR2 is set forth as SEQ ID NO:47, and the amino acid sequence of the light chain CDR3 is set forth as SEQ ID NO:48; the amino acid sequence of the light chain CDR1 is set forth as SEQ ID NO: 54, the amino acid sequence of the light chain CDR2 is set forth as SEQ ID NO:55, and the amino acid sequence of the light chain CDR3 is set forth as SEQ ID NO:56; the amino acid sequence of the light chain CDR1 is set forth as SEQ ID NO: 62, the amino acid sequence of the light chain CDR2 is set forth as SEQ ID NO:63, and the amino acid sequence of the light chain CDR3 is set forth as SEQ ID NO:64; the amino acid sequence of the light chain CDR1 is set forth as SEQ ID NO: 70, the amino acid sequence of the light chain CDR2 is set forth as SEQ ID NO:71, and the amino acid sequence of the light chain CDR3 is set forth as SEQ ID NO:72; or the amino acid sequence of the light chain CDR1 is set forth as SEQ ID NO: 78, the amino acid sequence of the light chain CDR2 is set forth as SEQ ID NO:79, and the amino acid sequence of the light chain CDR3 is set forth as SEQ ID NO:80.

The present invention further provides an isolated protein comprising the heavy chain variable region of TPBG antibody and/or the light chain variable region of TPBG antibody. The amino acid sequence of the heavy chain variable region is set forth as SEQ ID NO:1, SEQ ID NO:9, SEQ ID NO:17, SEQ ID NO:25, SEQ ID NO:33, SEQ ID NO:41, SEQ ID NO:49, SEQ ID NO:57, SEQ ID NO:65 or SEQ ID NO:73; the amino acid sequence of the light chain variable region is set forth as SEQ ID NO:5, SEQ ID NO:13, SEQ ID NO:21, SEQ ID NO:29, SEQ ID NO:37, SEQ ID NO:45, SEQ ID NO:53, SEQ ID NO:61, SEQ ID NO:69 or SEQ ID NO:77.

Preferably, the amino acid sequence of the heavy chain variable region is set forth as SEQ ID NO:1, and the amino acid sequence of the light chain variable region is set forth as SEQ ID NO:5; the amino acid sequence of the heavy chain variable region is set forth as SEQ ID NO:9, and the amino acid sequence of the light chain variable region is set forth as SEQ ID NO:13; the amino acid sequence of the heavy chain variable region is set forth as SEQ ID NO:17, and the amino acid sequence of the light chain variable region is set forth as SEQ ID NO:21; the amino acid sequence of the heavy chain variable region is set forth as SEQ ID NO:25, and the amino acid sequence of the light chain variable region is set forth as SEQ ID NO:29; the amino acid sequence of the heavy chain variable region is set forth as SEQ ID NO:33, and the amino acid sequence of the light chain variable region is set forth as SEQ ID NO:37; the amino acid sequence of the heavy chain variable region is set forth as SEQ ID NO:41, and the amino acid sequence of the light chain variable region is set forth as SEQ ID NO:45; the amino acid sequence of the heavy chain variable region is set forth as SEQ ID NO:49, and the amino acid sequence of the light chain variable region is set forth as SEQ ID NO:53; the amino acid sequence of the heavy chain variable region is set forth as SEQ ID NO:57, and the amino acid sequence of the light chain variable region is set forth as SEQ ID NO:61; the amino acid sequence of the heavy chain variable region is set forth as SEQ ID NO:65, and the amino acid sequence of the light chain variable region is set forth as SEQ ID NO:69; or the amino acid sequence of the heavy chain variable region is set forth as SEQ ID NO:73, and the amino acid sequence of the light chain variable region is set forth as SEQ ID NO:77.

In summary, the SEQ ID numbers of the above amino acid sequences are listed in Table 1:

**Table 1 Protein Sequence NOs of the TPBG antibody**

| clone number | heavy chain protein | | | | light chain protein | | | |
|---|---|---|---|---|---|---|---|---|
| | variable region | CDR1 | CDR2 | CDR3 | variable region | CDR1 | CDR2 | CDR3 |
| 12B12C7C3 | 1 | 2 | 3 | 4 | 5 | 6 | 7 | 8 |
| 5G4H10G5 | 9 | 10 | 11 | 12 | 13 | 14 | 15 | 16 |
| 37H9C5G2 | 17 | 18 | 19 | 20 | 21 | 22 | 23 | 24 |
| 39A11G5F2 | 25 | 26 | 27 | 28 | 29 | 30 | 31 | 32 |
| 52C9E9F6 | 33 | 34 | 35 | 36 | 37 | 38 | 39 | 40 |
| 28D4E6A9 | 41 | 42 | 43 | 44 | 45 | 46 | 47 | 48 |
| 36A10D8B12 | 49 | 50 | 51 | 52 | 53 | 54 | 55 | 56 |
| 99E12C7H1 | 57 | 58 | 59 | 60 | 61 | 62 | 63 | 64 |
| 103E2E9C2 | 65 | 66 | 67 | 68 | 69 | 70 | 71 | 72 |
| 106D5G3D10 | 73 | 74 | 75 | 76 | 77 | 78 | 79 | 80 |

wherein, the numbers in Table 1 are the SEQ ID NOs in the sequence listing, e.g. the amino acid sequence of the heavy chain protein variable region of 12B 12C7C3 is set forth as SEQ ID NO:1, while the amino acid sequence of CDR1 in the heavy chain protein variable region of 12B 12C7C3 is set forth as SEQ ID NO:2.

Preferably, the protein further comprises heavy chain constant region of antibody and/or light chain constant region of antibody. The heavy chain constant region of antibody is conventional heavy chain constant region of antibody known in the art, preferably mouse or human heavy chain constant region of antibody, more preferably human heavy chain constant region. The light chain constant region of antibody is conventional light chain constant region of antibody known in the art, preferably mouse or human light chain constant region of antibody, more preferably human light chain constant region.

The protein is a conventional protein known in the art, preferably TPBG antibody, more preferably one or more of full-length antibody, protein fragment of antigen-antibody binding domain, bi-specific antibody, multi-specific antibody, single chain antibody fragment (scFv), single domain antibody (sdAb), single region antibody, and monoclonal antibodies or polyclonal antibodies prepared by the above antibodies. The monoclonal antibodies can be developed by various means and techniques, including the hybridoma technique, phage display technique, single lymphocyte gene cloning technique, etc. The monoclonal antibodies are mainly prepared from wild-type or transgenic mice by the hybridoma technique.

The full-length antibody is a conventional full-length antibody known in the art, which comprises heavy chain variable region, light chain variable region, heavy chain constant region and light chain constant region. The heavy chain variable region and light chain variable region, along with human heavy chain constant region and human light chain constant region, constitute the full-length human antibody. Preferably, the full-length antibody is IgG1, IgG2, IgG3 or IgG4.

The single chain antibody is a conventional single chain antibody known in the art, which comprises heavy chain variable region, light chain variable region and a short peptide of 15-20 amino acids.

The protein fragment of antigen-antibody binding domain is a conventional protein fragment of antigen-antibody binding domain known in the art, which comprises light chain variable region, light chain constant region and Fc fragment of the heavy chain constant region. Preferably, the protein fragment of antigen-antibody binding domain is Fab and F(ab')2.

The single domain antibody is a conventional single domain antibody known in the art, which comprises heavy chain variable region and heavy chain constant region.

The single region antibody is a conventional single region antibody known in the art, which comprises only heavy chain variable region.

As described herein, the preparation method of protein is a preparation method of protein known in the art, preferably isolating the protein from the expression transformant recombinantly expressing the protein or artificial synthesis of the amino acid sequence of the protein. Preferably, the method for isolating the protein from the transformant recombinantly expressing the protein is as follows: the nucleic acid sequence that encodes the protein and contains a site mutation was inserted into the recombinant vector, the recombinant vector was transformed into a transformant, thus a recombinant expression transformant was obtained, and the protein from the culture of the recombinant expression transformant was purified and isolated.

The present invention further provides a nucleic acid encoding the above-mentioned protein.

Preferably, the nucleotide sequence of the nucleic acid encoding the heavy chain variable region is set forth as SEQ ID NO:81, SEQ ID NO:83, SEQ ID NO:85, SEQ ID NO:87, SEQ ID NO:89, SEQ ID NO:91, SEQ ID NO:93, SEQ ID NO:95, SEQ ID NO:97 or SEQ ID NO:99; and/or the nucleotide sequence encoding the light chain variable region is set forth as SEQ ID NO:82, SEQ ID NO:84, SEQ ID NO:86, SEQ ID NO:88, SEQ ID NO:90, SEQ ID NO:92, SEQ ID NO:94, SEQ ID NO:96, SEQ ID NO:98 or SEQ ID NO:100.

More preferably, the nucleotide sequence of the nucleic acid encoding the heavy chain variable region is set forth as SEQ ID NO:81, and the nucleotide sequence encoding the light chain variable region is set forth as SEQ ID NO:82; the nucleotide sequence encoding the heavy chain variable region is set forth as SEQ ID NO:83, and the nucleotide sequence encoding the light chain variable region is set forth as SEQ ID NO:84; the nucleotide sequence encoding the heavy chain variable region is set forth as SEQ ID NO:85, and the nucleotide sequence encoding the light chain variable region is set forth as SEQ ID NO:86; the nucleotide sequence encoding the heavy chain variable region is set forth as SEQ ID NO:87, and the nucleotide sequence encoding the light chain variable region is set forth as SEQ ID NO:88; the nucleotide sequence encoding the heavy chain variable region is set forth as SEQ ID NO:89, and the nucleotide sequence encoding the light chain variable region is set forth as SEQ ID NO:90; the nucleotide sequence encoding the heavy chain variable region is set forth as SEQ ID NO:91, and the nucleotide sequence encoding the light chain variable region is set forth as SEQ ID NO:92; the nucleotide sequence encoding the heavy chain variable region is set forth as SEQ ID NO:93, and the nucleotide sequence encoding the light chain variable region is set forth as SEQ ID NO:94; the nucleotide sequence encoding the heavy chain variable region is set forth as SEQ ID NO:95, and the nucleotide sequence encoding the light chain variable region is set forth as SEQ ID NO:96; the nucleotide sequence encoding the heavy chain variable region is set forth as SEQ ID NO:97, and the nucleotide sequence encoding the light chain variable region is set forth as SEQ ID NO:98; the nucleotide sequence encoding the heavy chain variable region is set forth as SEQ ID NO:99, and the nucleotide sequence encoding the light chain variable region is set forth as SEQ ID NO:100.

In summary, the SEQ ID numbers of above nucleotide sequences are listed in Table 2:

**Table 2 NOs of TPBG antibody genes**

| Clone Number | Heavy Chain Variable Region | Light Chain Variable Region |
|---|---|---|
| 12B12C7C3 | 81 | 82 |
| 5G4H10G5 | 83 | 84 |
| 37H9C5G2 | 85 | 86 |
| 39A11G5F2 | 87 | 88 |
| 52C9E9F6 | 89 | 90 |
| 28D4E6A9 | 91 | 92 |
| 36A10D8B12 | 93 | 94 |
| 99E12C7H1 | 95 | 96 |
| 103E2E9C2 | 97 | 98 |
| 106D5G3D10 | 99 | 100 |

wherein the numbers in Table 2 are the SEQ ID NOs in the sequence listing, e.g. the nucleotide sequence encoding the heavy chain variable region of 12B12C7C3 is SEQ ID NO:81;
wherein the nucleotide sequence encoding the CDR1 in the heavy chain variable region of 12B12C7C3 is the nucleotide sequence from position 91 to position 105 of SEQ ID NO:81;
the nucleotide sequence encoding the CDR2 in the heavy chain variable region of 12B12C7C3 is the nucleotide sequence from position 151 to position 198 of SEQ ID NO:81;
the nucleotide sequence encoding the CDR3 in the heavy chain variable region of 12B12C7C3 is the nucleotide sequence from position 295 to position 327 of SEQ ID NO:81;
the nucleotide sequence encoding the CDR1 in the light chain variable region of 12B12C7C3 is the nucleotide sequence from position 70 to position 114 of SEQ ID NO:82;
the nucleotide sequence encoding the CDR2 in the light chain variable region of 12B12C7C3 is the nucleotide sequence from position 157 to position 180 of SEQ ID NO:82;
the nucleotide sequence encoding the CDR3 in the light chain variable region of 12B12C7C3 is the nucleotide sequence from position 277 to position 303 of SEQ ID NO:82;
the nucleotide sequence encoding the CDR1 in the heavy chain variable region of 5G4H10G5 is the nucleotide sequence from position 91 to position 105 of SEQ ID NO:83;
the nucleotide sequence encoding the CDR2 in the heavy chain variable region of 5G4H10G5 is the nucleotide sequence from position 148 to position 198 of SEQ ID NO:83;
the nucleotide sequence encoding the CDR3 in the heavy chain variable region of 5G4H10G5 is the nucleotide sequence from position 295 to position 330 of SEQ ID NO:83;
the nucleotide sequence encoding the CDR1 in the light chain variable region of 5G4H10G5 is the nucleotide sequence from position 70 to position 102 of SEQ ID NO:84;
the nucleotide sequence encoding the CDR2 in the light chain variable region of 5G4H10G5 is the nucleotide sequence from position 148 to position 168 of SEQ ID NO:84;
the nucleotide sequence encoding the CDR3 in the light chain variable region of 5G4H10G5 is the nucleotide sequence from position 265 to position 288 of SEQ ID NO:84;
the nucleotide sequence encoding the CDR1 in the heavy chain variable region of 37H9C5G2 is the nucleotide sequence from position 91 to position 105 of SEQ ID NO:85;
the nucleotide sequence encoding the CDR2 in the heavy chain variable region of 37H9C5G2 is the nucleotide sequence from position 151 to position 198 of SEQ ID NO:85;
the nucleotide sequence encoding the CDR3 in the heavy chain variable region of 37H9C5G2 is the nucleotide sequence from position 295 to position 327 of SEQ ID NO:85;
the nucleotide sequence encoding the CDR1 in the light chain variable region of 37H9C5G2 is the nucleotide sequence from position 70 to position 102 of SEQ ID NO:86;
the nucleotide sequence encoding the CDR2 in the light chain variable region of 37H9C5G2 is the nucleotide sequence from position 148 to position 168 of SEQ ID NO:86;
the nucleotide sequence encoding the CDR3 in the light chain variable region of 37H9C5G2 is the nucleotide sequence from position 265 to position 291 of SEQ ID NO:86;
the nucleotide sequence encoding the CDR1 in the heavy chain variable region of 39A11G5F2 is the nucleotide sequence from position 91 to position 105 of SEQ ID NO:87;
the nucleotide sequence encoding the CDR2 in the heavy chain variable region of 39A11G5F2 is the nucleotide sequence from position 151 to position 198 of SEQ ID NO:87;
the nucleotide sequence encoding the CDR3 in the heavy chain variable region of 39A11G5F2 is the nucleotide sequence from position 295 to position 315 of SEQ ID NO:87;
the nucleotide sequence encoding the CDR1 in the light chain variable region of 39A11G5F2 is the nucleotide sequence from position 70 to position 102 of SEQ ID NO:88;
the nucleotide sequence encoding the CDR2 in the light chain variable region of 39A11G5F2 is the nucleotide sequence from position 148 to position 168 of SEQ ID NO:88;
the nucleotide sequence encoding the CDR3 in the light chain variable region of 39A11G5F2 is the nucleotide sequence from position 265 to position 291of SEQ ID NO:88;
the nucleotide sequence encoding the CDR1 in the heavy chain variable region of 52C9E9F6 is the nucleotide sequence from position 91 to position 105 of SEQ ID NO:89;
the nucleotide sequence encoding the CDR2 in the heavy chain variable region of 52C9E9F6 is the nucleotide sequence from position151 to position 198 of SEQ ID NO:89;
the nucleotide sequence encoding the CDR3 in the heavy chain variable region of 52C9E9F6 is the nucleotide sequence from position 295 to position 315 of SEQ ID NO:89;
the nucleotide sequence encoding the CDR1 in the light chain variable region of 52C9E9F6 is the nucleotide sequence from position 70 to position 102 of SEQ ID NO:90;
the nucleotide sequence encoding the CDR2 in the light chain variable region of 52C9E9F6 is the nucleotide sequence from position 148 to position 168 of SEQ ID NO:90;
the nucleotide sequence encoding the CDR3 in the light chain variable region of 52C9E9F6 is the nucleotide sequence from position 268 to position 291 of SEQ ID NO:90;
the nucleotide sequence encoding the CDR1 in the heavy chain variable region of 28D4E6A9 is the nucleotide sequence from position 91 to position 105 of SEQ ID NO:91;
the nucleotide sequence encoding the CDR2 in the heavy chain variable region of 28D4E6A9 is the nucleotide sequence from position 148 to position 198 of SEQ ID NO:91;
the nucleotide sequence encoding the CDR3 in the heavy chain variable region of 28D4E6A9 is the nucleotide sequence from position 295 to position 327 of SEQ ID NO:91;
the nucleotide sequence encoding the CDR1 in the light chain variable region of 28D4E6A9 is the nucleotide sequence from position 70 to position 102 of SEQ ID NO:92;
the nucleotide sequence encoding the CDR2 in the light chain variable region of 28D4E6A9 is the nucleotide sequence from position 148 to position 168 of SEQ ID NO:92;
the nucleotide sequence encoding the CDR3 in the light chain variable region of 28D4E6A9 is the nucleotide sequence from position 265 to position 291 of SEQ ID NO:92;
the nucleotide sequence encoding the CDR1 in the heavy chain variable region of 36A10D8B12 is the nucleotide sequence from position 91 to position 108 of SEQ ID NO:93;
the nucleotide sequence encoding the CDR2 in the heavy chain variable region of 36A10D8B12 is the nucleotide sequence from position 154 to position 198 of SEQ ID NO:93;
the nucleotide sequence encoding the CDR3 in the heavy chain variable region of 36A10D8B12 is the nucleotide sequence from position 295 to position 324 of SEQ ID NO:93;
the nucleotide sequence encoding the CDR1 in the light chain variable region of 36A10D8B12 is the nucleotide sequence from position 70 to position 102 of SEQ ID NO:94;
the nucleotide sequence encoding the CDR2 in the light chain variable region of 36A10D8B12 is the nucleotide sequence from position 148 to position 168 of SEQ ID NO:94;
the nucleotide sequence encoding the CDR3 in the light chain variable region of 36A10D8B12 is the nucleotide sequence from position 265 to position 291of SEQ ID NO:94;
the nucleotide sequence encoding the CDR1 in the heavy chain variable region of 99E12C7H1 is the nucleotide sequence from position 91 to position 105 of SEQ ID NO:95;
the nucleotide sequence encoding the CDR2 in the heavy chain variable region of 99E12C7H1 is the nucleotide sequence from position 151 to position 198 of SEQ ID NO:95;
the nucleotide sequence encoding the CDR3 in the heavy chain variable region of 99E12C7H1 is the nucleotide sequence from position 295 to position 318 of SEQ ID NO:95;
the nucleotide sequence encoding the CDR1 in the light chain variable region of 99E12C7H1 is the nucleotide sequence from position 70 to position 102 of SEQ ID NO:96;
the nucleotide sequence encoding the CDR2 in the light chain variable region of 99E12C7H1 is the nucleotide sequence from position 148 to position 168 of SEQ ID NO:96;
the nucleotide sequence encoding the CDR3 in the light chain variable region of 99E12C7H1 is the nucleotide sequence from position 265 to position 291 of SEQ ID NO:96;
the nucleotide sequence encoding the CDR1 in the heavy chain variable region of 103E2E9C2 is the nucleotide sequence from position 91 to position 105 of SEQ ID NO:97;
the nucleotide sequence encoding the CDR2 in the heavy chain variable region of 103E2E9C2 is the nucleotide sequence from position 151 to position 198 of SEQ ID NO:97;
the nucleotide sequence encoding the CDR3 in the heavy chain variable region of 103E2E9C2 is the nucleotide sequence from position 295 to position 324 of SEQ ID NO:97;
the nucleotide sequence encoding the CDR1 in the light chain variable region of 103E2E9C2 is the nucleotide sequence from position 70 to position 105 of SEQ ID NO:98;
the nucleotide sequence encoding the CDR2 in the light chain variable region of 103E2E9C2 is the nucleotide sequence from position 151 to position 171 of SEQ ID NO:98;
the nucleotide sequence encoding the CDR3 in the light chain variable region of 103E2E9C2 is the nucleotide sequence from position 268 to position 294 of SEQ ID NO:98;
the nucleotide sequence encoding the CDR1 in the heavy chain variable region of 106D5G3D10 is the nucleotide sequence from position 91 to position 105 of SEQ ID NO:99;
the nucleotide sequence encoding the CDR2 in the heavy chain variable region of 106D5G3D10 is the nucleotide sequence from position 151 to position 198 of SEQ ID NO:99;
the nucleotide sequence encoding the CDR3 in the heavy chain variable region of 106D5G3D10 is the nucleotide sequence from position 295 to position 318 of SEQ ID NO:99;
the nucleotide sequence encoding the CDR1 in the light chain variable region of 106D5G3D10 is the nucleotide sequence from position 70 to position 99 of SEQ ID NO:100;
the nucleotide sequence encoding the CDR2 in the light chain variable region of 106D5G3D10 is the nucleotide sequence from position 145 to position 165 of SEQ ID NO:100;
the nucleotide sequence encoding the CDR3 in the light chain variable region of 106D5G3D10 is the nucleotide sequence from position 262 to position 294 of SEQ ID NO:100;

The preparation method of the nucleic acid is a conventional preparation method of the nucleic acid known in the art, preferably comprising the following steps: the nucleic acid molecule encoding the above protein was obtained by gene cloning technique or artificial synthesis of the full sequence.

One skilled in the art should know that one homologs of the polynucleotide sequence encoding the above proteins can be provided by proper introduction of substitution, deletion, alteration, insertion or addition. The homologs of the polynucleotide in the present invention can be prepared by substitution, deletion or addition of one or more nucleotides in the gene encoding the protein sequence on condition that the antibody activity is maintained.

The present invention further provides a recombinant expression vector comprising the nucleic acid,
wherein the recombinant expression vector can be obtained by the conventional methods known in the art, i.e. ligating the nucleic acid molecule of the present invention to various expression vectors. The expression vectors are various types of vectors conventionally known in the art as long as they are able to accommodate the aforementioned nucleic acid molecule. The vectors preferably include various plasmids, cosmids, phagemids or viral vectors.

The present invention further provides a recombinant expression transformant comprising the above-mentioned recombinant expression vectors,
wherein the preparation method of the recombinant expression transformant is a preparation method conventionally known in the art, preferably transforming the above-mentioned recombinant expression vectors into host cells. The host cells are the various types of host cells conventionally known in the art, as long as they are able to stably self-replicate the above-mentioned recombinant expression vectors and efficiently express the carried nucleic acid. Preferably, the host cell is *E. coli* TG1 or BL21 cells (expressing scFv or Fab antibody), or CHO-K1 cells (expressing full-length IgG antibody). The preferred recombinant expression transformant can be obtained by transforming the above-mentioned recombinant expression plasmid into host cells, wherein the method of transformation is a conventional method of transformation known in the art, preferably chemical transformation, heat shock or electrotransformation.

The present invention further provides a preparation method of TPBG antibody comprising the following steps: culturing the above-mentioned recombinant expression transformant and obtaining TPBG antibody from the culture.

The present invention provides an immunoconjugate comprising the above-mentioned protein covalently linked to a cytotoxic agent.

Preferably, in the immunoconjugate, 1 equivalent of the above-mentioned protein is linked to y equivalents of cytotoxic agent through x equivalents of linker, which has a structure shown in formula 1:

Ab-(L)x-(D)y Formula 1

wherein Ab is the above-mentioned protein, L is a linker and D is a cytotoxic agent. The x is the crosslinking degree conventionally known in the art, x is a natural number, preferably an integer selected from 1-20; y is 0 or natural number, preferably an integer selected from 0-20; x and y are preferably integers selected from 1-2, or 2-4, or 4-8, or 8-20, independently; preferably the ratio of x to y is 1:1.

The L is a conventional linker known in the art (also known as crosslinking agent or coupling agent). The L is comprised of two functional groups, i.e. one group reacting with antibody and the other group reacting with drug (e.g. aldehyde or ketone).

The drug is coupled to the above-mentioned protein through linker. The L is released upon entry into cell, which comprises but not limited to the following functional groups: active esters, carbonates, carbamates, imine phosphate esters, oximes, hydrazones, acetals, orthoesters, amino groups, small peptides or nucleotide fragments.

Preferably, the L mainly comprises the structure shown in formula 2, which corresponds to the remaining part when the leaving group in the L is removed:

(CO-Alk¹-Sp¹-Ar-Sp²-Alk²-C(Z¹)=Q-Sp) Formula2

wherein Alk¹ and Alk² are independently bonds or branched or unbranched (C₁-C₁₀) alkylene chain; Sp¹ is -S-, -O-, -CONH-, -NHCO-, -NR'-, -N(CH₂CH₂)₂Nor -X-Ar'-Y-(CH₂)ₙ-Z, wherein X, Y and Z are independent bonds, -NR'-, -S- or -O-, provide that when n=0, at least one of Y and Z has to be a bond, and Ar' is 1, 2-, 1, 3- or 1, 4- phenylene that is optionally substituted by 1,2 or 3 groups selected from (C₁-C₅) alkyl, (C₁-C₄) alkoxy, (C₁-C₄) thioalkoxy, halogen, nitro, -COOR', -CONHR', -(CH₂)ₙCOOR', S(CH₂)ₙCOOR', -O(CH₂)ₙCONHR' or -S(CH₂)ₙCONHR', n is an integer selected from 0-5, on condition that when Alk¹ is a bond, Sp¹ is a bond; R' is a branched or unbranched (C₁-C₅) chain that is optionally substituted by 1 or 2 groups selected from -OH, (C₁-C₄) alkoxy, (C₁-C₄) thioalkoxy, halogen, nitro, (C₁-C₃) dialkylamino, or (C₁-C₃) trialkylammonium-A, wherein A is pharmaceutically acceptable anion of salt; Ar is 1, 2-; 1, 3- or 1, 4- phenylene that is optionally substituted by 1, 2 or 3 groups selected from (C₁-C₆)alkyl, (C₁-C₅) alkoxy, (C₁-C₄) thioalkoxy, halogen, nitro, -COOR', -CONHR', -O(CH₂)ₙCOOR', -S(CH₂)ₙCOOR', -O(CH₂)ₙCONHR" or -S(CH₂)ₙCONHR', wherein n and R' is as defined above, or Ar is 1,2-; 1,3-; 1,4-; 1,5-; 1,6-; 1,7-; 1,8-; 2,3-; 2,6- or 2,7- naphthylene, wherein naphthylene or phenothiazine is optionally substituted by 1, 2, 3 or 4 groups selected from (C₁-C₆) alkyl, (C₁-C₅) alkoxy, (C₁-C₄) thioalkoxy, halogen, nitro, -COOR', -CONHR', -O(CH₂)ₙCOOR', -S(CH₂)ₙCOOR' or -S(CH₂)ₙCONHR', wherein n and R' is as defined above, on condition that when Aris phenothiazine, Sp¹ is a bond that is connected only with nitrogen.
Sp² is a bond, -S- or -O-, provideed that when Alk² is a bond, Sp² is a bond;
Z¹ is H, (C₁-C₅) alkyl, or phenyl that is optionally substituted by 1, 2, or 3 groups selected from (C₁-C₅) alkyl, (C₁-C₅) alkoxy, (C₁-C₄) thioalkoxy, halogen, nitro, -COOR', -CONHR', -O(CH₂)ₙCOOR', -S(CH₂)ₙCOOR', -O(CH₂)ₙCONHR' or -S(CH₂)ₙCONHR', in which n and R' is as defined above;
Sp is linear or branched divalent or trivalent (C₁-C₁₈) group, divalent or trivalent aryl or heteroaryl group, divalent or trivalent (C₃-C₁₈) naphthenic or heterocyclic alkyl group, divalent or trivalent aryl or heteroaryl- aryl (C₁-C₁₈) group, divalent or trivalent naphthenic or heterocyclic alkyl- alkyl (C₁-C₁₈) group, or divalent or trivalent (C₂-C₁₈) unsaturated alkyl groups, wherein heteroaryl group is preferably furan, thiophene, N-methyl pyrroliy, pyridyl, N- methidazolyl, oxazolyl, pyrimidine, quinoline, isoquinoline, N-methyl carbazole group, amino-coumarin group, or phenazine group, and if Sp is a trivalent group, then Sp also can be optionally substituted by lower (C₁-C₅) dialkylamino, lower (C₁-C₅) alkoxy, hydroxyl, or lower (C₁-C₅) alkylthiol; and, Q is =NHNCO-, =NHNCS-, =NHNCONH-, =NHNCSNH- or =NHO-.

Preferably, Alk¹ is branched or unbranched (C₁-C₅) alkylene chain, Sp¹ is a bond, -S-, -O-, -CONH-, -NHCO- or -NR', wherein R' is as defined above, provided that when Alk¹ is bond, Sp¹ is bond;
Ar is 1, 2-, 1, 3- or 1, 4- phenylene that is optionally substituted by 1,2 or 3 groups selected from (C₁-C₆) alkyl,(C₁-C₅) alkoxy,(C₁-C₄) thioalkoxy, halogen, nitro, -COOR', -CONHR', wherein n and R' is as defined above, or Ar is 1,2-; 1,3-; 1,4-; 1,5-; 1,6-; 1,7-; 1,8-; 2,3-; 2,6- or 2,7- naphthylene that is optionally substituted by 1, 2, 3 or 4 groups selected from (C₁-C₆) alkyl, (C₁-C₅) alkoxy, (C₁-C₄) thioalkoxy, halogen, nitro, -COOR', -CONHR', -O(CH₂)ₙCOOR', -S(CH₂)ₙCOOR', -O(CH₂)ₙCONHR' or -S(CH₂)ₙCONHR'.

Z¹ is (C₁-C₅) alkyl, or phenyl that is optionally substituted by 1, 2, or 3 groups selected from (C₁-C₅) alkyl, (C₁-C₄) alkoxy, (C₁-C₄) thioalkoxy, halogen, nitro, -COOR', -CONHR', -O(CH₂)ₙCOOR', -S(CH₂)ₙCOOR', -O(CH₂)ₙCONHR' or -S(CH₂)ₙCONHR'; both Alk² and Sp² are bonds, and Sp and Q are only as defined above. The meaning of the bond mentioned above is covalent bond.

The L is preferably maleimidocaproyl (MC), maleimidocaproyl-L-valine-L-citrulline p-aminobenzyl alcohol (MC-VC-PAB) or 4-(N-maleimide methyl) cyclohexane-1-carboxylic succinimide (SMCC).

The D is a conventional cytotoxic agent known in the art, preferably selected from cytotoxin, chemotherapeutic agent, radioisotope, therapeutic nucleic acid, immunomodulator, anti-angiogenic agent, anti-proliferative apoptosis promoter, or cytolytic enzyme,
wherein, the cytotoxin is a conventional cytotoxin known in the art, which generally refers to an active agent that inhibits or prevents cell function and/or causes cell destruction, preferably selected from antibiotics, microtubulin polymerization inhibitors, alkylating agents, protein synthesis inhibitors, protein kinase inhibitors, phosphatase inhibitors, topoisomerase inhibitors, protein kinase, phosphatase, topoisomerase or cyclin, more preferably selected from doxorubicin, daunorubicin, idarubicin, aclarubicin, zorubicin, mitoxantrone, epirubicin, carirubicin, nogalamycin, menogaril, pirarubicin, valrubicin, cytarabine, gemcitabine, trifluridine, ancitabine, enocitabine, azacitidine , doxifluridine, pentostatin, broxuridine, capecitabine, cladribine, decitabine, floxuridine, fludarabine, gougerotin, puromycin, tegafur, tiazofurin, adriamycin, cisplatin, carboplatin, cyclophosphamide, dacarbazine, vinblastine sulfate, vincristine, bleomycin, mustard nitrogen, prednisone, procarbazine, methotrexate, fluorouracil, etoposide, paclitaxel and paclitaxel analogue, platin (such as cisplatin or carboplatin), mitomycin, thiotepa, taxane, daunorubicin, actinomycin, anthramycin, azaserine, tamoxifen, dolastatin, auristatin and derivatives, hemiasterlin, esperamicin or maytansine analogue, most preferably selected from monomethyl auristatin E (MMAE), monomethyl auristatin F (MMAF) or N2'-deacetyl-N2'-(3-mercapto-1-oxypropyl) methesteine (DM1),
wherein the chemotherapeutic agent is conventional chemotherapeutic agent known in the art, preferably selected from alkylating agents, alkyl sulfonic acid esters chemotherapeutic agents, aziridine chemotherapeutic agents, vinyl amides and methylmelamine chemotherapeutic agents, mustard nitrogen, nitrourea chemotherapeutic agents, antibiotics, anti-metabolites, and folic acid chemotherapeutic agents, purine analogues, pyrimidine analogues, androgens, anti-adrenalines, folic acid supplements, maytansinol, polysaccharide complexes, taxane, platin analogues or retinoids, or pharmaceutically acceptable salts, acids, and derivatives thereof.

The alkylating agent is conventional alkylating agent known in the art, preferably selected from thiotepa or cyclophosphamide. The alkyl sulfonic acid ester chemotherapeutic agent is conventional alkyl sulfonic acid ester chemotherapeutic agent known in the art, preferably selected from busulfan, improsulfan or piposulfan. The aziridine chemotherapeutic agent is conventional aziridine chemotherapeutic agent known in the art, preferably selected from the aziridine, such as carboquinone, meturedepa, or uredepa. The ethernamides and methylmelamine chemotherapeutic agent is conventional ethernamides and methylmelamine chemotherapeutic agent known in the art, preferably selected from hexamethyl melamine, triethyl melamine, triethylenephosphoramide, triethylenethiophosphoramide or trimethylolmelamine. The mustard nitrogen is conventional mustard nitrogen known in the art, preferably selected from phenylbutyrate mustard, naphthalene mustard, estramustine, isosphosphamide, mustard nitrogen, mechlorethaminoxide hydrochloride, melphalan, novembichin, phenesterine, prednimustine, trofosfamide or uracilmustard. The nitrourea chemotherapeutic agent is conventional nitrourea chemotherapeutic agent known in the art, preferably selected from carmustine, chlorozotocin, fotemustine, lomustin, nimustine or ranimustine. The antibiotic is conventional antibiotic known in the art, preferably selected from aclacinomycin, actinomycin, anthramycin, azaserine, bleomycin, actinomycin C, calicheamicin, carabicin, caminomycin, carzinophilin, chromomycin, dactinomycin, daunorubicin, detorubicin, and 6-Diazo-5-Oxo-L-Norleucine, doxorubicin, epirubicin, esorubicin, darubicin, marcellomycin, mitomycin, mycophenolic acid, nogalamycin, olivomycin, peplomycin, potfiromycin, puromycin, triferricdoxorubicin, rodorubicin, streptonigrin, streptozocin, tuberculocidin, ubenimex, zinostatin or zorubicin. The anti-metaboilte is conventional anti-metaboilte known in the art, preferably selected from methotrexate and 5-fluorouracil (5-FU). The folic acid chemotherapeutic agent is conventional folic acid chemotherapeutic agent known in the art, preferably selected from denopterin, pteropterin, or trimetrexate. The purine analogue is a conventional analogue known in the art, preferably selected from fludarabine, 6-mercaptopurine, tiamiprine or thioguanine. The pyrimidine analogue is conventional pyrimidine analogue known in the art, preferably selected from ancitabine, azacitidine, 6-azauridine, carmofur, cytarabine, dideoxyuridine, doxifluridine, enocitabine, floxuridine, or 5-EU. The androgen is conventional androgen known in the art, preferably selected from calusterone, Dromostanolone propionate, epitiostanol, methasterone or testolactone. The anti-adrenaline is conventional anti-adrenaline known in the art, preferably selected from aminoglutethimide, mitotane or trilostane. The folic acid supplement agent is folic acid supplement agent known in the art, preferably selected from frolinic acid, aceglatone, aldophosphamide glycoside, aminolevulinic acid, amsacrine, atrimustine, bisantrene, edatraxate, defofamine, demecolcine, diaziquone, eflornithine, elliptinium acetate, epothilone, etoglucid, gallium nitrate, hydroxyurea, lentinan or lonidainine. The maytansinol is conventional maytansinol known in the art, preferably selected from maytansine, ansamitocin, mitoguazone, mitoxantrone, mopidanmol, nitrcerine, pentostatin, phenamet, pirarubicin, losoxantrone, podophyllinic acid, 2-ethyl hydrazide or procarbazine. The polysaccharide complex is conventional polysaccharide complex known in the art, preferably selected from razoxane, rhizomycin, sizofiran, spirogermanium, tenuazonic acid, triaziquone, 2,2',2"-trichloro-triethylamine, trichothecene, urethane, vindesine, dacarbazine, mannomustine, dibromannitol, mitolactol, pipobroman, gacytosine, cytarabine, cyclophosphamide, or thiotepa, more preferably selected from T-2 toxin, verracurin A, roridin A, or anguidine. The taxane is conventional taxane known in the art, preferably selected from paclitaxel, unhydrogenated castor oil, Abraxane Nanoparticle (American Pharmaceutical Partners, Schaumberg, Illinois), docetaxel, chlorambuci, gemcitabin, 6-thioguanine, mercaptopurine or methotrexate. The platinum analogue is conventional platinum analogue known in the art, preferably selected from cisplatin, carboplatin, vinblastine, etoposid, ifosfamide, mitoxantrone, vinorelbine, novantrone, teniposide, edatrexate, daunomycin, aminopterin, capecitabine ibandronate, CPT-11, topoisomerase inhibitors RFS 2000 or difluorometylornithine. The retinoid is conventional retinoid known in the art, preferably selected from retinoic acid;
wherein the radioisotope is conventional radioisotope known in the art, which preferably binding directly to the above-mentioned proteins or bind to the above-mentioned proteins via chelating agents. More preferably, binding directly to the cysteine residues of the proteins. Preferably, the radioisotope is selected from alpha emitters, beta emitters and auger electrons suitable for radiation therapy, and positron emitters or gamma emitters suitable for diagnosis. More preferably, the radioisotope is selected from 18 fluorine, 64 copper and 65 copper. 67 gallium, 68 gallium, 77 bromine, 80m bromine, 95 ruthenium, 97 ruthenium, 103 ruthenium, 105 ruthenium, 99m technetium, 107 mercury, 203 mercury, 123 iodine, 124 iodine, 125 iodine, 126 iodine, 99m rhenium, 105 rhenium, 186 rhenium, 188 rhenium, 121m tellurium, 122m tellurium, 125m tellurium, 165thulium, 167thulium, 168thulium, 90thulium, 213bismuth, 213lead or 225actinide, or derivatives of nitrides or oxides thereof;
wherein the therapeutic nucleic acid is conventional therapeutic nucleic acid known in the art, preferably genes encoding immunomodulato, anti-angiogenic agents, anti-proliferative agents or pro-apoptotic agents. The therapeutic agents comprise the therapeutic agents, derivatives thereof and pharmaceutically acceptable salts, acids and derivatives of the therapeutic agents;
wherein the immunomodulator is conventional immunomodulator known in the art, which triggers immune responses, including humoral immune responses (e.g. production of an antigen specific antibody), and cell mediated immune responses (e.g. lymphocyte proliferation), preferably selected from cytokines, growth factors, hormones, anti-hormone drugs, immunosuppressants or corticosteroids. The cytokine is conventional known cytokine in the art, preferably selected from xanthine, interleukin or interferon. The growth factor is conventional known growth factor in the art, preferably selected from TNF, CSF, GM-CSF or G-CSF. The hormone is conventional hormone known in the art, preferably selected from estrogen, androgen or progestogen. More preferably, the estrogen is diethylstilbestrol or estradiol. More preferably, the androgen is testosterone or fluoxymesterone. More preferably, the progesterone is megestrol acetate or medroxyprogesterone acetate. The corticosteroid is conventional corticosteroid known in the art, preferably selected from prednisone, dexamethasone or cortisone. The anti-steroid drug is conventional anti-steroid drug known in the art, which can block the effect of hormones on tumor, inhibit the production of cytokines, down-regulate the expression of autoantigens, or mask the immunosuppressant of MHC antigen, preferably selected from anti-estrogen drugs, anti-androgen drugs or anti-adrenergic drugs. More preferably, the anti-estrogen drugs are selected from tamoxifen, raloxifene, aromatase inhibitory 4 (5) - imidazole, 4-hydroxy tamoxifen, trioxifene or toremifene. The anti-androgenic drugs are selected from flutamide, nilutamide, bicalutamide, leuprorelin, or goserelin. The immunosuppressant is conventional immunosuppressant known in the art, preferably selected from 2- amino -6-aryl-5-pyrimidine, azathioprine, cyclophosphamide, bromocriptine, danazol, dapsone, glutaraldehyde, anti-idiotype antibody against MHC antigens and MHC fragments, cyclosporin A, steroids such as glucocorticoids, streptokinases, TGFb, rapamycin, T cell receptors, T cell receptor fragments, cytokine receptor antagonists or T cell receptor antibodies. More preferably, the cytokine receptor antagonists are selected from anti-interferon antibodies, anti-IL10 antibodies, anti-TNFa antibodies or anti-IL2 antibodies;
wherein the anti-angiogenic agent is conventional anti-angiogenic agent known in the art, preferably selected from farnesyl transferase inhibitors, COX-2 inhibitors, VEGF inhibitors, bFGF inhibitors, steroid sulfate esterase inhibitors, interleukin-24, thrombus protein, metallospondin protein, type-I interferon, interleukin-12, and protamine, angiostatin, laminin, endostatin or prolactin fragment. More preferably 2-methoxy estradiol diamino sulfonate (2-MeOE2bisMATE);
wherein the anti-proliferation and pro-apoptosis agent is conventional anti-proliferation and pro-apoptosis agent known in the art, preferably selected from PPAR-γ activators, retinoids, triterpenes, EGF receptor inhibitors, telomere terminal transferase inhibitors, iron chelating agents, apoptotic proteins, Bcl-2 and Bcl-X (L) inhibitors, and TNF-α/FAS ligand/TNF-associated apoptosis-inducing ligand and its signal transduction activator or PI3K-Akt survival pathway signal inhibitors. The PPAR-γ activator is conventional PPAR-γ activator known in the art, preferably cyclopentenone prostaglandin (cyPGs). The triterpenes is conventional triterpenes known in the art, preferably selected from cycloartane, lupanes, ursane, oleanane, friedelane, damanane, cucurbitacin, limonoid analogues, or triterpenes compound. The EGF receptor inhibitor is conventional EGF receptor inhibitor known in the art, preferably selected from HER4, rapamycin or 1,25-dihydroxycholecalciferol (vitamin D). The iron chelate is conventional iron chelate known in the art, preferably 3-aminopyridine-2-carboxaldehyde-thiosemicarbazone. The apoptosis protein is conventional apoptosis protein known in the art, preferably the viral protein 3-VP3 of chicken anemia virus. The PI3K-Akt survival pathway signal inhibitor is conventional PI3K-Akt survival pathway signal inhibitor known in the art, preferably UCN-01 or geldanamycin;
wherein the cytolytic enzyme is conventional cytolytic enzyme known in the art, preferably RNA enzyme.

In the present invention, preferably, x=y=n in Formula 1; according to this,
in one preferred embodiment, - (L)ₓ-(D)_{y} is: 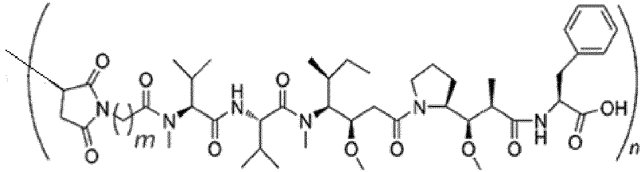 wherein m is 1-10, preferably is 5.

In one preferred embodiment, - (L)ₓ-(D)_{y} is:

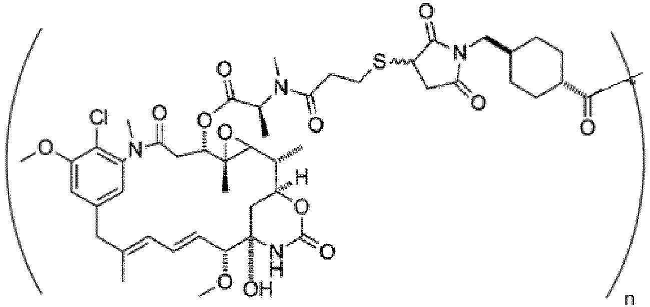

In one preferred embodiment, -(L)ₓ-(D)_{y} is:

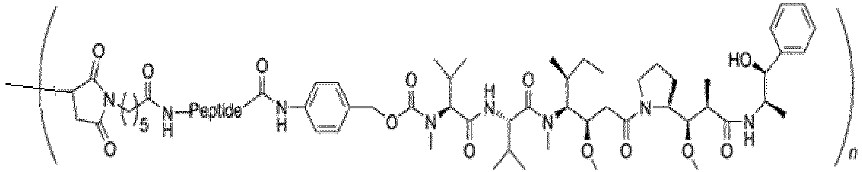

Most preferably, the D is a microtubulin synthetase inhibitor, monomethylauristatin F (MMAF), and the linker L is maleimidocaproyl (maleimidocaproyl, MC), and the structure of the immunoconjugate is shown in Formula 3, 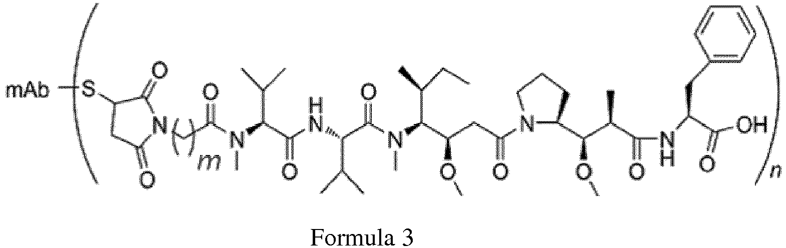 or, the L is N-succinimidyl 4-(maleimidomethyl) cyclo-hexanecarboxylate; D is N2'-Deacetyl-N2'-(3-mercapto-1-oxopropyl) maytansine (DM1), and the structure of the immunoconjugate is shown in Formula 4, 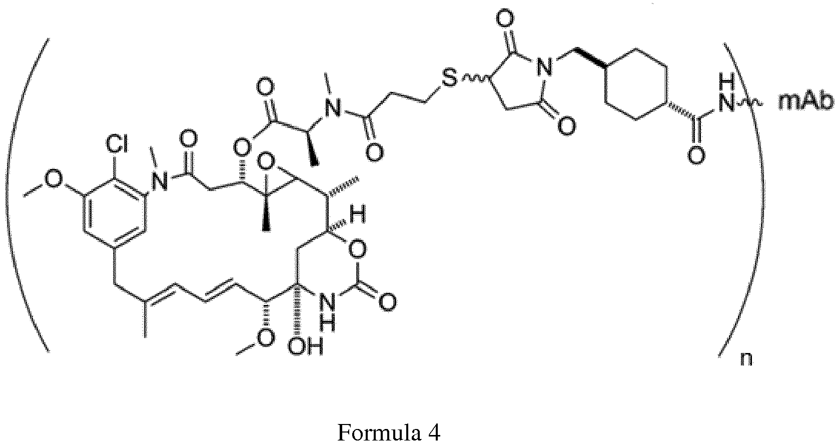 or, L is Maleimidocaproyl-L-valine-L-citrulline-p-aminobenzyl alcohol, and D is Monomethyl auristatin E (MMAE), and the structure of the immunoconjugate is shown in Formula 5, 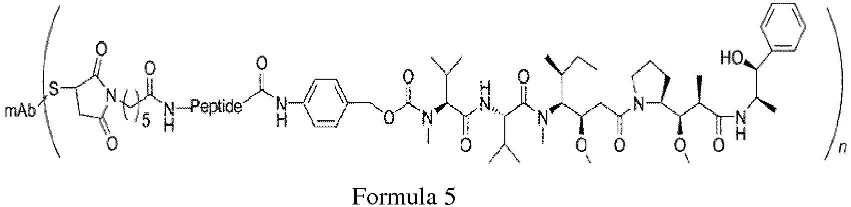 wherein n is a natural number, preferably an integer selected from 1-20, more preferably an integer selected from 1-2, or 2-4, or 4-8, or 8-20.

The preparation method of the immunoconjugate is conventional preparation method known in the art, preferably adopting the method described in Doronina, 2006, Bioconjugate Chem.17114-124. Preferably, the preparation method of which produces the immunoconjugate with the minimum low coupling fraction (LCF) of less than 10%.

More preferably, the preparation method comprises the following steps: dialyzing the protein with sodium borate buffer, pH 6.5-8.5, and adding with tris(2-carboxyethyl) phosphine (TCEP), wherein the molar ratio of TCEP to the above protein is 2-10. Reduction reacting for 1-4 hours at room temperature to obtain reaction solution A. Eluting the reaction solution A to remove excess above protein and obtain reaction solution B. Adding MC-MMAF to reaction solution B, wherein the molar ratio of MC-MMAF to the purified TPBG antibody was 5-20, and reacting for 4 hours at 10-37 °C.

The immunoconjugate can exist in any physical form known in the art, preferably is clear solution.

The present invention further provides a pharmaceutical composition comprising the above-mentioned immunoconjugate and pharmaceutically acceptable vehicle.

The pharmaceutically acceptable vehicle can be conventional vehicle known in the art. The vehicle can be any physiologically or pharmaceutically acceptable drug excipient. The drug excipient is conventional drug excipient known in the art, preferably comprising pharmaceutically acceptable excipients, fillers or diluents. More preferably, the pharmaceutical composition comprises 0.01-99.99% of the above-mentioned protein and 0.01-99.99% of pharmaceutical vehicle, wherein the percentage is the mass percentage of the pharmaceutical composition.

Preferably, the pharmaceutical composition is an anti-tumor drug, more preferably a drug against squamous/adenomatous lung cancer (non-small cell lung cancer), invasive breast cancer, colon cancer, rectal cancer, gastric cancer, squamous cervical cancer, invasive endometrial adenocarcinoma, invasive pancreatic cancer, ovarian cancer, squamous bladder cancer, choriocarcinoma, bronchial cancer, breast cancer, cervical cancer, pancreatic cancer or seminal vesicle cancer.

The pharmaceutical composition in the present invention is preferably parenteral administration, injection administration or oral administration. The injection administration preferably comprising intravenous injection, intramuscular injection, intraperitoneal injection, intradermal injection or subcutaneous injection, etc. The pharmaceutical composition has a variety of conventional dosage forms known in the art, preferably solid, semi-solid or liquid, i.e. aqueous solution, non-aqueous solution or suspension, more preferably tablets, capsules, granules, injection agents or infusion agents. More preferably administered intravascularly, subcutaneously, intraperitoneally or intramuscularly. Preferably, the pharmaceutical composition further can be administered as aerosol or rough spray, i.e. administered nasally; or administered intrathecally, intramedullarily or intraventricularly. More preferably, the pharmaceutical composition further can be administered transdermally, percutaneously, topically, enterally, intravaginally, sublingually or rectally.

The dosage of the pharmaceutical composition in the present invention can be adjusted according to the amount of composition that achieves the desired diagnostic or therapeutic results. Administration plan also can be single injection or multiple injections, or adjustment thereof. The chosen and appropriate adjustment of dosage and regimen depend on the factors comprising the activity and stability (i.e. half-life) of the pharmaceutical composition, preparation, routes of administration, combination with other drugs or therapies, diseases or conditions to be detected and/or treated, and the health condition and previous medical history of the recipients to be treated.

Therapeutically effective dose of the pharmaceutical composition of present invention can be preliminarily estimated in cell culture experiments or animal models such as rodents, rabbits, dogs, pigs and/or primates. Animal models can also be used to determine the appropriate concentration and route of administration, and the effective dose and route of administration of human being can be determined subsequently. Generally, the determination and adjustment of an effective amount or dose to be administered, and the evaluation of when and how such adjustment will be made are known to a person skilled in the art.

For combination therapy, the above-mentioned protein, immunoconjugate and/or additional therapeutic or diagnostic agents can be used separately as single drugs within any time frame appropriate for performing the anticipated therapy or diagnostics. Therefore, these single drugs can be administered simultaneously (i.e. as single preparation within minutes or hours) or sequentially. For instance, these single drugs can be administered within a year, or 10, 8, 6, 4 or 2 months, or within 4, 3, 2, or 1 week (s), or within 5, 4, 3, 2 or 1 day (s).

As for additional guidance on preparation, dosage, administration regimen and measurable therapeutic results, please refer to Berkow et al. (2000) The Merck Manual of Medical Information and Merck&Co.Inc., Whitehouse Station, New Jersey; Ebadi (1998) CRC Desk Reference of Clinical Pharmacology and etc.

The present invention provides a use of above-mentioned protein in preparing anti-tumor drugs.

The present invention provides a use of above-mentioned immunoconjugate in preparing anti-tumor drugs.

The present invention provides a use of above-mentioned pharmaceutical composition in preparing anti-tumor drugs.

The present invention provides a use of above-mentioned protein in treating tumors.

The present invention provides a use of above-mentioned immunoconjugate in treating tumors.

The present invention provides a use of above-mentioned pharmaceutical composition in treating tumors.

The present invention further provides a method for detecting cells overexpressing TPBG protein, which comprises the following steps: contacting the above-mentioned protein with the sample to be tested *in vitro,* and detecting the binding of the above-mentioned protein with the sample to be tested.

The meaning of overexpression is conventional meaning of overexpression known in the art, preferably the mean fluorescence intensity (MFI) of the above-mentioned protein is three times or more than the MFI of IgG subtype when subjecting the cells in the sample to be tested to FACS.

The combined detection method is conventional detection method known in the art, preferably FACS detection method.

The "TPBG-positive" cells in the present invention are cells that overexpress TPBG protein, e.g. the NCI-H1568 cell strain; otherwise, they are designated as "TPBG-negative" cells, e.g. tumor cell line NCI-H1770.

On the basis of common knowledge in the art, the above preferred conditions can be arbitrarily combined to obtain the preferred embodiments of the present invention.

All the materials and agents are commercially available.

The positive and progressive effect of the present invention is that the TPBG antibody in the present invention is a chimeric antibody which has high affinity with the TPBG protein and is capable of binding the extracellular domain of TPBG protein receptor in both protein and cellular levels. A conjugate is formed when the TPBG protein is coupled with a small-molecule compound such as MC-MMAF, and the conjugate can perform effective cytotoxic killing on TPBG-positive cells. Besides, TPBG antibody can bring small-molecule compounds, such as MMAF, into the cell via endocytosis and degrade to release the small-molecule compounds in cells. Therefore, the antibody cross-linked drug prepared by the TPBG antibody can effectively kill tumor cells and treat tumors.

### Description of the Drawings

Fig.1 shows FACS screening results of of HEK293 cells transfected with human TPBG protein.
Fig.2 shows FACS screening results of CHOK1 cells transfected with human TPBG protein.
Fig.3 shows FACS screening results of CHOK1 cells transfected with cynomolgus monkey TPBG protein.
Fig.4 shows FACS screening results of CHOK1 cells transfected with mouse TPBG protein.
Fig.5 shows the titer of serum antibody in mice immunized with TPBG by ELISA.
Fig.6A and Fig.6B show the binding reaction of TPBG antibody to human TPBG-hFc protein by ELISA.
Fig.7A and Fig.7B show the binding reaction of the TPBG antibody to CHOk1-hTPBG by FACS.
Fig.8A and Fig.8B show the binding reaction of the TPBG antibody to CHOk1-cTPBG by FACS.
Fig.9A and Fig.9B show the binding of TPBG antibody to CHOk1-mTPBG by FACS.
Fig.10A and Fig.10B show the binding reaction of the TPBG antibody to CHOk1 by FACS.
Fig.11A and Fig.11B show the cytotoxicity effects of TPBG antibody-MMAF antibody-drug conjugate on the TPBG expression-positive non-small cell lung cancer cell strain NCI-H1568.
Fig.11C shows the cytotoxicity effects of TPBG antibody on TPBG expression-positive non-small cell lung cancer cell strain NCI-H1568.
Fig.12A and Fig.12B show the cytotoxicity effects of TPBG antibody-MMAF antibody-drug conjugate n the TPBG expression-negative non-small cell lung cancer cell strain NCI-H1770.
Fig.13 shows the cytotoxicity effects of TPBG chimeric antibody-drug conjugate on the TPBG-positive tumor cell line NCI-H1299.
Fig.14A shows tumor volume change after the treatment of antibody-drug conjugate of the TPBG chimeric antibody 12B12 and its naked antibody.
Fig.14B shows tumor volume change after the treatment of antibody-drug conjugate of the TPBG chimeric antibody 5G4 and its naked antibody.
Fig.14C shows tumor volume change after the treatment of antibody-drug conjugate of the TPBG chimeric antibody 39A11 and its naked antibody.
Fig.14D shows tumor volume change after the treatment of antibody-drug conjugate of the TPBG chimeric antibody 28D4 and its naked antibody.
Fig.14E shows tumor volume change after the treatment of antibody-drug conjugate of the TPBG chimeric antibody 36A10 and its naked antibody.
Fig.15A shows bodyweight change of mice after the treatment of antibody-drug conjugate of the TPBG chimeric antibody 12B12 and its naked antibody.
Fig.15B shows bodyweight change of mice after the treatment of antibody-drug conjugate of the TPBG chimeric antibody 5G4 and its naked antibody.
Fig.15C shows bodyweight change of mice after the treatment of antibody-drug conjugate of the TPBG chimeric antibody 39A11 and its naked antibody.
Fig.15D shows bodyweight change of mice after the treatment of antibody-drug conjugate of the TPBG chimeric antibody 28D4 and its naked antibody.
Fig.15E shows bodyweight change of mice after the treatment of antibody-drug conjugate of the TPBG chimeric antibody 36A10 and its naked antibody.
Fig.16A and Fig.16B show cytotoxicity effects of the TPBG chimeric antibody 12B12 and its small-molecule antibody-drug conjugates coupled with various small-molecule drugs on the TPBG-positive tumor cell line NCI-H1299, respectively.
Fig.16C and Fig.16D show cytotoxicity effects of the TPBG chimeric antibody 12B12 and its small-molecule antibody-drug conjugates coupled with various small-molecule drugs on the TPBG-positive tumor cell line NCI-H1568, respectively.
Fig.17A and Fig.17B show cytotoxicity effects of TPBG chimeric antibody-drug conjugate on the TPBG expression-positive recombinant cell strain 293-hTPBG.
Fig.17C and Fig.17D show cytotoxicity effects of TPBG chimeric antibody-drug conjugate on 293 cell strain without expressing TPBG.

### Embodiments

The present invention will be further illustrated by the following examples, but is not therefore limited to the scope of the described embodiments. The experimental methods that do not specify the specific conditions in the following embodiments are conventional methods and conditions, or selected according to the instruction of the products.

The room temperatures described in the examples are conventional room temperatures known in the art, which is generally 10-30°C.

Unless otherwise specified, PBS described in the examples is PBS phosphate buffer, pH7.2.

### Embodiment 1 Preparation of TPBG antibody

The nucleotide sequence containing amino acid sequence 32-355 (Ser32-Ser355) encoding the extracellular domain of human TPBG protein (wherein the accession number of the nucleotide sequence encoding human TPBG protein is Genbank ID: AAH37161.1) was cloned into the pCpC vector including human IgG Fc fragment (purchased from Invitrogen, V044-50) and the plasmid was prepared according to established standard molecular biology protocol. For specific methods, please refer to Sambrook, J., Fritsch, E. F., and Maniatis, T. (1989). Molecular Cloning: A Laboratory Manual, Second Edition (Plainview, New York: Cold Spring Harbor Laboratory Press). HEK293 cells (purchased from Invitrogen) were transiently transfected (polyether imide, PEI, purchased from Polysciences) and expanded using FreeStyle ™293 (purchased from Invitrogen) at 37°C. After 4 days, cell culture was harvested, cell pellets were removed by centrifugation and culture supernatant containing the extracellular domain of TPBG protein was obtained. The culture supernatant was loaded onto protein A affinity chromatography column (Mabselect Sure, purchased from GE Healthcare), and the change in ultraviolet absorption was monitored using a UV detector. Once the sample was loaded, the protein A affinity chromatography column was equilibrated with PBS buffer (pH7.2) until the ultraviolet absorption value returned to baseline, and then elution was performed using 0.1M glycine hydrochloride acid (pH2.5), and hFc tagged TPBG protein (i.e. human TPBG-hFc) eluting from the protein A affinity chromatography column was harvested and dialyzed against PBS (pH 7.2) at 4°C overnight in refrigerator. The dialyzed protein was aseptically filtered through 0.22µm and stored at -80°C in aliquots, and purified immunogen A was then obtained.

Prior to being used, Immunogen A was subjected to a series of quality control tests, e.g. protein concentration, purity, molecular weight, biological activity, etc., and it was found that the immunogen A was qualified and can be used as the antigen for the subsequent preparation of TPBG antibodies.

### (2) Preparation of Immunogen B

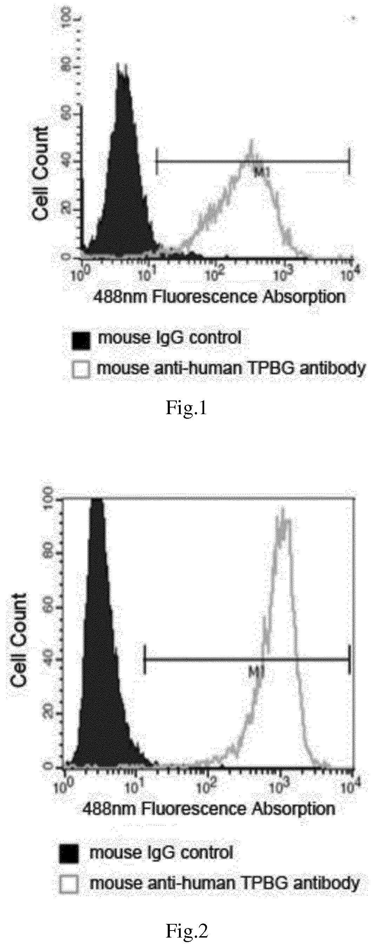

The nucleotide sequence encoding the full-length amino acid sequence of human TPBG (wherein the Genbank accession number of the nucleotide sequence encoding human TPBG protein is Genbank ID: AAH37161.1) was cloned into the pIRES vector (purchased from Clontech) and the plasmid was prepared. Recombinant plasmid (PEI, purchased from Polysciences) was transfected to HEK293 cell line and CHOK1 cell line (both purchased from Invitrogen), and the transfected cells were selectively cultured in 0.5µg/ml of DMEM culture medium containing 10% (w/w) fetal bovine serum for two weeks. Subcloning was performed using limiting dilution assay in 96-well plates and the plates were cultured at 37°C, 5% (v/v) CO₂, and after two weeks a portion of the wells containing monoclones were selected and expanded into 6-well plates. The expanded clones were screened by FACS analysis using commercial available TPBG antibody (purchased from Sigma, Lot NO: SAB1404485). Robust monoclonal cell lines with high fluorescence intensity were selected and further expanded and cryopreserved in liquid nitrogen to obtain immunogen B. The specific selection results were shown in Table 3 and Fig.1. The IgG subclass control herein was mouse IgG. Table3 indicates that a series of TPBG expression-positive HEK 293 cell lines have been prepared. In Fig.1 the abscissa indicates the fluorescence intensity of cells, and the ordinate indicates cell count. Fig.1 demonstrates that 5E5E9 is a cell strain with high level of TPBG expression, wherein the MFI of the cells labeled with anti-TPBG antibody is 216, and the migration rate of the cells is 98.5%.

**Table 3 FACS screening results of HEK293 cells transfected with human TPBG**

| NO. | Clone number of transfected cell | Cellular MFI | |
|---|---|---|---|
| | | IgG isotype control | TPBG antibody |
| 1 | 293F-hTPBG 4E1 | 5.2 | 145.0 |
| 2 | 293F-hTPBG 4A8 | 3.1 | 33.4 |
| 3 | 293F-hTPBG 4A9 | 6.3 | 203.9 |
| 5 | 293F-hTPBG 4B9 | 6.3 | 126.1 |
| 6 | 293F-hTPBG 4C3 | 3.2 | 27.2 |
| 7 | 293F-hTPBG 4C5 | 5.6 | 171.5 |
| 8 | 293F-hTPBG 4E1 | 4.8 | 91.8 |
| 9 | 293F-hTPBG 4F9 | 3.9 | 47.7 |
| 10 | 293F-hTPBG 4G1 | 4.0 | 131.2 |
| 11 | 293F-hTPBG 4G6 | 3.1 | 31.8 |
| 12 | 293F-hTPBG 4H12 | 4.0 | 9.5 |
| 13 | 293F-hTPBG 5E6 | 2.6 | 13.2 |
| 15 | 293F-hTPBG 5A11 | 4.6 | 166.5 |
| 16 | 293F-hTPBG 5A9 | 5.0 | 53.2 |
| 17 | 293F-hTPBG 5C12 | 3.7 | 75.4 |
| 18 | 293F-hTPBG 5D4 | 3.0 | 70.6 |
| 19 | 293F-hTPBG 5E5 | 5.6 | 280.5 |
| 20 | 293F-hTPBG 5G11 | 4.1 | 11.3 |
| 21 | 293F-hTPBG 5G8 | 6.0 | 167.8 |
| 22 | 293F-hTPBG 5H6 | 3.0 | 36.4 |

### (3) Preparation of hybridoma cells and screening of antibody

### A. Immunization with immunogen A

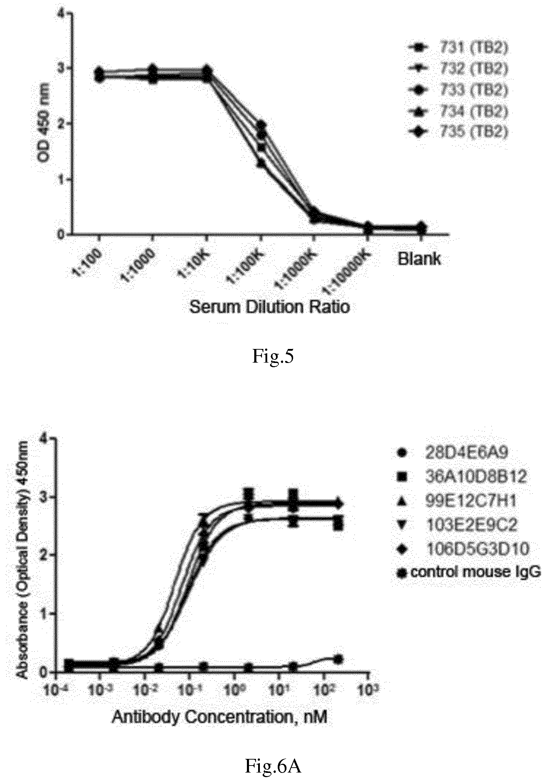

BALB/cAnNCrl mice or SJL/JorllcoCrl mice (both purchased from SLAC Co.Ltd, Shanghai) aged 6-8 weeks were used and raised in SPF condition. In the primary immunization, 0.25ml of immunogen A being emulsified with Freund's complete adjuvant, i.e. 50mg of immunogen A per mouse was intraperitoneally injected. In booster immunization, 0.25ml of immunogen A being emulsified with Freund's incomplete adjuvant, i.e. 50mg of immunogen A per mouse was intraperitoneally injected. The interval between the primary immunization and first booster is two weeks and the interval between each booster is three weeks. Blood was collected 1 week after each booster, and the antibody titer and specificity of the immunogen in serum were tested by ELISA and FACS. The results were shown in Fig.5 and Table 4. Table 4 indicates that the serums of mouse immunized with immunogen A have different degrees of binding to immunogen A and show antigen-antibody reaction, wherein the highest dilution rate is around one million. The blank control is 1% (w/w) BSA, and batch refers to the mouse serum at day 7 after the second booster immunization. The data in the table refers to OD450nm values.

**Table 4 Detection of antibody titers in the serum of Balb/c mice immunized with TPBG protein using ELISA**

| OD₄₅₀ₙₘ | Serum Dilution Rate | | | | | | |
|---|---|---|---|---|---|---|---|
| Batch | 1:100 | 1:10³ | 1:10⁴ | 1:10⁵ | 1:10⁶ | 1:10⁷ | Blank |
| 731(TB2) | 2.8722 | 2.8084 | 2.8186 | 1.5772 | 0.3892 | 0.1201 | 0.0935 |
| 732(TB2) | 2.8715 | 2.8171 | 2.857 | 1.2767 | 0.2601 | 0.1353 | 0.0985 |
| 733(TB2) | 2.8411 | 2.8841 | 2.9258 | 1.7943 | 0.3336 | 0.1178 | 0.1418 |
| 734(TB2) | 2.8735 | 2.8503 | 2.861 | 1.3150 | 0.3052 | 0.1129 | 0.1365 |
| 735(TB2) | 2.9460 | 2.9859 | 2.9761 | 1.9749 | 0.4203 | 0.1463 | 0.1531 |

### B. Immunization with immunogen B

BALB/cAnNCrl mice or SJL/JorllcoCrl mice (both purchased from SLAC Co.Ltd, Shanghai) at 6-8 weeks of age were used and feed under SPF condition. The pIRES plasmid containing the nucleotide sequence encoding full-length human TPBG (please refer to step 2, Example 1) was transfected into HEK293 cell line and the recombinant HEK293 cell line containing human TPBG was obtained (transfection was performed using X-treme GENE HP DNA Transfection Reagent, purchased from Roche Co. Ltd, and operated according to the manual). Culture was expanded in T-75 flasks to a confluency of 90%, the culture medium was removed via pipetting, and cells were washed twice with DMEM basic medium (purchased from Invitrogen). Then cells were treated using enzyme-free cell dissociation buffer (purchased from Invitrogen) at 37°C until the cells can be peeled off from the dish wall, and then harvested. The cells were washed twice with DMEM basic medium and diluted to a concentration of 2×10⁷ cell/mL using phosphate buffer after cell counting. Every time, 0.5ml of cell suspension was injected intraperitoneally into each mouse, and there was a two-week interval between the primary and booster immunizations. After that booster immunization was performed every three weeks. Blood sample was taken one week after every immunization except for the primary immunization, and the titers and specificity of the antibody in the serum was detected using FACS. After the second booster immunization, the antibody titer in the serum reached to a value higher than 1:1000.

Prior to the completion of step A and B, each selected mice was injected intraperitoneally with 100µg of purified immunogen A (for mice immunized with immunogen A) or recombinant HEK293 cell line containing human TPBG (for mice immunized with immunogen B) for the last immunization, and sacrificed 5 days later. Their spleen cells were harvested, and NH₄OH was added to a final concentration of 1% (w/w) to lyze the red blood cells in the spleen cells, follow by preparing a splenocyte suspension. Cells were washed for three times by centrifugation at 1000rpm using DMEM basic medium, and the viable cells were mixed with mouse myeloma cell SP2/0 (purchased from ATCC) at a ratio of 5:1. Cell fusion was performed using a high efficient electrofusion method (please refer to METHODS IN ENZYMOLOGY, VOL. 220). Fused cells were diluted in DMEM medium containing 20% (w/w) FBS and 1×HAT, and then plated onto 96-well cell culture plates at the density of 1×10⁵ cells/200ml per well and cultured in 5%(v/v) CO₂ incubator at 37°C. After 14 days, the supernatants from the cell fusion plates were screened using ELISA and Acumen (microplate cytometry), and positive clones with an ELISA OD₄₅₀ₙₘ>1.0 and Acumen MFI value >100 were subjected to expansion culture in DMEM culture medium containing 10% (w/w) HT FBS in 24-wells plate at 37°C in 5%(v/v) CO₂. The culture medium from the expansion culture in 24-wells plate was centrifuged after 3 days of culture and the supernatant was collected and subjected to antibody subclass analysis. Its binding activity to TPBG protein and TPBG-positive cells (for the method of measuring binding activity, refers to the related information in Example 3A and 3B) and mouse TPBG antibody-MMAF indirect cytotoxicity assay (for the detection method for indirect cytotoxic killing assay, refers to the related information in Example 4) were determined using ELISA and FACS.

Based on the screening results of 24-wells plate, hybridoma cells having ELISA OD₄₅₀ₙₘ>1.0, FACS MFI>50 and the hybridoma supernatant killing rate on TPBG-positive cells reaching up to 50% in indirect cytotoxicity assay were selected as eligible positive clones. Selected eligible hybridoma cells were subjected to subcloning by limiting dilution assay in 96-well plates and cultured in DMEM medium (purchased from Invitrogen) containing 10% (w/w) HT FBS at 37°C in 5% (v/v) CO₂. 10 days after the subcloning, initial screening was performed using ELISA and Acumen, and single positive monclones were selected and subjected to expansion culture in 24-wells plate. After 3 days, positive antigen binding was confirmed by FACS and mouse antibody-MMAF indirect cytotoxicity assay was performed to evaluate biologic activity, while the criteria for the evaluation is that OD₄₅₀ₙₘ>1.0 for ELISA, MFI>50 for FACS and the hybridoma supernatant killing rate on TPBG-positive cells shall reach up to 50% in indirect cytotoxicity assay.

According to the detection results of 24-wells plate, the optimal clones were picked and expanded in DMEM medium (purchased from Invitrogen) containing 10% (w/w) HT FBS at 37°C in 5%(v/v) CO₂. The optimal clones were obtained and stored in liquid nitrogen, and then used for subsequently production and purification of antibody.

### Embodiment 2 Production and Purification of lead antibody

The antibody concentration generated by hybridoma cells was relatively low, about only 1-10mg/mL with a broad-range variation, and the various proteins derived from the culture medium and the contents of FBS contained in the culture medium were interfered by many biologic activity assays to different degrees. Therefore a small-scale (1-5mg) production and purification of antibody is necessary.

The hybridoma cells obtained by Embodiment 1 were seeded into T-75 cell culture flask and passaged for three times. Until reaching a robust growing state, cells were seeded into the cell culture spinner flasks. 200mL of production culture medium were added to each 2L spinner flask with a 1.0×10⁵/mL of seeding density. The flask lid was tightened and the spinner flask was placed on the rotary machine in a 37°C incubator with a 3 rpm of rotation speed. The cell culture was collected after continuous spinning culture for 14 days and cells were removed by filtration through a 0.45 µm filter until the culture supernatant was clear and can be subjected to immediate purification or stored at -30°C.

The TPBG antibody in the obtained culture supernatant (200mL) was purified using 2mL of protein A column (purchased from GE Healthcare). The protein G column was first equilibrated with equilibration buffer (PBS buffer, pH7.4), and the culture supernatant was then applied to the protein A column at a controlled flow rate of 3mL/min. The equilibration buffer was used to wash the protein G column once sample loading was complete, and the volume of the equilibration buffer used herein was four times as much as the bed volume of protein A column. The TPBG antibody bound to protein A column was eluted using elution buffer (0.1M sodium citrate buffer, pH3.5), and the elution was monitored by UV detector (A280nm absorption). The antibody eluate was collected and pH was neutralized by adding with 10% (v/v) 1.0M Tris-HCl buffer, then the eluate was immediately subjected to dialysis against PBS buffer overnight, and the buffer was changed once the next day and the dialysis was continued for 3h. The dialyzed TPBG antibody was harvested, filtered by 0.22µm sterile filter, and then stored in sterile condition, therefore a purified TPBG antibody was obtained.

The purified TPBG antibody was tested for its protein concentration, purity, endotoxin (Lonza kit) and the like. Results shown in table 5 indicates that the concentration of endotoxin in the final antibody product is below 1.0 EU/mg.

**Table 5 Analysis of purified TPBG antibody**

| Clone Number | Antibody Purity | Protein Concentration (mg/mL) | Endotoxin (EU/mg) |
|---|---|---|---|
| 12B12C7C3 | > 90% | 0.82 | <0.12 |
| 5G4H10G5 | > 90% | 0.68 | <0.12 |
| 37H9C5G2 | > 90% | 0.65 | <0.12 |
| 39A11G5F2 | > 90% | 0.85 | <0.12 |
| 52C9E9F6 | > 90% | 1.2 | <0.12 |
| 28D4E6A9 | > 90% | 1.02 | <0.12 |
| 36A10D8B12 | > 90% | 0.28 | <0.12 |
| 99E12C7H1 | > 90% | 1.12 | <0.12 |
| 103E2E9C2 | > 90% | 0.73 | <0.12 |
| 106D5G3D10 | > 90% | 0.54 | <0.12 |

### Embodiment 3 Identification of lead antibody

### A. Detection of TPBG antibody binding to TPBG protein by enzyme-linked immunosorbent assay (ELISA)

The purified TPBG antibody obtained from Embodiment 2 reacted with human TPBG-hFc.

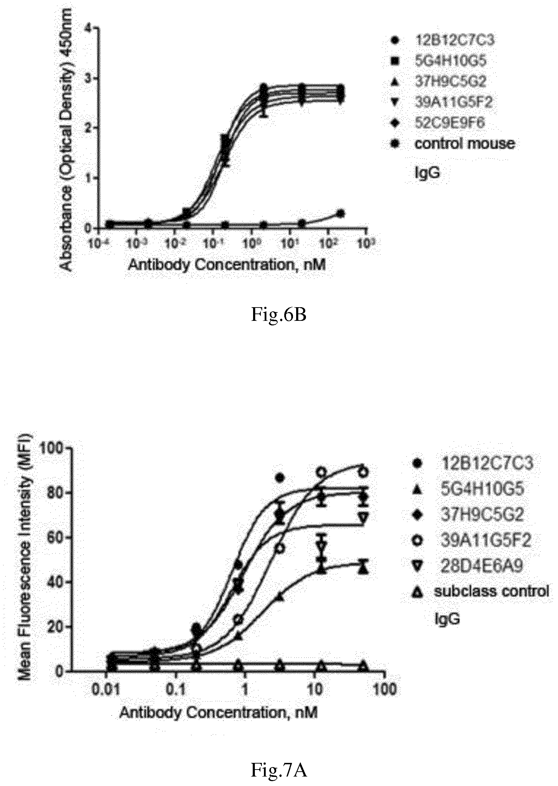

The purified immunogen A (as for its method of preparation please refer to step 1 in Example 1) diluting with PBS to a final concentration of 1.0µg/mL was aliquoted into a 96-well ELISA plate at 100µL per well, and sealed with a parafilm and incubated at 4°C overnight. The plate was washed twice with plate washing buffer (PBS containing 0.01% (v/v) Tween 20), and blocked with blocking buffer (PBS containing 0.01% (v/v) Tween 20 and 1% (w/w) BSA) at room temperature for 2h. The blocking buffer was discarded, and 100 µL per well of the purified antibody obtained from Embodiment 2 was added. After incubating at 37°C for 2h, the plate was washed for three times with plate washing buffer (PBS containing 0.01% (v/v) Tween 20). HRP (horseradish peroxidase)-labeled secondary antibody (purchased from Sigma) was added and incubated at 37°C for 2h, and the plate was washed for three times using plate washing buffer (PBS containing 0.01% (v/v) Tween 20). 100µL of TMB substrate was aliquoted into each well, incubated at room temperature for 30min, and 100 µL of stop buffer (1.0N HCl) per well was subsequently added. A₄₅₀ₙₘ values were read using the ELISA plate reader (SpectraMax 384plus purchased from Molecular Device), and the results were shown in Fig.6 and table 6. Table 6 indicates that purified TPBG antibody can combine with recombinant TPBG protein at the level of ELISA. In Table 6 the IgG control herein is a mice IgG control, the data in the Table are OD₄₅₀ₙₘ values, and Blank means the OD₄₅₀ₙₘ value when there is only PBS buffer in the plate.

**Table 6 ELISA detection of the binding reaction between the TPBG antibody and human TPBG-hFc protein**

| OD₄₅₀ₙₘ | Antibody Concentration(nM) | | | | | | | |
|---|---|---|---|---|---|---|---|---|
| Clone Number | 200 | 20 | 2 | 0.2 | 0.02 | 0.002 | 0.0002 | Blank |
| 12B12C7C3 | 2.82 | 2.83 | 2.84 | 1.67 | 0.29 | 0.13 | 0.10 | 0.11 |
| 5G4H10G5 | 2.72 | 2.80 | 2.68 | 1.76 | 0.34 | 0.10 | 0.08 | 0.07 |
| 37H9C5G2 | 2.61 | 2.63 | 2.52 | 1.55 | 0.35 | 0.11 | 0.09 | 0.09 |
| 39A11G5F2 | 2.55 | 2.50 | 2.43 | 1.36 | 0.30 | 0.11 | 0.09 | 0.09 |
| 52C9E9F6 | 2.65 | 2.69 | 2.68 | 1.39 | 0.27 | 0.12 | 0.09 | 0.08 |
| 28D4E6A9 | 2.60 | 2.59 | 2.64 | 1.97 | 0.46 | 0.14 | 0.10 | 0.09 |
| 36A10D8B12 | 2.51 | 3.07 | 3.06 | 2.15 | 0.51 | 0.18 | 0.16 | 0.19 |
| 99E12C7H1 | 2.93 | 2.88 | 2.93 | 2.62 | 0.78 | 0.21 | 0.11 | 0.08 |
| 103E2E9C2 | 2.60 | 2.62 | 2.64 | 1.89 | 0.48 | 0.14 | 0.10 | 0.13 |
| 106D5G3D10 | 2.88 | 2.82 | 2.81 | 2.36 | 0.56 | 0.16 | 0.11 | 0.10 |
| IgG control | 0.24 | 0.11 | 0.10 | 0.11 | 0.10 | 0.10 | 0.11 | 0.11 |

### B. The binding of TPBG antibody to TPBG-expressing cells was detected by fluorescence activated cell sorting (FACS)

pIRES plasmid containing the nucleotide sequence encoding human TPBG full-length amino acid sequence in step 2 of Embodiment 1 was transfected into CHOK1 cell line, and a recombinant CHOK1 cell line containing human TPBG (herein referred to as CHOK1-hTPBG stable cell line) was obtained. Similarly, pIRES plasmid containing cynomolgus monkey full-length TPBG gene (the preparation method of which is the same as that of the pCpC vector containing human IgG Fc fragment in the "Preparation of Immunogen A" part of step 1 in Embodiment 1, the full-length amino acid sequence of cynomolgus monkey with Genbank ID: BAE00432.1 was transfected into CHOK1 cell line, and CHOK1 stable cell line containing cynomolgus monkey TPBG (herein referred to as CHOk1-cTPBG stable cell line) was obtained. pIRES plasmid containing full-length mouse TPBG gene (the method of preparation of which is the same as that of the pCpC vector containing human IgG Fc fragment in the "Preparation of Immunogen A" part of step 1 in Example 1, the Genbank ID of mouse TPBG full-length gene sequence can be accessed by: CAA09931.1) was transfected into CHOK1 cell line, and a recombinant CHOK1 cell line containing mouse TPBG (herein referred to as CHOK1-mTPBG stable cell line) was prepared.

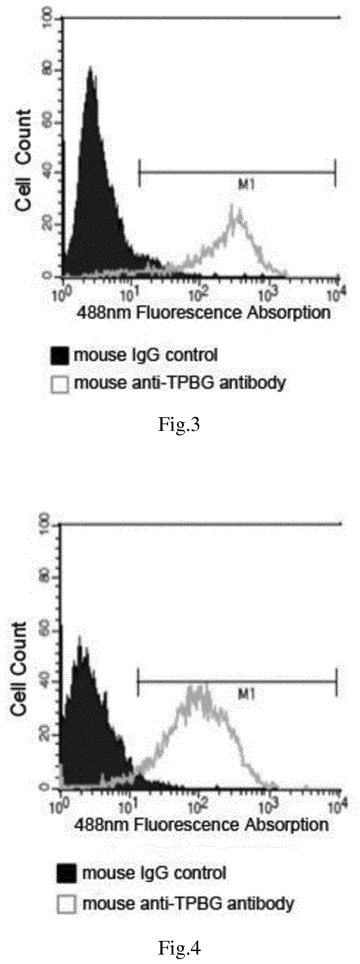

The titers and specificity of the TPBG antibody in serum were measured by FACS. For the detection method, please refer to the method identifying the stable cell line HEK293-hTPBG in the "Preparation of Immunogen B", step 2 in Embodiment 1. Results were shown in Table 7 and Figs. 2-4. In Figs.2-4 the abscissa indicates the fluorescence intensity of the cells, and the ordinate stands for the number of cells. Among them, CHOK1-hTPBG 3A2F1 is a human TPBG-expressing cell line used for screening, and the FACS screening and detection results are shown in Fig.2. CHOK1-cTPBG 3F13G4 is a cynomolgus monkey TPBG-expressing cell line used for screening, and the FACS screening result is shown in Fig.3. CHOK1-mTPBG 3A3 herein is mouse TPBG-expressing cell line used for screening, and the FACS screening result is shown in Fig.4. Results in table 7 indicate that human, monkey or mouse TPBG protein are overexpressed on the cell membrane of the stable cell lines CHOk1-hTPBG, CHOk1-cTPBG and CHOk1-mTPBG, respectively, which can be used for the screening of TPBG antibody.

**Table 7 Results of FACS screening and detection of human/monkey/mouse TPBG-transfected CHOK1 cells**

| Transfected Cell Clone Number | Cellular MFI | |
|---|---|---|
| | IgG Control | Anti-TPBG antibody |
| CHOk1-hTPBG 3A2F1 | 3.24 | 798.37 |
| CHOk1-cTPBG 3F13G4 | 2.66 | 198.35 |
| CHOk1-mTPBG 3A3 | 2.38 | 135.25 |

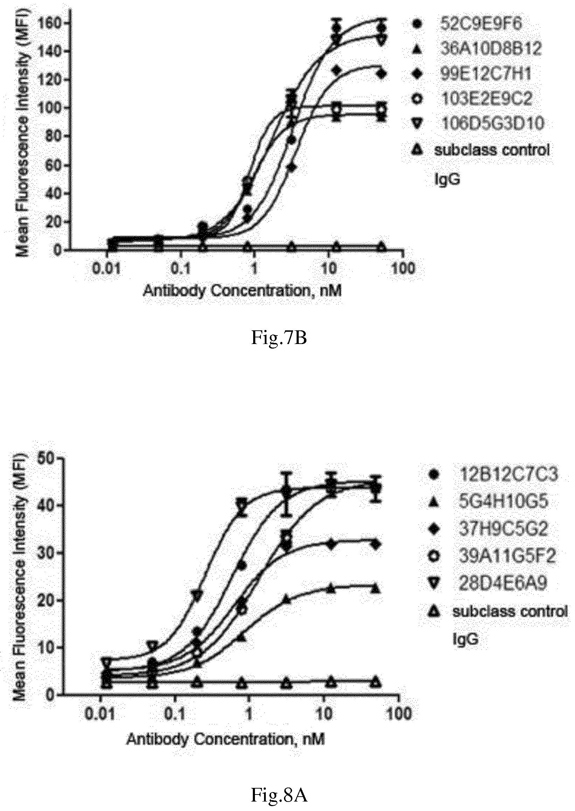

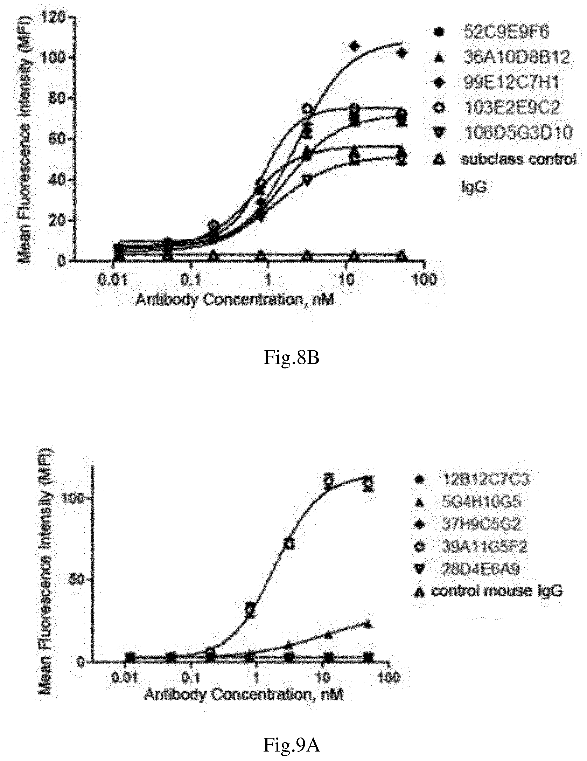

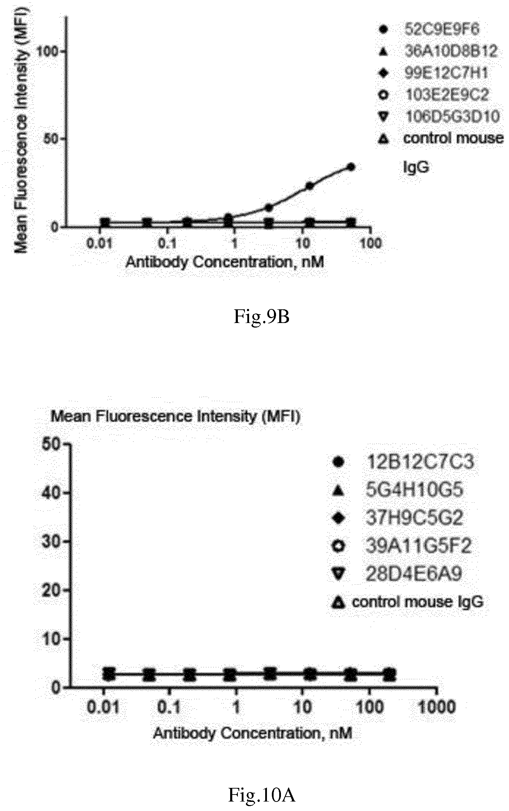

The stable cell lines CHOk1-hTPBG, CHOk1-cTPBG, CHOk1-mTPBG (i.e. the CHOk1-hTPBG 3A2F1, CHOk1-cTPBG 3F13G4 and CHOk1-mTPBG 3A3 shown in table 7) and CHOK1 cells were expanded to a 90% of confluency in T-75 cell culture flasks, the culture medium were removed, and cells were then rinsed twice with HBSS buffer (Hanks Balanced Salt Solution, purchased from Invitrogen) and lysed with enzyme-free cell dissociation buffer (Versene solution purchased from Life technology) and harvested. Harvested cells were rinsed with HBSS buffer twice and counted before diluting with HBSS buffer to 2×10⁶ cells /mL, and 10% goat serum blocking buffer was then added, wherein the percentage is the mass percentage. Cells were incubated on ice for 30min, and then washed twice with HBSS buffer by centrifugation. The harvested cells were resuspended with FACS buffer (HBSS+1%BSA, wherein the percentage is mass percentage) to a concentration of 2 ×10⁶ cells /mL. 100µL per well of the cells was aliquoted to 96-well FACS reaction plates, and 100µL per well of test sample-the purified TPBG antibody obtaining from Embodiment 2 was added before incubating on ice for 2h. The plate was washed with the FACS buffer twice by centrifugation, and 100 µL per well of fluorescence (Alexa 488) labeled secondary antibody (purchased from Invitrogen) was then added and incubated on ice for 1h. The plate was washed with the FACS buffer for three times by centrifugation, follow by resuspending in 100µL of fixation buffer (4% (v/v) paraformaldehyde). Cells were washed with FACS buffer twice after 10min, follow by resuspending with 100 µL of FACS buffer. The results were measured and analyzed by FACS (FACS Calibur, purchased from BD). Data analysis was performed by software (CellQuest) to obtain mean fluorescence intensity (MFI) of the cells, follow by calculating EC50 value by software (GraphPad Prism) analysis and data fitting. The analysis results were shown in Table 8 and Figs.7-10, and data in Figs.7-10 show the mean fluorescence intensity (MFI) of cells. Data in Table 8 is the EC50 values calculated from the MFI, and Table 8 indicates that TPBG antibody is able to bind cell-surface TPBG proteins.

**Table 8 FACS Analysis of Binding Activity of TPBG Antibody to Human/Monkey/Mouse TPBG-expressing Cell Lines**

| Clone Number | EC50(nM) | | | |
|---|---|---|---|---|
| | CHOK1-hTPB G | CHOK1-cTPB G | CHOK1-mTP BG | CHOK1 |
| 12B12C7C3 | 0.65 | 0.59 | Negative | Negative |
| 5G4H10G5 | 1.75 | 0.84 | 9.07 | Negative |
| 37H9C5G2 | 0.94 | 0.62 | 11.05 | Negative |
| 39A11G5F2 | 2.34 | 1.35 | 1.88 | Negative |
| 52C9E9F6 | 3.27 | 1.54 | Negative | Negative |
| 28D4E6A9 | 0.63 | 0.25 | Negative | Negative |
| 36A10D8B12 | 0.94 | 0.60 | Negative | Negative |
| 99E12C7H1 | 3.54 | 2.29 | Negative | Negative |
| 103E2E9C2 | 0.85 | 0.83 | Negative | Negative |
| 106D5G3D10 | 1.72 | 1.26 | Negative | Negative |

### Embodiment 4 Cell Cytotoxicity Activity Assay of TPBG Antibody-Drug Conjugate

After dialyzing the purified TPBG antibody obtained from Embodiment 2 in sodium borate buffer, pH 6.5-8.5, Tris (2-carboxyethyl) phosphine (TCEP) was then added, wherein the molar ratio of TCEP to the purified TPBG antibody was 2. Reaction solution A was obtained after 1h of reductive reaction at room temperature. The excess TCEP in reaction solution A was removed by G25 desalting column (purchased from GE), and reaction solution B was obtained. MC-MMAF (purchased from Levena Biopharma, Nanjing) was added to reaction solution B, wherein the molar ratio of MC-MMAF to purified TPBG antibody was 5 and the reaction was carried out at RT for 4h. Cysteine was further added to neutralize excess MC-MMAF and excess small molecules were removed by G25 desalting column to obtain purified TPBG antibody-drug conjugate (for the method of conjugation, please refer to Doronina, 2006, Bioconjugate Chem.17,114-124). Parameters of the drug, such as drug antibody ratio, purity etc. were determined by HIC prior to analyzing cytotoxicity effect of cells. The drug antibody ratio (DAR) of all antibody-drug conjugate is 8, wherein DAR (drug antibody ratio) refers to the average number of small-molecule drugs carried by a single antibody molecule after antibody conjugation.

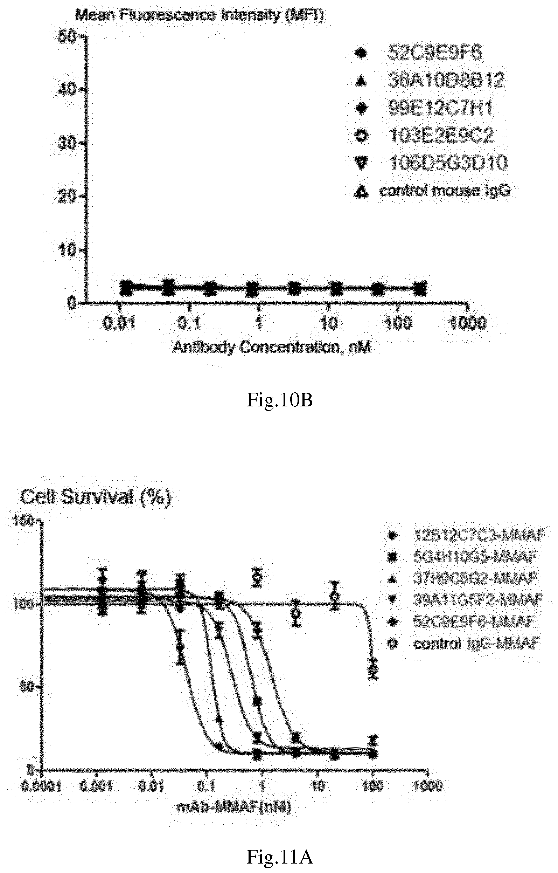

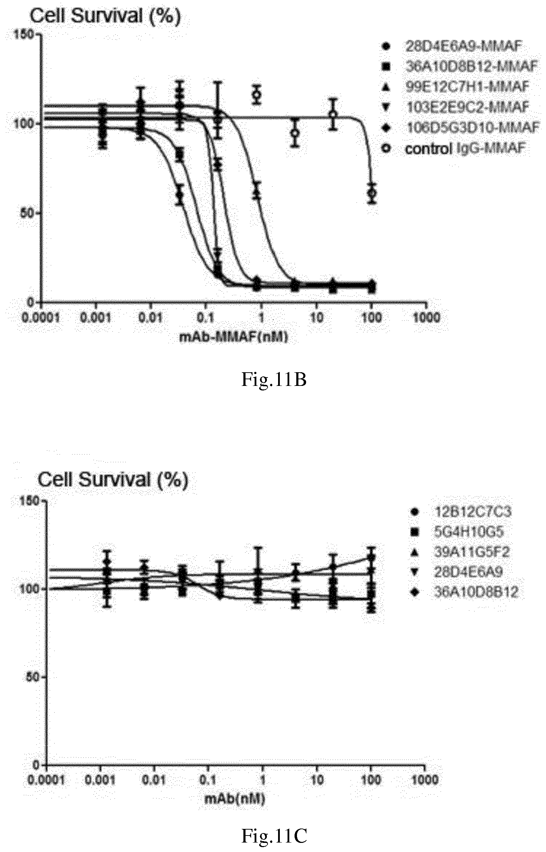

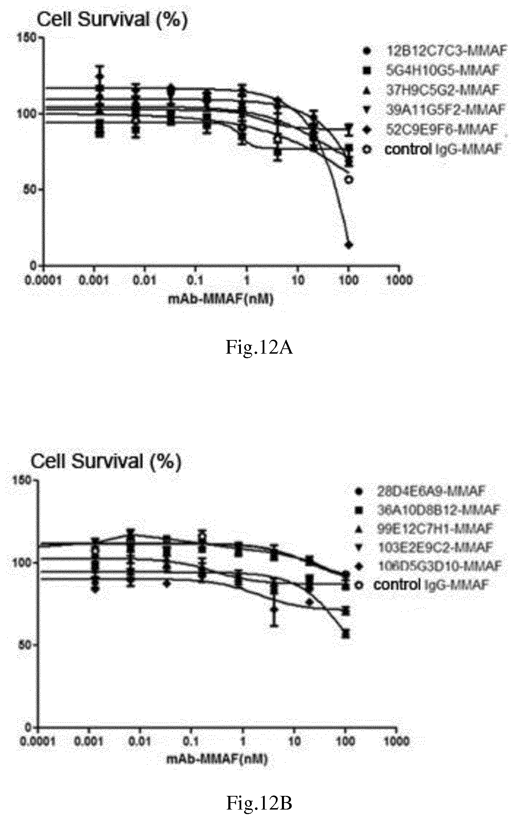

The obtained purified TPBG antibody-drug conjugates were serially diluted with complete medium, respectively. Each 100µL of TPBG-positive NCI-H1568 cell suspension (purchased from ATCC, Catalog NO:#CRL5876) containing 2000 cells was added to 96-wells cell culture plate and cultured overnight before adding with each 10 µL of purified TPBG antibody-drug conjugate at different concentrations. After further culturing the cells for 5 days, the cell viability was measured using the CellTiter-Glo kit (purchased from Promega and used according to instructions). Meanwhile, TPBG-negative tumor cell line NCI-H1770 (purchased from ATCC, Catalog NO: #CRL5893) was selected to perform cytotoxicity test as described above. Results were shown in Table 9 and Figs. 11-12, wherein the EC50 in Table 9 refers to a half effective amount of the cell activity that is inhibited after drug treatment, which reflects the cytotoxicity effects by measuring the cellular activity. Fig.11A and 11B represent the cytotoxicity of purified TPBG antibody-drug conjugate on TPBG-positive tumor cell line NCI-H1568. Fig.12A and 12B represent the cytotoxicity of purified TPBG antibody-drug conjugate on TPBG-negative tumor cell line NCI-H1770.

In addition, the cytotoxicity effects of the purified TPBG antibody obtained from Embodiment 2 alone were further measured, and the method of which is the same as that in Embodiment 4 for detecting cell cytotoxicity of TPBG antibody-drug conjugate. Results were shown in Table 9 and Fig.11C indicates that purified TPBG antibody alone does not have significant cytotoxicity effect on TPBG-positive cells.

**Table 9 Detection of Specific Cytotoxicity of TPBG Antibody-Drug Conjugate on TPBG-Positive Cells via Cellular Killing Assay**

| Clone Number | DAR | EC50(nM) | |
|---|---|---|---|
| | | NCI-1568(+) | NCI-1770(-) |
| 12B12C7C3-MMAF | 8 | 0.042 | Negative |
| 5G4H10G5-MMAF | 8 | 0.626 | Negative |
| 37H9C5G2-MMAF | 8 | 0.120 | Negative |
| 39A11G5F2-MMAF | 8 | 0.285 | Negative |
| 52C9E9F6-MMAF | 8 | 1.485 | Negative |
| 28D4E6A9-MMAF | 8 | 0.038 | Negative |
| 36A10D8B12-MMAF | 8 | 0.066 | Negative |
| 99E12C7H1-MMAF | 8 | 0.844 | Negative |
| 103E2E9C2-MMAF | 8 | 0.135 | Negative |
| 106D5G3D10-MMAF | 8 | 0.208 | Negative |
| 12B12C7C3 | 0 | Negative | Negative |
| 5G4H10G5 | 0 | Negative | Negative |
| 39A11G5F2 | 0 | Negative | Negative |
| 28D4E6A9 | 0 | Negative | Negative |
| 36A10D8B12 | 0 | Negative | Negative |

### Embodiment 5 Analysis of Epitope Distribution of TPBG Antibody and Antibody via Competitive ELISA Assay

The TPBG antibodies were grouped by competitive ELISA in order to identify the binding sites of the antibodies to antigens.

The purified antibodies to be tested were diluted to 1µg/mL using PBS, and coated with high-adsorption of 96-well microplates at 50ug per well. After overnight coating at 4°C, 250µL of blocking buffer (PBS containing 0.01% (v/v) Tween20 and 1% (w/w) BSA) was added to block coating at room temperature for 1h, and 0.05µg/mL of biotinylated recombinant TPBG protein was added to each well. Meanwhile, 5 µg/mL final concentration of competitive antibodies, i.e. the purified antibodies obtained from Embodiment 2 with the clone number of 12B12C7C3, 5G4H10G5, 37H9C5G2, 39A11G5F2, 52C9E9F6, 28D4E6A9, 36A10D8B12, 99E12C7H1, 103E2E9C2 and 106D5G3D10 were added and incubated at 25-37°C for 1-2h. The plates were washed three times with plate-washing buffer (PBS containing 0.01% (v/v) Tween20) and HRP (horseradish peroxidase)-labeled streptavidin (purchased from Sigma) was added. After incubating at 37°C for 0.5h, the plates were washed three times with plate-washing buffer (PBS containing 0.01% (v/v) Tween20), and 100µL of TMB substrate was added to each well and the plates were further incubated at room temperature for 30min prior to adding 100µL of stop buffer (1.0N HCl) to each well. A₄₅₀ₙₘ values were read using an ELISA microplate reader (SpectraMax 384plus purchased from Molecular Device) and results were shown in Fig.6. The competition rates between the antibodies were calculated according to the A₄₅₀ₙₘ values, and results were shown in Table 10. The higher the values of the competition rate, the closer the antigen surface of the two antibodies are.

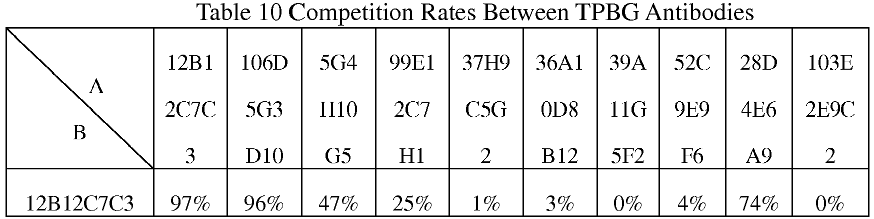

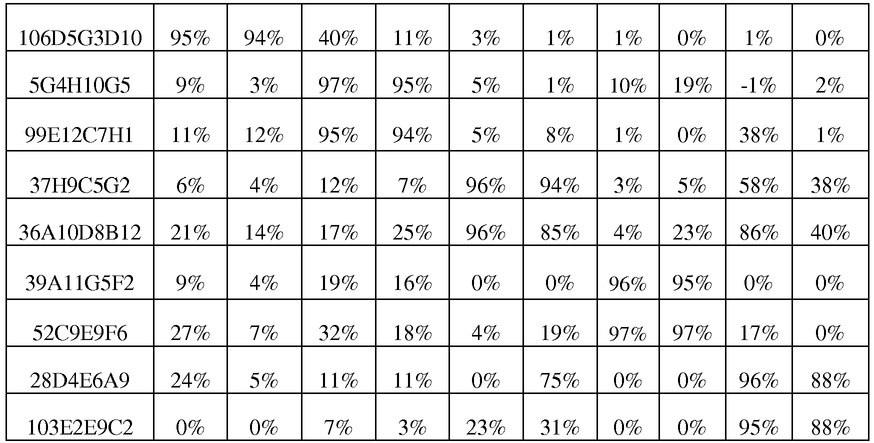

Among them, the meaning of A in Table 10 is that the concentration of coated antibody in each column is 11µg/mL; The meaning of B is that the concentration of competitive antibody in each row is 5µg/mL.

The results indicate that 12B12C7C3 and 106D5G3D10 can compete with each other and share similar epitopes; 5G4H10G5 and 99E12C7H1 can compete with each other and share similar epitopes; 37H9C5G2 and 36A10D8B12 can compete with each other and share similar epitopes; 39A11G5F2 and 52C9E9F6 can compete with each other and share similar epitopes; 28D4E6A9 and 103E2E9C2 can compete with each other and share similar epitopes.

### Embodiment 6 Amino Acid Sequence Determination of the Light Chain and Heavy Chain Variable Regions

Total RNA Extraction: 5×10⁷ hybridoma cells preparing in Embodiment 1 were collected by centrifugation, and 1mL of Trizol was added, mixed and then the mixture was transferred to a 1.5mL Eppendorf tube and allowed to stand at room temperature for 5min. Next, 0.2mL of chloroform was added, vortexed for 15s and the tube was centrifugated at 12,000g, 4°C for 5min and allowing to stand for 10min. The supernatant was transferred to a new 1.5mL Eppendorf tube, and 0.5mL of isopropanol was added, and the contents in the tube were mixed gently and allowed to stand for 10min at room temperature prior to centrifuging at 12,000g, 4°C for 15min. The supernatant was discarded, 1mL of 75% (v/v) ethanol was added and the pellets were rinsed gently and centrifugated at 12,000g for 5min. The supernatant was removed, the pellets were air-dried before dissolving with DEPC-treated water (water bath at 55°C for 10min facilitating dissolving), and finally the total RNA was extracted.

Reverse transcription and PCR: 1µg of total RNA was took and the reverse transcriptase was added, a 20µL system was set up and reacted at 42 °C for 60 minutes, the reaction was terminated at 85 °C for 10 minutes. A 50 µL PCR system was set with 1 µL of cDNA, 25 pmol of each primer, 1 µL of DNA polymerase, 250 µmol of dNTPs and a compatible buffer system. PCR program consisted of pre-denaturing at 95 °C for 3 minutes, 35 cycles of denaturing at 95 °C for 30 seconds, annealing at 55 °C for 30 seconds and extending at 72 °C for 35 seconds, followed by extension at 72 °C for 5 minutes to obtain a PCR product. The kit used for reverse transcription was PrimeScript RT Master Mix purchased from Takara, with Cat. No. RR036; the kit used for PCR including the Q5 high-fidelity enzyme was purchased from NEB, with Cat. No. M0492.

Cloning and sequencing: 5 µL of PCR product was used to test by agarose gel electrophoresis, and positive samples were purified by column recovery kit NucleoSpin® Gel & PCR Clean-up, purchased from MACHEREY-NAGEL with a Cat. No. of 740609. Ligation was carried out by using 50 ng of sample, 50 ng of T vector, 0.5 µL of recombinase Exnaseligase and 1 µL of buffer to a final reaction system volume of 10µL, and reacted at 16 °C for half an hour to obtain the ligated product, wherein the ligation kit is T4 DNA ligase purchased from NEB with a Cat. No. of M0402; 5 µL of the ligation product was pipetted into 100 µL of competent cells (Ecos 101competent cells, purchased from Yeastern, Cat. No. FYE607) and ice-cooled for 5 minutes. Subsequently the competent cells were heat shocked at 42 °C for 1 minute in water bath and placed on ice for 1 minutes, then 650 µL of antibiotic-free SOC medium was added and the competent cells were resuscitated at 200 RPM on a shaker at 37 °C for 30 minutes, 200 µL of cells suspension was pipetted and spread on LB solid medium containing antibiotic and cultured overnight in 37 °C incubator. On the next day, colony PCR was performed in a 30 µL PCR system using primers M13F and M13R specifically deisigned for T vector, bacterial colonies were picked by a tip and pipetted into the PCR system and mixed, and 0.5 µL of suspension was picked onto another LB solid plate containing 100 µg/mL ampicillin to preserve the bacterial strain; 5 µL of product was subjected to the detection of agarose gel electrophoresis when the PCR reaction is completed, and the positive samples were sequenced and analyzed [see Kabat, "Sequences of Proteins of Immunological Interest," National Institutes of Health, Bethesda, Md. (1991)]. The sequencing results were shown in Tables 11-12.

**Table 11 Protein Sequences Number of TPBG Antibodies**

| Clone Number | Heavy Chain Protein | | | | Light Chain Protein | | | |
|---|---|---|---|---|---|---|---|---|
| | Variable Region | CDR1 | CDR2 | CDR3 | Variable Region | CDR1 | CDR2 | CDR3 |
| 12B12C7C3 | 1 | 2 | 3 | 4 | 5 | 6 | 7 | 8 |
| 5G4H10G5 | 9 | 10 | 11 | 12 | 13 | 14 | 15 | 16 |
| 37H9C5G2 | 17 | 18 | 19 | 20 | 21 | 22 | 23 | 24 |
| 39A11G5F2 | 25 | 26 | 27 | 28 | 29 | 30 | 31 | 32 |
| 52C9E9F6 | 33 | 34 | 35 | 36 | 37 | 38 | 39 | 40 |
| 28D4E6A9 | 41 | 42 | 43 | 44 | 45 | 46 | 47 | 48 |
| 36A10D8B12 | 49 | 50 | 51 | 52 | 53 | 54 | 55 | 56 |
| 99E12C7H1 | 57 | 58 | 59 | 60 | 61 | 62 | 63 | 64 |
| 103E2E9C2 | 65 | 66 | 67 | 68 | 69 | 70 | 71 | 72 |
| 106D5G3D10 | 73 | 74 | 75 | 76 | 77 | 78 | 79 | 80 |

In Table 11, the numbers are the sequence numbers in sequence listing, e.g. the amino acid sequence of the variable region in the heavy chain protein of 12B 12C7C3 is set forth as SEQ ID NO:1, while the CDR1 amino acid sequence in the heavy chain protein variable region of 12B 12C7C3 is set forth as SEQ ID NO:2.

**Table 12 The gene sequence numbers of TPBG antibody**

| Clone Number | Heavy Chain Protein Variable Region | Light Chain Protein Variable Region |
|---|---|---|
| 12B12C7C3 | 81 | 82 |
| 5G4H10G5 | 83 | 84 |
| 37H9C5G2 | 85 | 86 |
| 39A11G5F2 | 87 | 88 |
| 52C9E9F6 | 89 | 90 |
| 28D4E6A9 | 91 | 92 |
| 36A10D8B12 | 93 | 94 |
| 99E12C7H1 | 95 | 96 |
| 103E2E9C2 | 97 | 98 |
| 106D5G3D10 | 99 | 100 |

The numbers in Table 12 are the sequence numbers in sequence listing, e.g. the nucleotide sequence encoding the variable region in the heavy chain protein of 12B12C7C3 is the nucleotide sequence shown in SEQ ID NO:81;
wherein the nucleotide sequence encoding the CDR1 in the heavy chain variable region of 12B12C7C3 is the nucleotide sequence from 91 to 105 shown in SEQ ID NO:81;
the nucleotide sequence encoding the CDR2 in the heavy chain variable region of 12B12C7C3 is the nucleotide sequence from 151 to 198 in SEQ ID NO:81;
the nucleotide sequence encoding the CDR3 in the heavy chain variable region of 12B12C7C3 is the nucleotide sequence from 295 to 327 in SEQ ID NO:81;
the nucleotide sequence encoding the CDR1 in the light chain variable region of 12B12C7C3 is the nucleotide sequence from 70 to 114 in SEQ ID NO:82;
the nucleotide sequence encoding the CDR2 in the light chain variable region of 12B12C7C3 is the nucleotide sequence from 157 to 180 in SEQ ID NO:82;
the nucleotide sequence encoding the CDR3 in the light chain variable region of 12B12C7C3 is the nucleotide sequence from 277 to 303 in SEQ ID NO:82;
the nucleotide sequence encoding the CDR1 in the heavy chain variable region of 5G4H10G5 is the nucleotide sequence from 91 to 105 in SEQ ID NO:83;
the nucleotide sequence encoding the CDR2 in the heavy chain variable region of 5G4H10G5 is the nucleotide sequence from 148 to 198 in SEQ ID NO:83;
the nucleotide sequence encoding the CDR3 in the heavy chain variable region of 5G4H10G5 is the nucleotide sequence from 295 to 330 in SEQ ID NO:83;
the nucleotide sequence encoding the CDR1 in the light chain variable region of 5G4H10G5 is the nucleotide sequence from 70 to 102 in SEQ ID NO:84;
the nucleotide sequence encoding the CDR2 in the light chain variable region of 5G4H10G5 is the nucleotide sequence from 148 to 168 in SEQ ID NO:84;
the nucleotide sequence encoding the CDR3 in the light chain variable region of 5G4H10G5 is the nucleotide sequence from 265 to 288 in SEQ ID NO:84;
the nucleotide sequence encoding the CDR1 in the heavy chain variable region of 37H9C5G2 is the nucleotide sequence from 91 to 105 in SEQ ID NO:85;
the nucleotide sequence encoding the CDR2 in the heavy chain variable region of 37H9C5G2 is the nucleotide sequence from 151 to 198 in SEQ ID NO:85;
the nucleotide sequence encoding the CDR3 in the heavy chain variable region of 37H9C5G2 is the nucleotide sequence from 295 to 327 in SEQ ID NO:85;
the nucleotide sequence encoding the CDR1 in the light chain variable region of 37H9C5G2 is the nucleotide sequence from 70 to 102 in SEQ ID NO:86;
the nucleotide sequence encoding the CDR2 in the light chain variable region of 37H9C5G2 is the nucleotide sequence from 148 to 168 in SEQ ID NO:86;
the nucleotide sequence encoding the CDR3 in the light chain variable region of 37H9C5G2 is the nucleotide sequence from 265 to 291 in SEQ ID NO:86;
the nucleotide sequence encoding the CDR1 in the heavy chain variable region of 39A11G5F2 is the nucleotide sequence from 91 to 105 in SEQ ID NO:87;
the nucleotide sequence encoding the CDR2 in the heavy chain variable region of 39A11G5F2 is the nucleotide sequence from 151 to 198 in SEQ ID NO:87;
the nucleotide sequence encoding the CDR3 in the heavy chain variable region of 39A11G5F2 is the nucleotide sequence from 295 to 315 in SEQ ID NO:87;
the nucleotide sequence encoding the CDR1 in the light chain variable region of 39A11G5F2 is the nucleotide sequence from 70 to 102 in SEQ ID NO:88;
the nucleotide sequence encoding the CDR2 in the light chain variable region of 39A11G5F2 is the nucleotide sequence from 148 to 168 in SEQ ID NO:88;
the nucleotide sequence encoding the CDR3 in the light chain variable region of 39A11G5F2 is the nucleotide sequence from 265 to 291in SEQ ID NO:88;
the nucleotide sequence encoding the CDR1 in the heavy chain variable region of 52C9E9F6 is the nucleotide sequence from 91 to 105 in SEQ ID NO:89;
the nucleotide sequence encoding the CDR2 in the heavy chain variable region of 52C9E9F6 is the nucleotide sequence from151 to 198 in SEQ ID NO:89;
the nucleotide sequence encoding the CDR3 in the heavy chain variable region of 52C9E9F6 is the nucleotide sequence from 295 to 315 in SEQ ID NO:89;
the nucleotide sequence encoding the CDR1 in the light chain variable region of 52C9E9F6 is the nucleotide sequence from 70 to 102 in SEQ ID NO:90;
the nucleotide sequence encoding the CDR2 in the light chain variable region of 52C9E9F6 is the nucleotide sequence from 148 to 168 in SEQ ID NO:90;
the nucleotide sequence encoding the CDR3 in the light chain variable region of 52C9E9F6 is the nucleotide sequence from 268 to 291 in SEQ ID NO:90;
the nucleotide sequence encoding the CDR1 in the heavy chain variable region of 28D4E6A9 is the nucleotide sequence from 91 to 105 in SEQ ID NO:91;
the nucleotide sequence encoding the CDR2 in the heavy chain variable region of 28D4E6A9 is the nucleotide sequence from 148 to 198 in SEQ ID NO:91;
the nucleotide sequence encoding the CDR3 in the heavy chain variable region of 28D4E6A9 is the nucleotide sequence from 295 to 327 in SEQ ID NO:91;
the nucleotide sequence encoding the CDR1 in the light chain variable region of 28D4E6A9 is the nucleotide sequence from 70 to 102 in SEQ ID NO:92;
the nucleotide sequence encoding the CDR2 in the light chain variable region of 28D4E6A9 is the nucleotide sequence from 148 to 168 in SEQ ID NO:92;
the nucleotide sequence encoding the CDR3 in the light chain variable region of 28D4E6A9 is the nucleotide sequence from 265 to 291 in SEQ ID NO:92;
the nucleotide sequence encoding the CDR1 in the heavy chain variable region of 36A10D8B12 is the nucleotide sequence from 91 to 108 in SEQ ID NO:93;
the nucleotide sequence encoding the CDR2 in the heavy chain variable region of 36A10D8B12 is the nucleotide sequence from 154 to 198 in SEQ ID NO:93;
the nucleotide sequence encoding the CDR3 in the heavy chain variable region of 36A10D8B12 is the nucleotide sequence from 295 to 324 in SEQ ID NO:93;
the nucleotide sequence encoding the CDR1 in the light chain variable region of 36A10D8B12 is the nucleotide sequence from 70 to 102 in SEQ ID NO:94;
the nucleotide sequence encoding the CDR2 in the light chain variable region of 36A10D8B12 is the nucleotide sequence from 148 to 168 in SEQ ID NO:94;
the nucleotide sequence encoding the CDR3 in the light chain variable region of 36A10D8B12 is the nucleotide sequence from 265 to 291in SEQ ID NO:94;
the nucleotide sequence encoding the CDR1 in the heavy chain variable region of 99E12C7H1 is the nucleotide sequence from 91 to 105 in SEQ ID NO:95;
the nucleotide sequence encoding the CDR2 in the heavy chain variable region of 99E12C7H1 is the nucleotide sequence from 151 to 198 in SEQ ID NO:95;
the nucleotide sequence encoding the CDR3 in the heavy chain variable region of 99E12C7H1 is the nucleotide sequence from 295 to 318 in SEQ ID NO:95;
the nucleotide sequence encoding the CDR1 in the light chain variable region of 99E12C7H1 is the nucleotide sequence from 70 to 102 in SEQ ID NO:96;
the nucleotide sequence encoding the CDR2 in the light chain variable region of 99E12C7H1 is the nucleotide sequence from 148 to 168 in SEQ ID NO:96;
the nucleotide sequence encoding the CDR3 in the light chain variable region of 99E12C7H1 is the nucleotide sequence from 265 to 291 in SEQ ID NO:96;
the nucleotide sequence encoding the CDR1 in the heavy chain variable region of 103E2E9C2 is the nucleotide sequence from 91 to 105 in SEQ ID NO:97;
the nucleotide sequence encoding the CDR2 in the heavy chain variable region of 103E2E9C2 is the nucleotide sequence from 151 to 198 in SEQ ID NO:97;
the nucleotide sequence encoding the CDR3 in the heavy chain variable region of 103E2E9C2 is the nucleotide sequence from 295 to 324 in SEQ ID NO:97;
the nucleotide sequence encoding the CDR1 in the light chain variable region of 103E2E9C2 is the nucleotide sequence from 70 to 105 in SEQ ID NO:98;
the nucleotide sequence encoding the CDR2 in the light chain variable region of 103E2E9C2 is the nucleotide sequence from 151 to 171 in SEQ ID NO:98;
the nucleotide sequence encoding the CDR3 in the light chain variable region of 103E2E9C2 is the nucleotide sequence from 268 to 294 in SEQ ID NO:98;
the nucleotide sequence encoding the CDR1 in the heavy chain variable region of 106D5G3D10 is the nucleotide sequence from 91 to 105 in SEQ ID NO:99;
the nucleotide sequence encoding the CDR2 in the heavy chain variable region of 106D5G3D10 is the nucleotide sequence from 151 to 198 in SEQ ID NO:99;
the nucleotide sequence encoding the CDR3 in the heavy chain variable region of 106D5G3D10 is the nucleotide sequence from 295 to 318 in SEQ ID NO:99;
the nucleotide sequence encoding the CDR1 in the light chain variable region of 106D5G3D10 is the nucleotide sequence from 70 to 99 in SEQ ID NO:100;
the nucleotide sequence encoding the CDR2 in the light chain variable region of 106D5G3D10 is the nucleotide sequence from 145 to 165 in SEQ ID NO:100;
the nucleotide sequence encoding the CDR3 in the light chain variable region of 106D5G3D10 is the nucleotide sequence from 262 to 294 in SEQ ID NO:100.

### Embodiment 7 Construction, Production and Purification of Mouse-Human Chimeric Antibody

1. Plasmid Construction and Preparation: The sequences of heavy chain variable region and light chain variable region of TPBG antibodies were confirmed by the sequencing results from Embodiment 6. The sequences of the heavy chain variable regions in the lead antibodies obtained from Embodiment 2 and Embodiment3 were recombined into an expression vector containing a signal peptide and a constant region of human heavy chain antibody IgG1 (the expression vector was purchased from Invitrogen, and the recombination process was completed by ChemPartner, Shanghai), and the sequences of the light chain variable regions in the TPBG antibodies were recombined into an expression plasmid containing a signal peptide and a constant region of human light chain kappa, and the recombined plasmids were confirmed by sequencing (the sequencing method of which is the same as that in Embodiment 6). High-purity of recombinant plasmid was extracted by an alkaline lysis kit (purchased from MECHEREY-NAGEL), and more than 500µg of plasmid was collected and filtered through a 0.22 µm membrane filter (purchased from Millipore) for following transfection.
2. Cell Transfection: 293E cells (purchased from Invitrogen) were cultured in Freestyle 293 expression medium (purchased from Invitrogen). The shaker was set at 37°C, 130rpm and 8%CO₂ (v/v). During transfection, 10% (v/v) of F68 (purchased from Invitrogen) was added to Freestyle 293 expression medium to a final F68 concentration of 0.1% (v/v), and the Freestyle 293 culture medium containing 0.1% (v/v) F68, i.e. culture medium A was prepared. 5mL of culture medium A was mixed with 200µg/mL PEI (purchased from Sigma), obtaining culture medium B. 5mL of culture medium A was mixed with 100µg/mL of recombinant plasmid obtained in step 1 to prepare culture medium C. After 5min, culture medium B and C were pooled and mixed, and allowed to stand for 15min to prepare mixture D. 10 mL of mixture D was slowly added into 100mL of Freestyle 293 expression medium containing 293E cells, with simultaneously oscillation to avoid over-aggregation of PEI, until the density of the 293E cells reach 1.5×10⁶/mL and the cells were cultured in the shaker. Peptone was added the next day until its concentration reached 0.5% (w/v). From day5 to day7, the titer of antibodies in the culture were tested. From day6 to day7, the supernatant was collected by centrifugation (3500rpm, 30min) and filtered through a 0.22µm of membrane filter to prepare a filtered cell supernatant for purification.
3. Antibody Purification: Endotoxin-free chromatography columns and Protein A filler in continuous production state were treated with 0.1M NaOH for 30min or washed with 5 column bed volumes of 0.5M NaOH. Fillers and chromatography columns without being used for a long time were soaked in 1M NaOH for at least 1h, follow by neutralizing with endotoxin-free water and rinsing with 10 column bed volumes of 1% Triton X100. The column was equilibrated with 5 column bed volumes of PBS, and the filtered cells supernatant were loaded on the column and the flow-through portion of the sample was collected if necessary. After finishing sample loading, 5 column bed volumes of PBS was used to wash the column. 5 column bed volumes of 0.1M pH3.0 Glycine-HCl was used to elute, follow by collecting eluate and neutralizing column with 1/10 volume of 1M Tris-HCl, pH8.5. The antibody was harvested and then dialyzed in 1×PBS overnight to prevent endotoxin contamination. Then, the concentration was determined by spectrophotometer or kit, the purity of the antibody was determined by HPLC-SEC, and the content of endotoxin was determined by endotoxin detection kit (purchased from Lonza).

In the following embodiments, the first segment of characters in the names of the chimeric antibodies were selected from the first 3-5 characters in their corresponding lead antibodies, e.g. the clone number of the lead antibody corresponding to the chimeric antibody 12B12-MMAF is 12B12C7C3, and the clone number of the lead antibody corresponding to the chimeric antibody 5G4-MMAF is 5G4H10G5, etc.

### Embodiment 8 In Vitro Pharmacodynamic Studies of Chimeric Antibodies

The purified TPBG chimeric antibodies obtained from Embodiment 7 were coupled with MC-MMAF using the method described in Embodiment 4. After dialysis with sodium borate buffer pH 6.5-8.5, Tris (2-carboxyethyl) phosphine (TCEP) was added, wherein the molar ratio of TCEP to the purified TPBG antibodies was 2. Reaction solution A was obtained by reductive reaction at room temperature for 1h. The reaction solution A was desalted on G25 (purchased from GE) to remove excess TCEP and hence reaction buffer B was obtained. MC-MMAF was added to reaction buffer B, wherein the molar ratio of MC-MMAF to the purified TPBG antibody was 5, and the reaction was carried out at room temperature for 4h. Cysteine was added to neutralize excess MC-MMAF, excess small molecules were removed by G25 desalting, follow by obtaining purified TPBG antibody-drug conjugate (for the method of conjugation, refers to Doronina, 2006, Bioconjugate Chem.17, 114-124). Drug-antibody ratio of the drugs were analyzed by HIC, the purity of the antibody-drug conjugates was analyzed by SEC, and the cytotoxicity was analyzed subsequently. The drug-antibody ratio (DAR) of all antibody-drug conjugates was 3.0-5.0, wherein DAR (drug-antibody ratio) refers to the average number of small-molecule drugs carried on a single antibody molecule after antibody conjugation.

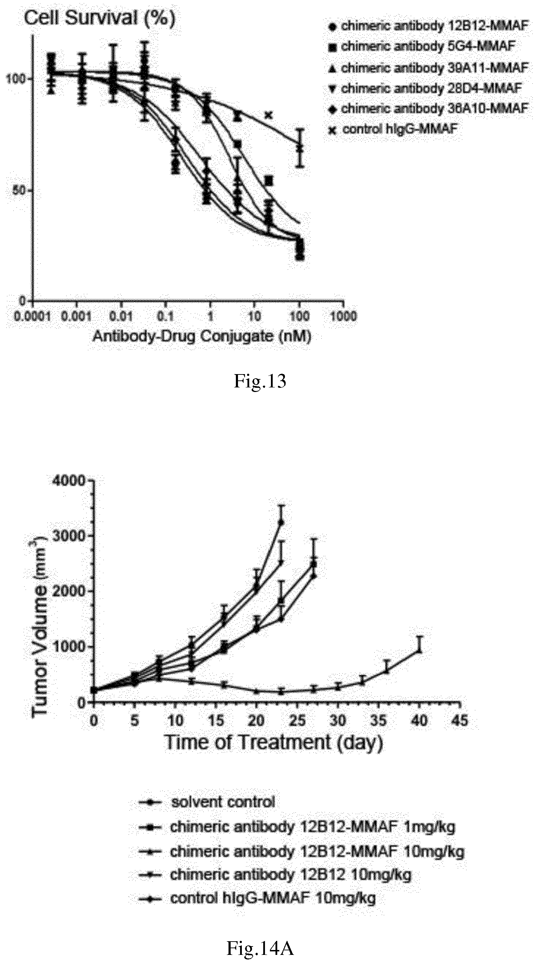

The obtained purified TPBG antibody-drug conjugates were serially diluted with complete medium, respectively. 100µL of TPBG-positive NCI-H1299 cell suspension was added to a 96-well plate at 2000 cells per well. After incubating the cell suspension overnight, 10µL of purified TPBG chimeric antibody-drug conjugate diluents at different concentrations were added to each well. After 5 days further culturing, the cell viability was measured using the CellTiter-Glo kit (purchased from Promega and used according to instructions). Results were shown in Table 13 and Fig. 13, wherein IC50 in Table 13 refers to a half effective amount of the cell activity that is inhibited after drug treatment, which can reflect the cytotoxicity by measuring cell viability. Fig. 13 shows the cytotoxicity of purified TPBG chimeric antibody-drug conjugate on the TPBG-positive tumor cell line NCI-H1299. The results indicate that the purified TPBG antibody-drug conjugates have cytotoxicity effects on TPBG-positive cells.

**Table 13 Detection of Specific Cytotoxicity of Purified TPBG Chimeric Antibody-Drug Conjugates on TPBG-Positive NCI-H1299 Cells via Cellular Killing Assay**

| Sample Name | IC50 (nM) |
|---|---|
| | NCI-H1299(+) |
| Chimeric Antibody 12B12-MMAF | 0.13 |
| Chimeric Antibody 5G4-MMAF | 87.27 |
| Chimeric Antibody 39A11-MMAF | 4.30 |
| Chimeric Antibody 28D4-MMAF | 0.21 |
| Chimeric Antibody 36A10-MMAF | 0.70 |
| Control hIgG-MMAF | >100 |

### Embodiment 9 In Vivo Pharmacodynamic Studies of Chimeric Antibodies

200µL of NCI-H1299 (non-small cell lung cancer cell line, ATCC, CRL-5803) (5×10⁶ cells) were inoculated subcutaneously in the right rib of Balb/c nude mice and allowed to grow for 7-10 days until the tumor volumes reach 200mm³. After removing the mice with too large or too small body weight and tumor size, the mice were randomly divided into several groups, i.e. 7 mice for each group, according to the tumor size. Antibody was injected intravenously to the tail at D0, and the injection was performed once every four days with a total of four injections. Tumor volume and body weight of mice were measured every two weeks, and the data were recorded. Formulation for calculating tumor volume is: V=1/2 × a × b2; wherein a and b represents length and width, respectively. Grouping was shown in Table 14.

**Table 14 In Vivo Pharmacodynamic Studies of TPBG Chimeric Antibodies and Its Antibody-Drug Conjugates**

| Group | Numb er of Anim als | Treating Group | Dose (mg/ kg) | Injected Amoun t (µl/g) | Route of Administa -tion | Administra-tio n Plan |
|---|---|---|---|---|---|---|
| 1 | 7 | Solvent Control | -- | 10 | Tail intravenous injection i.v. | Administer once every 4 days × 4 times |
| 2 | 7 | Chimeric Antibody 12B12-MMAF | 1 | 10 | Tail intravenous injection i.v. | Administer once every 4 days × 4 times |
| 3 | 7 | Chimeric Antibody 12B12-MMAF | 10 | 10 | Tail intravenous injection i.v. | Administer once every 4 days × 4 times |
| 4 | 7 | Chimeric Antibody 5G4-MMAF | 1 | 10 | Tail intravenous injection i.v. | Administer once every 4 days × 4 times |
| 5 | 7 | Chimeric Antibody 5G4-MMAF | 10 | 10 | Tail intravenous injection i.v. | Administer once every 4 days × 4 times |
| 6 | 7 | Chimeric Antibody 39A11-MMAF | 1 | 10 | Tail intravenous injection i.v. | Administer once every 4 days × 4 times |
| 7 | 7 | Chimeric Antibody 39A11-MMAF | 10 | 10 | Tail intravenous injection i.v. | Administer once every 4 days × 4 times |
| 8 | 7 | Chimeric Antibody 28D4-MMAF | 1 | 10 | Tail intravenous injection i.v. | Administer once every 4 days × 4 times |
| 9 | 7 | Chimeric Antibody 28D4-MMAF | 10 | 10 | Tail intravenous injection i.v. | Administer once every 4 days × 4 times |
| 10 | 7 | Chimeric Antibody 36A10-MMAF | 1 | 10 | Tail intravenous injection i.v. | Administer once every 4 days × 4 times |
| 11 | 7 | Chimeric Antibody 36A10-MMAF | 10 | 10 | Tail intravenous injection i.v. | Administer once every 4 days × 4 times |
| 12 | 7 | Chimeric Antibody 12B12 | 10 | 10 | Tail intravenous injection i.v. | Administer once every 4 days × 4 times |
| 13 | 7 | Chimeric Antibody 5G4 | 10 | 10 | Tail intravenous injection i.v. | Administer once every 4 days × 4 times |
| 14 | 7 | Chimeric Antibody 39A11 | 10 | 10 | Tail intravenous injection i.v. | Administer once every 4 days × 4 times |
| 15 | 7 | Chimeric Antibody 28D4 | 10 | 10 | Tail intravenous injection i.v. | Administer once every 4 days × 4 times |
| 16 | 7 | Chimeric Antibody 36A10 | 10 | 10 | Tail intravenous injection i.v. | Administer once every 4 days × 4 times |
| 17 | 7 | Control hIgG-MMAF | 10 | 10 | Tail intravenous injection i.v. | Administer once every 4 days × 4 times |

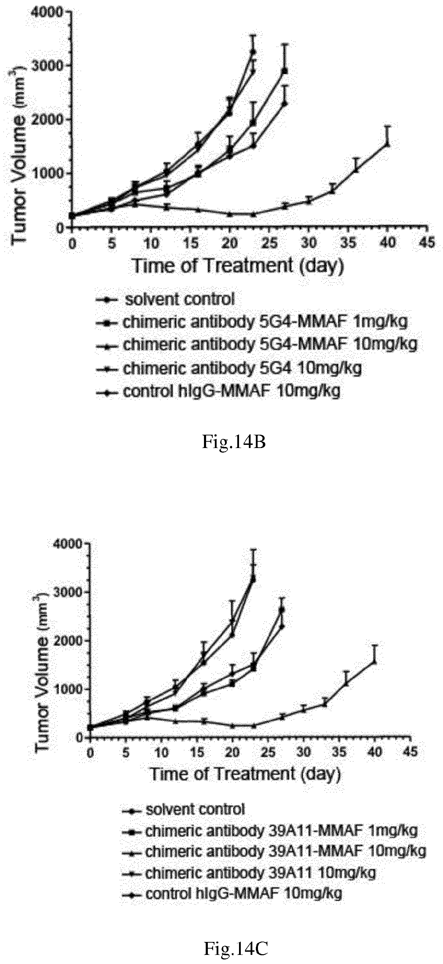

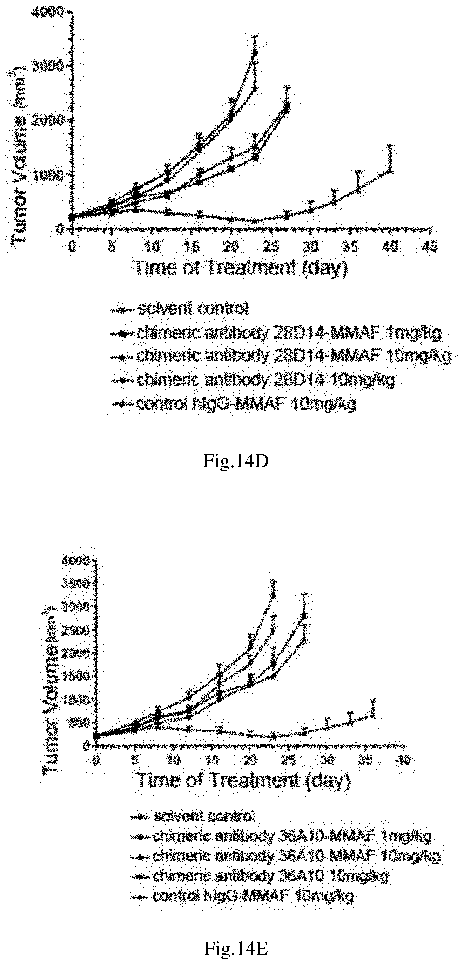

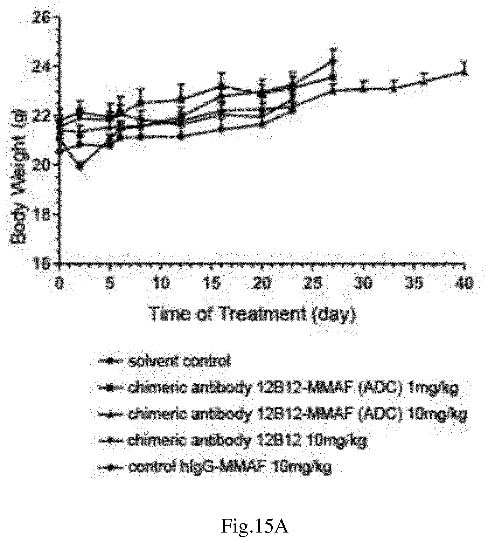

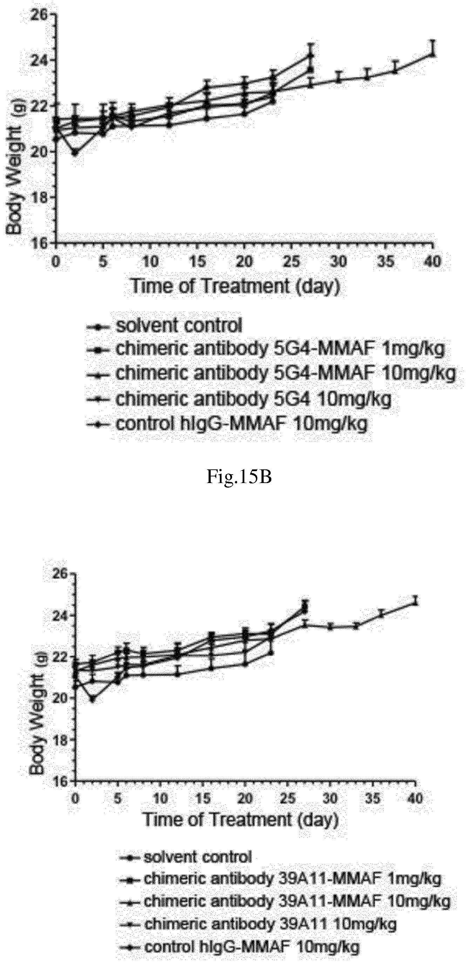

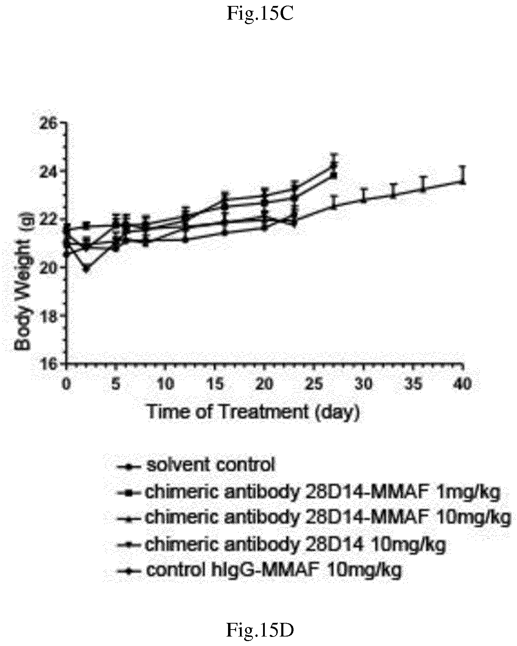

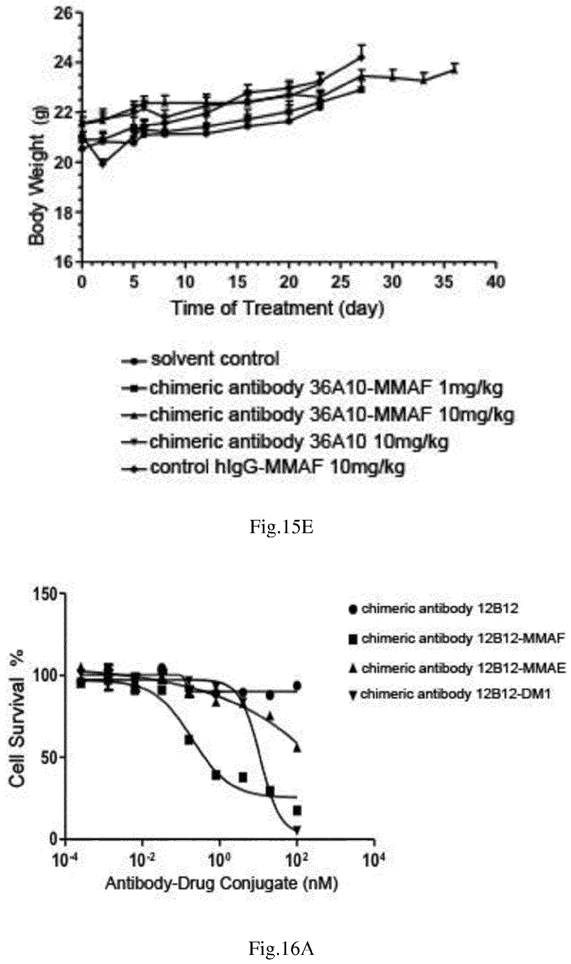

Results were shown in Fig. 14: changes in tumor size after treatment; and Fig.15: changes in body weight of mice after treatment, wherein Figs. 14A-E were changes in tumor size after treatment of antibody-drug conjugates of chimeric antibodies 12B12, 5G4, 39A11, 28D4 and 36A10 and their naked antibodies. Figs.15A-E were changes in body weight of mice after treatment of antibody-drug conjugates of chimeric antibodies 12B12, 5G4, 39A11, 28D4 and 36A10 and their naked antibodies. The results demonstrated that the above ADCs can efficiently inhibit the growth of NCI-H1299 tumor and have no significant effect on the body weight of mice.

### Embodiment 10 In Vitro Pharmacodynamic Studies of Antibody-drug Conjugates Coupled with Different Linker-Toxins

Purified TPBG chimeric antibodies 12B12 and 28D4 obtained from Embodiment 7 were coupled with MC-MMAF and MC-VC-PAB-MMAE, respectively, using the method described in Embodiment 8. After dialysis with sodium borate buffer with pH 6.5-8.5, tris(2-carboxyethyl) phosphine (TCEP) was added, wherein the molar ratio of TCEP to the purified TPBG antibodies was 2. Reductive reaction was carried out at room temperature for 1h, and reaction solution A was obtained. Excess TCEP was removed by desalting reaction solution A with G25 (purchased from GE), and then reaction solution B was obtained. MC-MMAF or MC-VC-PAB-MMAE (purchased from Levena Biomart, Nanjing) was added to reaction solution B, wherein the molar ratio of MC-MMAF or MC-VC-PAB-MMAE to purified TPBG antibody was 5. The reaction was carried out at room temperature for 4h. Cysteine was further added to neutralize excess MC-MMAF or MC-VC-PAB-MMAE, and excess small molecules were removed by G25 desalting, follow by obtaining purified TPBG antibody-drug conjugate-i.e. the chimeric antibodies 12B12-MMAF, 12B12-MMAE, 28D4-MMAF, 28D4-MMAE listed in the table (as for the method of conjugation please refer to Doronina, 2006, Bioconjugate Chem.17,114-124). Drug-antibody ratio of the drugs were analyzed by HIC, the purity of the antibody-drug conjugates was analyzed by SEC, follow by determining the cytotoxicity of conjugates.

The purified chimeric antibodies 12B12 and 28D4 obtained from Embodiment 7 were coupled with SMCC, respectively. After dialyzing the purified antibodies obtained in Embodiment 7 with phosphate buffer pH 6.5-7.4, succinimidyl 4-(N-maleimidomethyl) cyclohexane-1-carboxylate (SMCC) was added in the presence of 30% (v/v) dimethylacetamide (DMA), wherein the molar ratio of SMCC to the purified TPBG chimeric antibodies was 8. Reaction solution A was obtained after the reaction was carried out at room temperature for 1h. Excess small molecules were removed by desalting reaction solution A with G25 column (purchased from GE) to obtain reaction solution B. DMA was added to reaction solution B until a final volume percent of 10% was reached, and DM1 (N2 ' -deacetyl-N2 ' -(3-mercapto-1-oxypropyl)-maytansine) was then added, wherein the molar ratio of DM1 to purified TPBG antibodies was 9. Reaction solution C was obtained once the reaction was carried out at room temperature for 3.5h. Reaction solution C was subjected to desalting by G25 (purchased from GE) to remove excess small molecules, and the purified TPBG chimeric antibody-drug conjugates-i.e. the chimeric antibodies 12B12-DM1 and 28D4-DM1 listed in the table (for the coupling method please refer to US5208020) were obtained. After the drug-antibody ratio was determined by LC-MS and the purity of the antibody-drug conjugates is analyzed by SEC, the cytotoxicity effects of conjugates were determined as well. The drug-antibody ratios (DAR) of all chimeric antibody-drug conjugates were about 3.0-5.0, wherein DAR (drug antibody ratio) refers to the average number of small-molecule drugs carried by a single antibody molecule after antibody conjugation.

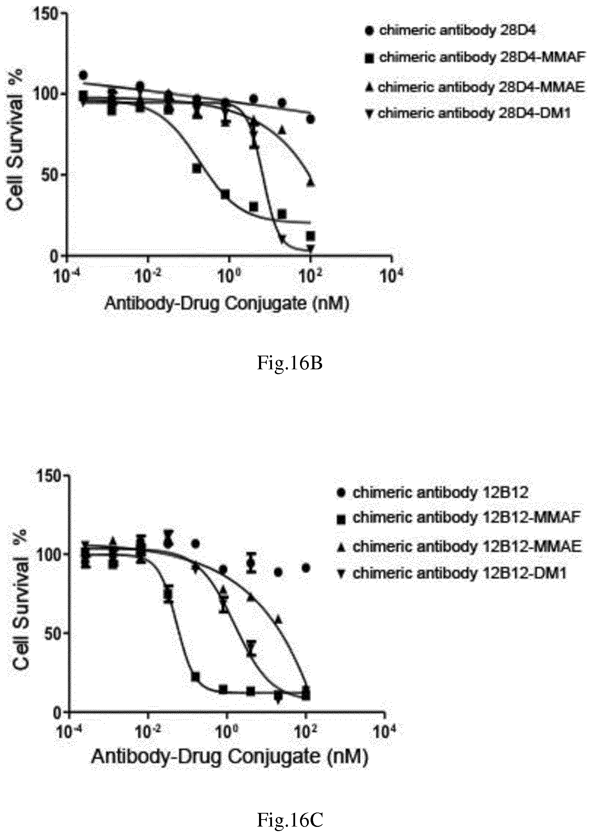

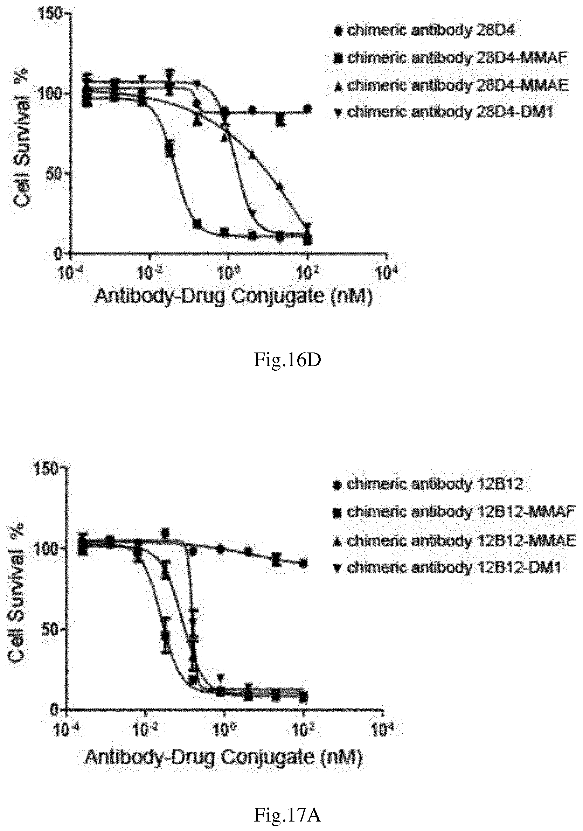

The obtained purified TPBG antibody-drug conjugates were serially diluted with complete medium. 100µL of TPBG-positive NCI-H1299 (purchased from ATCC, Catalog NO: #CRL5803) or NCI-H1568 (purchased from ATCC, Catalog NO: #CRL5876) cell suspension was added to the 96-well plate at 2000 cells per cell, cultured overnight, and 10µL of purified TPBG chimeric antibody-drug conjugate diluents of different concentrations were added to each well. After further culturing the cells for 5 days, cell viability was measured using the CellTiter-Glo kit (purchased from Promega and used according to instructions). Results were shown in Table 15 and Fig. 16, wherein IC50 in Table 13 refers to a half effective amount of the cell activity that is inhibited after drug treatment, reflecting the cytotoxicity by measuring cell viability. Fig.16A and 16B represent the cytotoxicity of the purified TPBG chimeric antibody 12B12 and its antibody-drug conjugates coupled with different small-molecule drugs on TPBG-positive tumor cell line NCI-H1299; Figs.16C and 16D represent the cytotoxicity of the purified TPBG chimeric antibody 12B12 and its antibody-drug conjugates coupled with different small-molecule drugs on TPBG-positive tumor cell line NCI-H1568. The results indicates that all the purified TPBG chimeric antibody-drug conjugates coupling with different small-molecule toxins have different degrees of cytotoxicity effects on TPBG-positive cells, and the TPBG chimeric antibody-drug conjugates coupling with MC-MMAF possess a low value of IC50, indicating that it has the strongest killing efficacy.

**Table 15 Detection of Specific Cytotoxicity of Purified TPBG Chimeric Antibody-Drug Conjugates on TPBG-Positive HCI-H1299 and HCI-H1568 Cells via Cellular Killing Assay**

| Sample Name | IC50 (nM) | |
|---|---|---|
| | NCI-H1299 | NCI-H1568 |
| Chimeric Antibody 12B12 | None | None |
| Chimeric Antibody 12B12-MMAF | 0.04 | 0.025 |
| Chimeric Antibody 12B12-MMAE | >100 | >100 |
| Chimeric Antibody 12B12-DM1 | 10.42 | 1.271 |
| Chimeric Antibody 28D4 | None | None |
| Chimeric Antibody 28D4-MMAF | 0.132 | 0.066 |
| Chimeric Antibody 28D4-MMAE | 368.6 | >100 |
| Chimeric Antibody 28D4-DM1 | 6.084 | 2.507 |

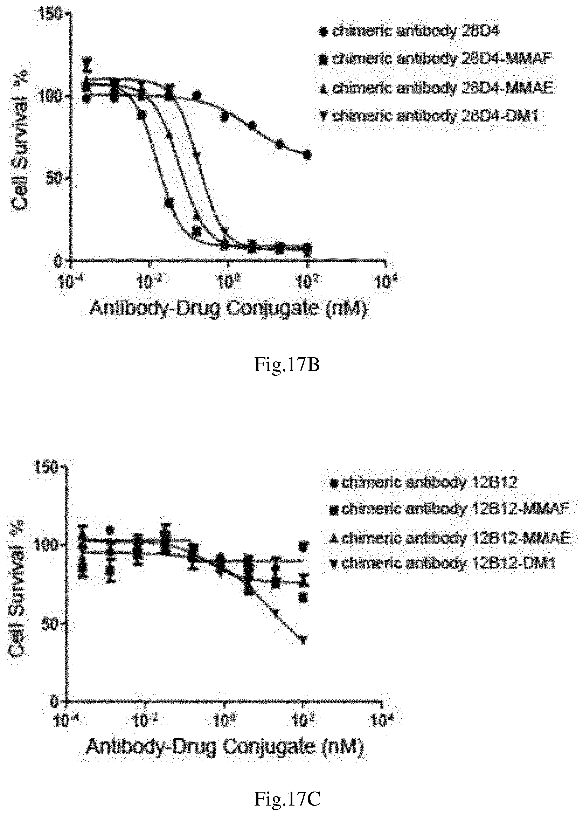

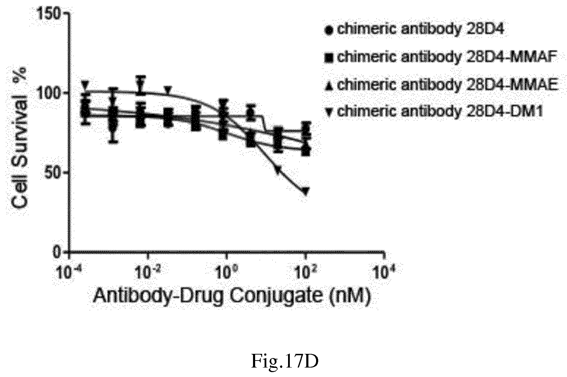

The obtained purified TPBG antibody-drug conjugates were serially diluted with complete medium. 100µL of TPBG-expressing 293-hTPBG recombinant cell line (the method of preparation please refer to Example 1: Preparation of Immnogen B) or 293 cell line not expressing TPBG (purchased from ATCC, Catalog NO: #CRL-1573) was added to a the 96-well plate at 2000 cells per cell, cultured overnight, and 10µL of purified TPBG chimeric antibody-drug conjugate diluents of different concentrations were added to each well. After further culturing the cells for 5 days, cell viability was measured by the CellTiter-Glo kit (purchased from Promega and used according to instructions). Results were shown in Table 16 and Fig. 17, wherein IC50 in Table 16 refers to a half effective amount of the cell activity that is inhibited after drug treatment, reflecting the cytotoxicity by measuring cell viability. Fig.17A and 17B are the cell cytotoxicity effects of the purified TPBG chimeric antibody-drug conjugates on TPBG expression-positive recombinant cell line 293-hTPBG; Figs.17C and 17D are the cytotoxicity effects of the purified TPBG chimeric antibody-drug conjugates on the 293 cell line without expressing TPBG. The results indicate that the purified TPBG chimeric antibody-drug conjugates coupling with different small-molecule toxins have different degrees of cytotoxicity effects on TPBG-positive cells, and no cytotoxicity effects on TPBG-negative 293 cells was found, indicating that the cytotoxicity of TPBG chimeric antibody-drug conjugates on TPBG-positive cells were specific, and the TPBG chimeric antibody-drug conjugates coupling with MC-MMAF possess a low value of IC50, indicating that it has the strongest cellular killing efficacy.

**Table 16 Detection of Specific Cytotoxicity of Purified TPBG Chimeric Antibody-Drug Conjugates on TPBG-Positive 293-hTPBG and TPBG-Negative 293Cells via Cellular Killing Assay**

| Sample Name | IC50 (nM) | |
|---|---|---|
| | 293-hTPBG | 293 |
| Chimeric Antibody 12B12 | 13.58 | None |
| Chimeric Antibody 12B12-MMAF | 0.001 | >100 |
| Chimeric Antibody 12B12-MMAE | 0.033 | >100 |
| Chimeric Antibody 12B12-DM1 | 0.154 | 8.885 |
| Chimeric Antibody 28D4 | 1.137 | None |
| Chimeric Antibody 28D4-MMAF | 0.024 | >100 |
| Chimeric Antibody 28D4-MMAE | 0.08 | >100 |
| Chimeric Antibody 28D4-DM1 | 0.406 | 7.658 |

It should be understood that after reading the above contents of the present invention, those skilled in the art can make various changes or modifications to the present invention, and these equivalent forms also fall within the scope defined by the appended claims of the present application.

## Claims

1. An isolated protein comprising one or more of heavy chain CDR1, heavy chain CDR2 and heavy chain CDR3 of TPBG antibody and/or one or more of light chain CDR1, light chain CDR2 and light chain CDR3 of the TPBG antibody,
wherein the amino acid sequence of the heavy chain CDR1 is set forth as SEQ ID NO:2, SEQ ID NO:10, SEQ ID NO:18, SEQ ID NO:26, SEQ ID NO:34, SEQ ID NO:42, SEQ ID NO:50, SEQ ID NO:58, SEQ ID NO:66 or SEQ ID NO:74; the amino acid sequence of the heavy chain CDR2 is set forth as SEQ ID NO:3, SEQ ID NO:11, SEQ ID NO:19, SEQ ID NO:27, SEQ ID NO:35, SEQ ID NO:43, SEQ ID NO:51, SEQ ID NO:59, SEQ ID NO:67 or SEQ ID NO:75; the amino acid sequence of the heavy chain CDR3 is set forth as SEQ ID NO:4, SEQ ID NO:12, SEQ ID NO:20, SEQ ID NO:28, SEQ ID NO:36, SEQ ID NO:44, SEQ ID NO:52, SEQ ID NO:60, SEQ ID NO:68 or SEQ ID NO:76;
the amino acid sequence of the light chain CDR1 is set forth as SEQ ID NO:6, SEQ ID NO:14, SEQ ID NO:22, SEQ ID NO:30, SEQ ID NO:38, SEQ ID NO:46, SEQ ID NO:54, SEQ ID NO:62, SEQ ID NO:70 or SEQ ID NO:78; the amino acid sequence of the light chain CDR2 is set forth as SEQ ID NO:7, SEQ ID NO:15, SEQ ID NO:23, SEQ ID NO:31, SEQ ID NO:39, SEQ ID NO:47, SEQ ID NO:55, SEQ ID NO:63, SEQ ID NO:71 or SEQ ID NO:79; the amino acid sequence of the light chain CDR3 is set forth as SEQ ID NO:8, SEQ ID NO:16, SEQ ID NO:24, SEQ ID NO:32, SEQ ID NO:40, SEQ ID NO:48, SEQ ID NO:56, SEQ ID NO:64, SEQ ID NO:72 or SEQ ID NO:80;
or the amino acid sequence of the heavy chain CDR1 has at least 80% sequence identity to the sequence set forth as SEQ ID NO:2, SEQ ID NO:10, SEQ ID NO:18, SEQ ID NO:26, SEQ ID NO:34, SEQ ID NO:42, SEQ ID NO:50, SEQ ID NO:58, SEQ ID NO:66 or SEQ ID NO:74; the amino acid sequence of the heavy chain CDR2 has at least 80% sequence identity to the sequence set forth as SEQ ID NO:3, SEQ ID NO:11, SEQ ID NO:19, SEQ ID NO:27, SEQ ID NO:35, SEQ ID NO:43, SEQ ID NO:51, SEQ ID NO:59, SEQ ID NO:67 or SEQ ID NO:75; the amino acid sequence of the heavy chain CDR3 has at least 80% sequence identity to the sequence set forth as SEQ ID NO:4, SEQ ID NO:12, SEQ ID NO:20, SEQ ID NO:28, SEQ ID NO:36, SEQ ID NO:44, SEQ ID NO:52, SEQ ID NO:60, SEQ ID NO:68 or SEQ ID NO:76;
the amino acid sequence of the light chain CDR1 has at least 80% sequence identity to the sequence set forth as SEQ ID NO:6, SEQ ID NO:14, SEQ ID NO:22, SEQ ID NO:30, SEQ ID NO:38, SEQ ID NO:46, SEQ ID NO:54, SEQ ID NO:62, SEQ ID NO:70 or SEQ ID NO:78; the amino acid sequence of the light chain CDR2 has at least 80% sequence identity to the sequence set forth as SEQ ID NO:7, SEQ ID NO:15, SEQ ID NO:23, SEQ ID NO:31, SEQ ID NO:39, SEQ ID NO:47, SEQ ID NO:55, SEQ ID NO:63, SEQ ID NO:71 or SEQ ID NO:79; the amino acid sequence of the light chain CDR3 has at least 80% sequence identity to the sequence set forth as SEQ ID NO:8, SEQ ID NO:16, SEQ ID NO:24, SEQ ID NO:32, SEQ ID NO:40, SEQ ID NO:48, SEQ ID NO:56, SEQ ID NO:64, SEQ ID NO:72 or SEQ ID NO:80.

2. The protein according to claim 1, wherein the amino acid sequence of the heavy chain CDR1 is set forth as SEQ ID NO:2, the amino acid sequence of the heavy chain CDR2 is set forth as SEQ ID NO:3, and the amino acid sequence of the heavy chain CDR3 is set forth as SEQ ID NO:4; the amino acid sequence of the heavy chain CDR1 is set forth as SEQ ID NO:10, the amino acid sequence of the heavy chain CDR2 is set forth as SEQ ID NO:11, and the amino acid sequence of the heavy chain CDR3 is set forth as SEQ ID NO:12; the amino acid sequence of the heavy chain CDR1 is set forth as SEQ ID NO:18, the amino acid sequence of the heavy chain CDR2 is set forth as SEQ ID NO:19, and the amino acid sequence of the heavy chain CDR3 is set forth as SEQ ID NO:20; the amino acid sequence of the heavy chain CDR1 is set forth as SEQ ID NO:26, the amino acid sequence of the heavy chain CDR2 is set forth as SEQ ID NO:27, and the amino acid sequence of the heavy chain CDR3 is set forth as SEQ ID NO:28; the amino acid sequence of the heavy chain CDR1 is set forth as SEQ ID NO:34, the amino acid sequence of the heavy chain CDR2 is set forth as SEQ ID NO:35, and the amino acid sequence of the heavy chain CDR3 is set forth as SEQ ID NO:36; the amino acid sequence of the heavy chain CDR1 is set forth as SEQ ID NO:42, the amino acid sequence of the heavy chain CDR2 is set forth as SEQ ID NO:43, and the amino acid sequence of the heavy chain CDR3 is set forth as SEQ ID NO:44; the amino acid sequence of the heavy chain CDR1 is set forth as SEQ ID NO:50, the amino acid sequence of the heavy chain CDR2 is set forth as SEQ ID NO:51, and the amino acid sequence of the heavy chain CDR3 is set forth as SEQ ID NO:52; the amino acid sequence of the heavy chain CDR1 is set forth as SEQ ID NO:58, the amino acid sequence of the heavy chain CDR2 is set forth as SEQ ID NO:59, and the amino acid sequence of the heavy chain CDR3 is set forth as SEQ ID NO:60; the amino acid sequence of the heavy chain CDR1 is set forth as SEQ ID NO:66, the amino acid sequence of the heavy chain CDR2 is set forth as SEQ ID NO:67, and the amino acid sequence of the heavy chain CDR3 is set forth as SEQ ID NO:68; the amino acid sequence of the heavy chain CDR1 is set forth as SEQ ID NO:74, the amino acid sequence of the heavy chain CDR2 is set forth as SEQ ID NO:75, and the amino acid sequence of the heavy chain CDR3 is set forth as SEQ ID NO:76;
the amino acid sequence of the light chain CDR1 is set forth as SEQ ID NO: 6, the amino acid sequence of the light chain CDR2 is set forth as SEQ ID NO:7, and the amino acid sequence of the light chain CDR3 is set forth as SEQ ID NO:8; the amino acid sequence of the light chain CDR1 is set forth as SEQ ID NO:14, the amino acid sequence of the light chain CDR2 is set forth as SEQ ID NO:15, and the amino acid sequence of the light chain CDR3 is set forth as SEQ ID NO:16; or the amino acid sequence of the light chain CDR1 is set forth as SEQ ID NO: 22, the amino acid sequence of the light chain CDR2 is set forth as SEQ ID NO:23, and the amino acid sequence of the light chain CDR3 is set forth as SEQ ID NO:24; the amino acid sequence of the light chain CDR1 is set forth as SEQ ID NO: 30, the amino acid sequence of the light chain CDR2 is set forth as SEQ ID NO:31, and the amino acid sequence of the light chain CDR3 is set forth as SEQ ID NO:32; the amino acid sequence of the light chain CDR1 is set forth as SEQ ID NO: 38, the amino acid sequence of the light chain CDR2 is set forth as SEQ ID NO:39, and the amino acid sequence of the light chain CDR3 is set forth as SEQ ID NO:40; the amino acid sequence of the light chain CDR1 is set forth as SEQ ID NO: 46, the amino acid sequence of the light chain CDR2 is set forth as SEQ ID NO:47, and the amino acid sequence of the light chain CDR3 is set forth as SEQ ID NO:48; the amino acid sequence of the light chain CDR1 is set forth as SEQ ID NO: 54, the amino acid sequence of the light chain CDR2 is set forth as SEQ ID NO:55, and the amino acid sequence of the light chain CDR3 is set forth as SEQ ID NO:56; the amino acid sequence of the light chain CDR1 is set forth as SEQ ID NO: 62, the amino acid sequence of the light chain CDR2 is set forth as SEQ ID NO:63, and the amino acid sequence of the light chain CDR3 is set forth as SEQ ID NO:64; the amino acid sequence of the light chain CDR1 is set forth as SEQ ID NO: 70, the amino acid sequence of the light chain CDR2 is set forth as SEQ ID NO:71, and the amino acid sequence of the light chain CDR3 is set forth as SEQ ID NO:72; or the amino acid sequence of the light chain CDR1 is set forth as SEQ ID NO: 78, the amino acid sequence of the light chain CDR2 is set forth as SEQ ID NO:79, and the amino acid sequence of the light chain CDR3 is set forth as SEQ ID NO:80.

3. An isolated protein comprising the heavy chain variable region of TPBG antibody and/or the light chain variable region of TPBG antibody; the amino acid sequence of the heavy chain variable region is set forth as SEQ ID NO:1, SEQ ID NO:9, SEQ ID NO:17, SEQ ID NO:25, SEQ ID NO:33, SEQ ID NO:41, SEQ ID NO:49, SEQ ID NO:57, SEQ ID NO:65 or SEQ ID NO:73; the amino acid sequence of the light chain variable region is set forth as SEQ ID NO:5, SEQ ID NO:13, SEQ ID NO:21, SEQ ID NO:29, SEQ ID NO:37, SEQ ID NO:45, SEQ ID NO:53, SEQ ID NO:61, SEQ ID NO:69 or SEQ ID NO:77.

4. The protein according to claim 3, wherein the amino acid sequence of the heavy chain variable region is set forth as SEQ ID NO:1, the amino acid sequence of the light chain variable region is set forth as SEQ ID NO:5; the amino acid sequence of the heavy chain variable region is set forth as SEQ ID NO:9, the amino acid sequence of the light chain variable region is set forth as SEQ ID NO:13; the amino acid sequence of the heavy chain variable region is set forth as SEQ ID NO:17, the amino acid sequence of the light chain variable region is set forth as SEQ ID NO:21; the amino acid sequence of the heavy chain variable region is set forth as SEQ ID NO:25, the amino acid sequence of the light chain variable region is set forth as SEQ ID NO:29; the amino acid sequence of the heavy chain variable region is set forth as SEQ ID NO:33, the amino acid sequence of the light chain variable region is set forth as SEQ ID NO:37; the amino acid sequence of the heavy chain variable region is set forth as SEQ ID NO:41, the amino acid sequence of the light chain variable region is set forth as SEQ ID NO:45; the amino acid sequence of the heavy chain variable region is set forth as SEQ ID NO:49, the amino acid sequence of the light chain variable region is set forth as SEQ ID NO:53; the amino acid sequence of the heavy chain variable region is set forth as SEQ ID NO:57, the amino acid sequence of the light chain variable region is set forth as SEQ ID NO:61; the amino acid sequence of the heavy chain variable region is set forth as SEQ ID NO:65, the amino acid sequence of the light chain variable region is set forth as SEQ ID NO:69; or the amino acid sequence of the heavy chain variable region is set forth as SEQ ID NO:73, the amino acid sequence of the light chain variable region is set forth as SEQ ID NO:77.

5. The protein according to at least one of claims 1-4 further comprising heavy chain constant region of antibody and/or light chain constant region of antibody.

6. The protein according to claim 5 wherein the heavy chain constant region of antibody is human or mouse heavy chain constant region of antibody; the light chain constant region is human or mouse light chain constant region of antibody.

7. The protein according to claim 6, wherein the heavy chain constant region of antibody is human heavy chain constant region of antibody; the light chain constant region is human light chain constant region of antibody.

8. The protein according to claims 1 or 3, wherein the protein is TPBG antibody, monoclonal antibody, antibody full-length protein, protein fragment of antigen-antibody binding domain, dual-specificity antibody, multiple-specificity antibody, single chain antibody, single domain antibody (sdAb) and single-domain antibody.

9. A nucleic acid encoding at least one of the proteins according to claims 1-7.

10. The nucleic acid according to claim 9, wherein the nucleotide sequence encoding the heavy chain variable region is set forth as SEQ ID NO:81, SEQ ID NO:83, SEQ ID NO:85, SEQ ID NO:87, SEQ ID NO:89, SEQ ID NO:91, SEQ ID NO:93, SEQ ID NO:95, SEQ ID NO:97 or SEQ ID NO:99; and/or the nucleotide sequence encoding the light chain variable region is set forth as SEQ ID NO:82, SEQ ID NO:84, SEQ ID NO:86, SEQ ID NO:88, SEQ ID NO:90, SEQ ID NO:92, SEQ ID NO:94, SEQ ID NO:96, SEQ ID NO:98 or SEQ ID NO:100.

11. The nucleic acid according to claim 10, wherein the nucleotide sequence encoding the heavy chain variable region is set forth as SEQ ID NO:81, and the nucleotide sequence encoding the light chain variable region is set forth as SEQ ID NO:82; the nucleotide sequence encoding the heavy chain variable region is set forth as SEQ ID NO:83, and the nucleotide sequence encoding the light chain variable region is set forth as SEQ ID NO:84; the nucleotide sequence encoding the heavy chain variable region is set forth as SEQ ID NO:85, and the nucleotide sequence encoding the light chain variable region is set forth as SEQ ID NO:86; the nucleotide sequence encoding the heavy chain variable region is set forth as SEQ ID NO:87, and the nucleotide sequence encoding the light chain variable region is set forth as SEQ ID NO:88; the nucleotide sequence encoding the heavy chain variable region is set forth as SEQ ID NO:89, and the nucleotide sequence encoding the light chain variable region is set forth as SEQ ID NO:90; the nucleotide sequence encoding the heavy chain variable region is set forth as SEQ ID NO:91, and the nucleotide sequence encoding the light chain variable region is set forth as SEQ ID NO:92; the nucleotide sequence encoding the heavy chain variable region is set forth as SEQ ID NO:93, and the nucleotide sequence encoding the light chain variable region is set forth as SEQ ID NO:94; the nucleotide sequence encoding the heavy chain variable region is set forth as SEQ ID NO:95, and the nucleotide sequence encoding the light chain variable region is set forth as SEQ ID NO:96; the nucleotide sequence encoding the heavy chain variable region is set forth as SEQ ID NO:97, and the nucleotide sequence encoding the light chain variable region is set forth as SEQ ID NO:98; or the nucleotide sequence encoding the heavy chain variable region is set forth as SEQ ID NO:99, and the nucleotide sequence encoding the light chain variable region is set forth as SEQ ID NO:100.

12. A recombinant expression vector comprising the nucleic acid according at least one of the claims 9-11.

13. A recombinant expression transformant comprising the recombinant expression vector according to claim 12.

14. A preparation method of TPBG antibody comprising culturing the recombinant expression transformant according to claim 13 and obtaining TPBG antibody from culture.

15. An immunoconjugate comprising the protein according to at least one of claims 1-7 which is covalently linked to a cytotoxic agent.

16. The immunoconjugate according to claim 15, wherein 1 equivalent of the protein according to at least one of claims 1-7 is linked to y equivalents of cytotoxic agent through x equivalents of linker, which has the structure shown in formula 1,
Ab-(L)x-(D)y Formula 1
wherein Ab is the protein according to at least one of claims 1-7, L is linker; D is cytotoxic agent; x is natural number, preferably an integer selected from 1-20; y is 0 or natural number, preferably an integer selected from 0-20; x and y are preferably integers selected from 1-2, or 2-4, or 4-8, or 8-20, independently; the ratio of x to y is preferably 1:1.

17. The immunoconjuate according to claim 16, wherein the linker is active esters, carbonates, carbamates, imine phosphate esters, oximes, hydrazones, acetals, orthoesters, aminos, small peptides or nucleotide fragments, preferably, the linker is maleimidocaproyl (MC), maleimidocaproyl-L-valine-L-citrulline p-amino benzyl alcohol (MC-VC-PAB) or 4-(N-maleimide methyl) cyclohexane-1-carboxylic succinimide (SMCC);
and/or the D is selected from cytotoxin, chemotherapeutic agent, radioisotope, therapeutic nucleic acid, immunomodulator, anti-angiogenic agents, anti-proliferative and pro-apoptotic agent or cytolytic enzyme, preferably selected from methylauristatin E, methylauristatin F or N2'-deacetyl-N2'-(3-mercapto-1-oxypropyl) methesteine.

18. The immunoconjugate according to claim 17, wherein x=y=n in formula 1, and the structure of the immunoconjugate is shown in formula 3, 4 or 5, 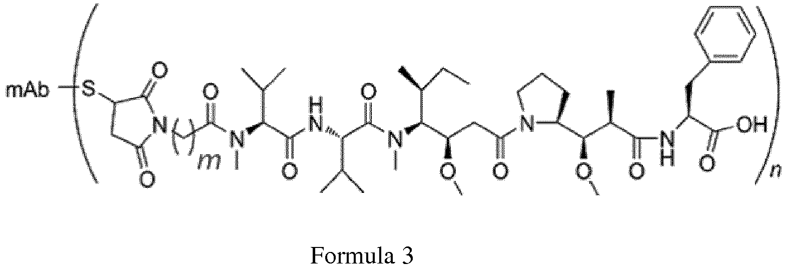 in formula 3, m is 1-10, preferably 5 (i.e. L is maleimidocaproyl); D is methylauristatin F; 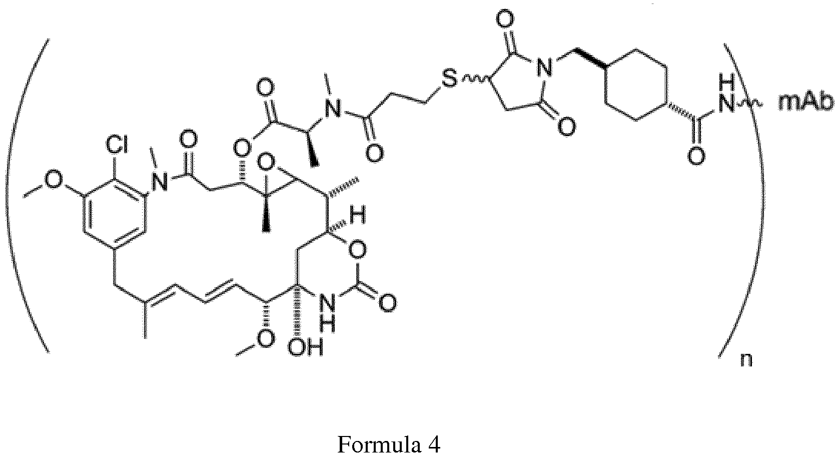 in formula 4, L is 4-(N-maleimide methyl) cyclohexane-1-carboxylic succinimide; D is N2'-deacetyl-N2'-(3-mercapto-1-oxypropyl) methesteine (DM1); 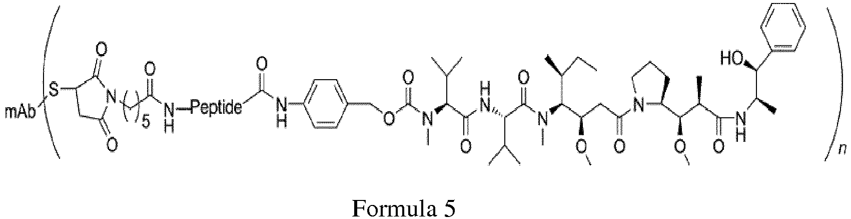 in formula 5, L is maleimidocaproyl-L-valine-L-citrulline p-amino benzyl alcohol, D is methylauristatin E (MMAE);
wherein n is a natural number, preferably an integer selected from 1-20, more preferably an integer selected from 1-2, or 2-4, or 4-8, or 8-20.

19. A pharmaceutical composition comprising the immunoconjugate according to at least one of claims 15-18 and pharmaceutically acceptable vehicle.

20. The pharmaceutical composition according to claim 19 comprising 0.01-99.99% of the protein according to at least one of claims 1-7 and 0.01-99.99% of pharmaceutical vehicle, wherein the percentage is the mass percentage of the pharmaceutical composition.

21. Use of the protein according to at least one of claims 1-7 in the preparation of anti-tumor drugs.

22. Use of the immunoconjugate according to at least one of claims 15-18 in preparation of anti-tumor drugs.

23. Use of the pharmaceutical composition according to at least one of claims 19-20 in the preparation of anti-tumor drugs.

24. A method for detecting cells overexpressing TPBG protein, which comprises the following steps: contacting the protein according to at least one of claims 1-7 with the sample to be tested *in vitro,* and detecting the binding of the protein according to at least one of claims 1-7 with the sample to be tested.
